# EUROPEAN PATENT APPLICATION

(11) **EP 4 437 839 A1**
(43) Date of publication of application: **02.10.2024**
(21) Application number: 24166986.0
(22) Date of filing: 27.03.2024
(51) Int. Cl.: A01H 6/54, C07H 3/06, C12N 9/10, C12N 15/82, C12N 15/52

(54) **COMPOSITIONS AND METHODS COMPRISING PLANTS WITH REDUCED RAFFINOSE FAMILY OLIGOSACCHARIDES AND/OR HIGH PROTEIN CONTENT**

(30) Priority: 28.03.2023 US 202363492726 P
(71) Applicant: Benson Hill, Inc., St. Louis MO 63132 (US)
(72) Inventor: BEGEMANN, Matthew Brett, 63132 St. Louis (US); GOETTEL, Herbert Wolfgang, 63132 St. Louis (US); FRICK, Elizabeth May, 63132 St. Louis (US); ZESS, Erin Kathleen, 63132 St. Louis (US)
(74) Representative: Inspicos P/S

(57) **Abstract**

Provided herein are plants, plant parts, a population of plants or plant parts, and plant products (e.g., seed composition, protein and/or oil composition) comprising reduced galactinol synthase (GAS) and/or raffinose synthase (RS) activity, and compositions and methods of producing such plants and plant parts. The plants, plant parts, population of plants or plant parts, or plant products can have a genetic mutation that reduces the GAS and/or RS activity, decreased RFO (e.g., raffinose, stachyose) content, increased sucrose content, and/or increased protein content. The mutation can be located at least partially in a *GAS* and/or RS gene or its homolog or its regulatory region.

## Description

### RELATED APPLICATIONS

This application claims priority to U.S. Provisional Application No. 63/492,726, filed on March 28, 2023, the content of which is incorporated herein by reference in its entirety.

### SEQUENCE LISTING

This application contains a Sequence Listing which is submitted herewith in electronically readable format. The Sequence Listing file was created on March 26, 2024, is named "B88552_1590_SL.xml" and its size is 106 kb. The entire contents of the Sequence Listing in the XML file are incorporated by reference herein.

### FIELD OF THE INVENTION

The present disclosure relates to the field of agricultural biotechnology. More specifically, this disclosure relates to plants and plant parts having reduced raffinose and/or stachyose, and associated methods and compositions.

### BACKGROUND OF THE INVENTION

With the ever-increasing world population and the dwindling supply of arable land available for agriculture, nutrient rich, resilient plants are desired. Plant-based meal or food composition can include a carbohydrate component, which can be comprised of sucrose and raffinose family oligosaccharides (RFOs, e.g., raffinose and stachyose). RFOs can be found abundantly in many plants, and can be an obstacle to the efficient utilization of some economically important crop species. Generally, RFOs are poorly digested by monogastric animals, including pigs, dogs, chickens, and humans, due to the lack of α-galactosidase activity in the gut. RFOs in the gut of an monogastric animal eventually undergo microbial fermentation by colonic bacteria resulting in production of hydrogen, methane, and CO₂ - major components of flatulent gases- and acidification of the gut. Accumulation of these gases can cause bloating, abdominal rumblings, cramps, diarrhea, nausea, and abdominal discomfort. Indigestibility and flatulence can deter consumption and utilization of plants or plant parts in human and animal diets.

The removal of RFOs from plant, plant part, or plant meal can increase the metabolizable energy of the diet and reduce flatulent production. Accordingly, providing plants and seeds that possess low content of RFOs (e.g., raffinose or stachyose) could offer important health and commercial advantages.

### SUMMARY OF THE INVENTION

Provided herein are plants, plant parts, a population of plants or plant parts, and plant products (e.g., seed composition, protein and/or oil composition) comprising reduced galactinol synthase (GAS) and/or raffinose synthase (RS) activity and/or increased protein content, and compositions and methods of producing such plants and plant parts. The plants, plant parts, population of plants or plant parts, or plant products can have a genetic mutation that decreases GAS and/or RS activity, e.g., one or more mutations in at least one native *GAS* and/or *RS* gene or its homolog or its regulatory region (e.g., promoter, 5'UTR), decreased expression levels of the *GAS* and/or *RS* gene, decreased level or activity of a GAS and/or RS protein, decreased RFO (e.g., raffinose, stachyose) content, increased sucrose content, and/or increased protein content compared to a control plant or plant part. The mutation can be located at least partially in a *GAS* and/or *RS* gene or its homolog or its regulatory region.

In one aspect, the present disclosure provides a plant or plant part comprising decreased galactinol synthase (GAS) activity and/or raffinose synthase (RS) activity compared to a control plant or plant part. The plant or plant part comprises a genetic mutation that decreases the GAS activity and/or the RS activity.

In some embodiments, the plant or plant part comprises decreased raffinose family oligosaccharide (RFO) content and/or increased sucrose content as compared to a control plant or plant part. In some embodiments, the RFO comprises raffinose and/or stachyose.

In some embodiments, the mutation comprises one or more insertions, substitutions, or deletions in at least one native *GAS* and/or *RS* gene or homolog thereof or regulatory region thereof in the plant or plant part. An expression level of the at least one mutated *GAS* and/or *RS* gene or homolog thereof is reduced compared to corresponding at least one native *GAS* and/or *RS* gene or homolog thereof without the mutation, and/or level or activity of a GAS and/or RS protein encoded by the at least one mutated *GAS* and/or *RS* gene or homolog thereof is reduced compared to a GAS and/or RS protein encoded by corresponding at least one native *GAS* and/or *RS* gene or homolog thereof without the mutation.

In some embodiments, the mutation is located at least partially in a *GAS* or *RS* gene or regulatory region thereof. The *GAS* or *RS* gene comprises a nucleic acid sequence having at least 80% sequence identity to a nucleic acid sequence of any one of SEQ ID NOs: 1-8, 25, 31, 37-40, 42, and 50, wherein the nucleic acid sequence encodes a polypeptide that retains GAS or RS activity; the *GAS* or *RS* gene comprises the nucleic acid sequence of SEQ ID NOs: 1-8, 25, 31, 37-40, 42, and 50; the *GAS* or *RS* gene encodes a polypeptide comprising an amino acid sequence having at least 80% sequence identity to an amino acid sequence of any one of SEQ ID NOs: 9-12, 41, 43, and 51, wherein the polypeptide retains GAS or RS activity; and/or the *GAS* or *RS* gene encodes a polypeptide comprising an amino acid sequence of any one of SEQ ID NOs: 9-12, 41, 43, and 51.

In some embodiments, the plant or plant part comprises a mutated *Pisum sativum GAS* gene comprising an insertion, a substitution, or a deletion of one or more nucleotides of SEQ ID NO: 1, 2, 5, 6, 25, 37, or 38, and/or a mutated *Pisum sativum RS* gene comprising an insertion, a substitution, or a deletion of one or more nucleotides of SEQ ID NO: 3, 4, 7, 8, 31, 39, or 40, and/or a mutated *Glycine max GAS* gene comprising an insertion, a substitution, or a deletion of one or more nucleotides of SEQ ID NO: 42 or 50. In some embodiments, the plant or plant part comprises a mutated *Pisum sativum GAS* gene comprising a G to A substitution of nucleotide 128 of SEQ ID NO: 5 or 25, or comprising a nucleic acid sequence of SEQ ID NO: 15 or 26; a mutated *Pisum sativum GAS* gene comprising a deletion of nucleotides 115-119 of SEQ ID NO: 25, or comprising a nucleic acid sequence of SEQ ID NO: 27; a mutated *Pisum sativum GAS* gene comprising a deletion of nucleotides 111-117 of SEQ ID NO: 25, or comprising a nucleic acid sequence of SEQ ID NO: 28; a mutated *Pisum sativum RS* gene comprising a G to A substitution of nucleotide 1044 of SEQ ID NO: 7 or 31, or comprising a nucleic acid sequence of SEQ ID NO: 16 or 32; a mutated *Pisum sativum RS* gene comprising a deletion of nucleotides 1246-1253 of SEQ ID NO: 31, or comprising a nucleic acid sequence of SEQ ID NO: 33; a mutated *Pisum sativum RS* gene comprising a deletion of nucleotides 1248-1254 of SEQ ID NO: 31, or comprising a nucleic acid sequence of SEQ ID NO: 34; a mutated *Pisum sativum* GAS protein comprising a G to D substitution of amino acid 43 of SEQ ID NO: 9, or comprising an amino acid sequence of SEQ ID NO: 17; a mutated *Pisum sativum* GAS protein encoded by the nucleic acid sequence of SEQ ID NO: 27, or comprising an amino acid sequence of SEQ ID NO: 29; a mutated *Pisum sativum* GAS protein encoded by the nucleic acid sequence of SEQ ID NO: 28, or comprising an amino acid sequence of SEQ ID NO: 30; a truncated *Pisum sativum* RS protein comprising a deletion of amino acids 348-798 of SEQ ID NO: 11, or comprising an amino acid sequence of SEQ ID NO: 18; a mutated *Pisum sativum* RS protein encoded by the nucleic acid sequence of SEQ ID NO: 33, or comprising an amino acid sequence of SEQ ID NO: 35; a mutated *Pisum sativum* RS protein encoded by the nucleic acid sequence of SEQ ID NO: 34, or comprising an amino acid sequence of SEQ ID NO: 36; a mutated *Glycine max GAS* gene comprising a deletion of nucleotides 197-209 of SEQ ID NO: 42, or comprising a nucleic acid sequence of SEQ ID NO: 44; a mutated *Glycine max GAS* gene comprising a deletion of nucleotides 199-209 of SEQ ID NO: 42, or comprising a nucleic acid sequence of SEQ ID NO: 45; a mutated *Glycine max GAS* gene comprising a deletion of nucleotide 204 of SEQ ID NO: 42, or comprising a nucleic acid sequence of SEQ ID NO: 46; and/or a mutated *Glycine max* GAS protein encoded by the nucleic acid sequence of any one of SEQ ID NOs: 44-46, or comprising an amino acid sequence of any one of SEQ ID NOs: 47-49.

In some embodiments, the mutation comprises a missense mutation, a nonsense mutation, or an out-of-frame mutation of the at least one *GAS* and/or *RS* gene or homolog thereof.

In some embodiments, the plant or plant part comprises 2-5 genes encoding a GAS and/or RS protein. In some embodiments, the 2-5 genes have less than 100% sequence identity to one another.

In some embodiments, the plant or plant part is a legume. In some embodiments, the plant or plant part is selected from the group consisting of pea *(Pisum sativum),* soybean *(Glycine max),* beans (*Phaseolus* spp., *Vigna* spp.), common bean (*Phaseolus vulgaris*), mung bean (*Vigna radiata*), cowpea (*Vigna unguiculata*), adzuki bean (*Vigna angularis*), fava bean (*Viciafaba*), chickpea (*Cicer arietinum*), peanut *(Arachis hypogaea*), lentils (*Lens culinaris, Lens esculenta*), lupins (*Lupinus* spp.), white lupin (*Lupinus albus*), mesquite (*Prosopis* spp.), carob (*Ceratonia siliqua*), tamarind (*Tamarindus indica*), alfalfa (*Medicago sativa*), barrel medic (*Medicago truncatula*), birdsfood trefoil (*Lotus japonicus*), licorice (*Glycyrrhiza glabra*), and clover (*Trifolium* spp.).

In some embodiments, the plant or plant part is selected from the group consisting of corn (*Zea mays*), Brassica species, *Brassica napus, Brassica rapa, Brassica juncea,* rice (*Oryza sativa*), rye (*Secale cereale*), sorghum (*Sorghum bicolor*, *Sorghum vulgare*), millet, pearl millet (*Pennisetum glaucum*), proso millet (*Panicum miliaceum*), foxtail millet (*Setaria italica*), finger millet (*Eleusine coracana*), sunflower (*Helianthus annuus*), safflower (*Carthamus tinctorius*), wheat (*Triticum aestivum*), tobacco (*Nicotiana tabacum*), potato (*Solanum tuberosum*), peanuts (*Arachis hypogaea*), cotton (*Gossypium barbadense*, *Gossypium hirsutum*), sweet potato (*Ipomoea batatus*), cassava (*Manihot esculenta*), coffee (*Coffea spp.*), coconut (*Cocos nucifera*), pineapple (*Ananas comosus*), citrus trees (*Citrus spp.*), cocoa (*Theobroma cacao*), tea (*Camellia sinensis*), banana (*Musa spp.*), avocado (*Persea americana*), fig (*Ficus casica*), guava (*Psidium guajava*), mango (*Mangifera indica*), olive (*Olea europaea*), papaya (*Carica papaya*), cashew (*Anacardium occidentale*), macadamia (*Macadamia integrifolia*), almond (*Prunus amygdalus*), sugar beets (*Beta vulgaris*), sugarcane (*Saccharum spp.*), oats, barley, vegetables, ornamentals, and conifers.

In some embodiments, the plant or plant part is a seed.

In one aspect, provided herein is a population of plants or plant parts comprising the plant or plant part provided herein. The population comprises decreased GAS and/or RS activity, decreased raffinose family oligonucleotide (RFO) content and/or increased sucrose content compared to a control population. In some embodiments, the plant or plant part is a seed, and the population is a population of seeds.

In some embodiments, the plant, plant part, or population of plants or plant parts comprises seed raffinose content that is 85% or less relative to a control, seed stachyose content that is 50% or less relative to a control, and/or seed sucrose content that is increased by about 10% or more relative to a control. In some embodiments, the plant, plant part, or population of plants or plant parts comprises seed raffinose content of about 0.7% or less, seed stachyose content of about 1.5% or less, and/or seed sucrose content of about 3.0% or more dry weight of total seed content. In some embodiments, the plant, plant part, or population of plants or plant parts comprises seed raffinose content of about 0.3% or less dry weight of total seed content. In some embodiments, the plant, plant part, or population of plants or plant parts comprises seed stachyose content of about 1.4% or less, and/or seed sucrose content of about 3.7% or more dry weight of total seed content.

In one aspect, provided herein is a method for decreasing raffinose family oligosaccharide (RFO) content, e.g., raffinose and/or stachyose content in a plant or plant part. The method comprises decreasing galactinol synthase (GAS) activity and/or raffinose synthase (RS) activity in the plant or plant part.

In some embodiments, the method includes introducing a genetic mutation that decreases the GAS and/or RS activity into the plant or plant part. The GAS and/or RS activity is decreased, and raffinose family oligosaccharide (RFO) content is decreased and/or sucrose content is increased in the plant or plant part relative to a control plant or plant part. In some embodiments, the method further comprises introducing the mutation into a plant cell, and regenerating the plant or plant part from the plant cell.

In some embodiments, the mutation comprises one or more insertions, substitutions, or deletions in at least one *GAS* and/or *RS* gene or homolog thereof or regulatory region thereof in the plant or plant part. An expression level of the at least one *GAS* and/or *RS* gene or homolog thereof is reduced by the mutation, and/or level or activity of a GAS and/or RS protein encoded by the at least one *GAS* and/or *RS* gene or homolog thereof is reduced by the mutation.

In some embodiments, the mutation is introduced at least partially into a *GAS* or *RS* gene or regulatory region thereof in the plant or plant part. The *GAS* or *RS* gene comprises a nucleic acid sequence having at least 80% sequence identity to a nucleic acid sequence of any one of SEQ ID NOs: 1-8, 25, 31, 37-40, 42, and 50, wherein the nucleic acid sequence encodes a polypeptide that retains GAS or RS activity, the *GAS* or *RS* gene comprises the nucleic acid sequence of any one of SEQ ID NOs: 1-8, 25, 31, 37-40, 42, and 50, the *GAS* or *RS* gene encodes a polypeptide comprising an amino acid sequence having at least 80% sequence identity to an amino acid sequence of any one of SEQ ID NOs: 9-12, 41, 43, and 51, wherein the polypeptide retains GAS or RS activity, and/or the *GAS* or *RS* gene encodes a polypeptide comprising an amino acid sequence of any one of SEQ ID NOs: 9-12, 41, 43, and 51.

In some embodiments, the mutation comprises an insertion, a substitution, or a deletion of one or more nucleotides of (i) SEQ ID NO: 1, 2, 5, 6, 25, 37, or 38 in *a Pisum sativum GAS* gene and/or (ii) SEQ ID NO: 3, 4, 7, 8, 31, 39, or 40 in a *Pisum sativum RS* gene and/or (iii) SEQ ID NO: 42 or 50 in a *Glycine max* GAS gene. In some embodiments, the mutation comprises a G to A substitution of nucleotide 128 of SEQ ID NO: 5 or 25, or said plant or plant part comprises a polynucleotide comprising a nucleic acid sequence of SEQ ID NO: 15 or 26 when said mutation is introduced; the mutation comprises a deletion of nucleotides 115-119 of SEQ ID NO: 25, or said plant or plant part comprises a polynucleotide comprising a nucleic acid sequence of SEQ ID NO: 27 when said mutation is introduced; the mutation comprises a deletion of nucleotides 111-117 of SEQ ID NO: 25, or said plant or plant part comprises a polynucleotide comprising a nucleic acid sequence of SEQ ID NO: 28 when said mutation is introduced; the mutation comprises a G to A substitution of nucleotide 1044 of SEQ ID NO: 7 or 31, or said plant or plant part comprises a polynucleotide comprising a nucleic acid sequence of SEQ ID NO: 16 or 32 when said mutation is introduced; the mutation comprises a deletion of nucleotides 1246-1253 of SEQ ID NO: 31, or said plant or plant part comprises a polynucleotide comprising a nucleic acid sequence of SEQ ID NO: 33 when said mutation is introduced; the mutation comprises a deletion of nucleotides 1248-1254 of SEQ ID NO: 31, or said plant or plant part comprises a polynucleotide comprising a nucleic acid sequence of SEQ ID NO: 34 when said mutation is introduced; the mutation produces a G to D substitution of amino acid 43 of SEQ ID NO: 9, or said plant or plant part comprises a polypeptide comprising an amino acid sequence of SEQ ID NO: 17 when said mutation is introduced; the mutation produces a mutated *Pisum sativum* GAS protein comprising an amino acid sequence of SEQ ID NO: 29; the mutation produces a mutated *Pisum sativum* GAS protein comprising an amino acid sequence of SEQ ID NO: 30; the mutation produces a deletion of amino acids 348-798 of SEQ ID NO: 11, or said plant or plant part comprises a polypeptide comprising an amino acid sequence of SEQ ID NO: 18 when said mutation is introduced; the mutation produces a mutated *Pisum sativum* RS protein comprising an amino acid sequence of SEQ ID NO: 35; and/or the mutation produces a mutated *Pisum sativum* RS protein comprising an amino acid sequence of SEQ ID NO: 36; the mutation comprises a deletion of nucleotides 197-209 of SEQ ID NO: 42, or said plant or plant part comprises a polynucleotide comprising a nucleic acid sequence of SEQ ID NO: 44 when said mutation is introduced; the mutation comprises a deletion of nucleotides 199-209 of SEQ ID NO: 42, or said plant or plant part comprises a polynucleotide comprising a nucleic acid sequence of SEQ ID NO: 45 when said mutation is introduced; the mutation comprises a deletion of nucleotide 204 of SEQ ID NO: 42, or said plant or plant part comprises a polynucleotide comprising a nucleic acid sequence of SEQ ID NO: 46 when said mutation is introduced; and/or the mutation produces a mutated Glycine max GAS protein comprising an amino acid sequence of any one of SEQ ID NO: 47-49.

In some embodiments, introducing the mutation comprises introducing a missense mutation, a nonsense mutation, or an out-of-frame mutation into the at least one native *GAS* and/or RS gene or homolog thereof.

In some embodiments, the method further comprises contacting the plant or plant part with a mutagen, thereby introducing the mutation into the plant or plant part. In some embodiments, the mutagen is ethyl methanesulfonate (EMS) and/or N-ethyl-N-nitrosourea (ENU).

In some embodiments, the method further comprises introducing editing reagents or a nucleic acid construct encoding the editing reagents into the plant, plant part, or plant cell, thereby introducing the mutation into the plant or plant part. In some embodiments, the editing reagents comprise at least one nuclease. The nuclease cleaves a target site in a genome of the plant, plant part, or plant cell, and the mutation is introduced at the cleaved target site. In some embodiments, at least one nuclease comprises a CRISPR nuclease. In some embodiments, the CRISPR nuclease is a Type II CRISPR system nuclease, a Type V CRISPR system nuclease, a Cas9 nuclease, a Cas12a (Cpf1) nuclease, or a Cms1 nuclease. In some embodiments, the CRISPR nuclease is a Cas12a nuclease or an ortholog thereof.

In some embodiments, the editing reagents comprise one or more guide RNAs (gRNAs). In some embodiments, one or more gRNAs comprise a nucleic acid sequence complementary to a region of a nucleic acid sequence comprising at least one native *GAS* or *RS* gene or homolog thereof or regulatory region thereof in the plant or plant part. In some embodiments, at least one of the one or more gRNAs comprises a nucleic acid sequence encoded by a nucleic acid sequence that shares at least 80% sequence identity with a nucleic acid sequence of SEQ ID NO: 13, 14, or 52, or the nucleic acid sequence of SEQ ID NO: 13, 14, or 52.

In some embodiments of the methods provided herein, the RFO comprises raffinose and/or stachyose.

In some embodiments, the plant or plant part is a legume. In some embodiments, the plant or plant part is selected from the group consisting of pea *(Pisum sativum),* soybean *(Glycine max),* beans (*Phaseolus* spp., *Vigna* spp.), common bean (*Phaseolus vulgaris*), mung bean (*Vigna radiata*), cowpea (*Vigna unguiculata*), adzuki bean (*Vigna angularis*), fava bean (*Viciafaba*), chickpea (*Cicer arietinum*), peanut *(Arachis hypogaea*), lentils (*Lens culinaris*, *Lens esculenta*), lupins (*Lupinus* spp.), white lupin (*Lupinus albus*), mesquite (*Prosopis* spp.), carob (*Ceratonia siliqua*), tamarind (*Tamarindus indica*), alfalfa (*Medicago sativa*), barrel medic (*Medicago truncatula*), birdsfood trefoil (*Lotus japonicus*), licorice (*Glycyrrhiza glabra*), and clover (*Trifolium* spp.).

In some embodiments, the plant or plant part is selected from the group consisting of corn (*Zea mays*), Brassica species, *Brassica napus*, *Brassica rapa*, *Brassica juncea,* rice (*Oryza sativa*), rye (*Secale cereale*), sorghum (*Sorghum bicolor*, *Sorghum vulgare*), millet, pearl millet (*Pennisetum glaucum*), proso millet (*Panicum miliaceum*), foxtail millet (*Setaria italica*), finger millet (*Eleusine coracana*), sunflower (*Helianthus annuus*), safflower (*Carthamus tinctorius*), wheat (*Triticum aestivum*), tobacco (*Nicotiana tabacum*), potato (*Solanum tuberosum*), peanuts *(Arachis hypogaea*), cotton (*Gossypium barbadense*, *Gossypium hirsutum*), sweet potato (*Ipomoea batatus*), cassava (*Manihot esculenta*), coffee (*Coffea spp.*), coconut (*Cocos nucifera*), pineapple (*Ananas comosus*), citrus trees (*Citrus spp.*), cocoa (*Theobroma cacao*), tea (*Camellia sinensis*), banana (*Musa spp.*), avocado (*Persea americana*), fig (*Ficus casica*), guava (*Psidium guajava*), mango (*Mangifera indica*), olive (*Olea europaea*), papaya (*Carica papaya*), cashew (*Anacardium occidentale*), macadamia (*Macadamia integrifolia*), almond (*Prunus amygdalus*), sugar beets (*Beta vulgaris*), sugarcane (*Saccharum spp.*), oats, barley, vegetables, ornamentals, and conifers.

In one aspect, the present disclosure provides a plant or plant part produced by the method provided herein. The plant or plant part comprises reduced GAS and/or RS activity compared to a control plant or plant part. In some embodiments, the plant or plant part comprises decreased raffinose family oligosaccharide content, increased sucrose content, and/or increased protein content compared to a control plant or plant part. In some embodiments, the plant or plant part is a seed.

In one aspect, provided herein is a population of plants or plant parts produced by the method provided herein. The population comprises decreased GAS and/or RS activity, decreased raffinose family oligosaccharide content, increased sucrose content, and/or increased protein content compared to a control population. In some embodiments, the population is a population of seeds.

In some embodiments, the plant, plant part, or population of plants or plant parts produced by the methods provided herein comprises seed raffinose content that is 85% or less relative to a control, seed stachyose content that is 50% or less relative to a control, and/or seed sucrose content that is increased by about 10% or more relative to a control. In some embodiments, the plant, plant part, or population of plants or plant parts produced by the methods provided herein comprises seed raffinose content of about 0.7% or less, seed stachyose content of about 1.5% or less, and/or seed sucrose content of about 3.0% or more dry weight of total seed content. In some embodiments, the plant, plant part, or population of plants or plant parts comprises seed raffinose content of about 0.3% or less dry weight of total seed content. In some embodiments, the plant, plant part, or population of plants or plant parts comprises seed stachyose content of about 1.4% or less, and/or seed sucrose content of about 3.7% or more dry weight of total seed content.

In one aspect, the present disclosure provides a seed composition produced from the plant, plant part, or population of plants or plant parts provided herein.

In one aspect, the present disclosure provides a protein and/or oil composition produced from the plant, plant part, population of plants or plant parts, or the seed composition provided herein.

In some embodiments, the present disclosure provides a food or beverage product comprising the plant, plant part, or plant population, the seed composition, or the protein and/or oil composition provided herein.

In some embodiments, the seed, protein, and/or oil composition, or the food or beverage product produced according to the methods provided herein comprises decreased raffinose family oligosaccharide content, increased sucrose content, and/or increased protein content compared to a control composition or product.

In one aspect, provided herein is a pea composition comprising raffinose content of about 0.7% or less, and/or stachyose content of about 1.5% or less dry weight of total content. In some embodiments, the pea composition comprises raffinose content about 0.3% or less and stachyose content of about 1.5% or less dry weight. In some embodiments, the pea composition further comprises sucrose content of about 3.0% or more dry weight. In some embodiments, the pea composition comprises stachyose content of about 1.4% or less and/or sucrose content of about 3.7% or more. In some embodiments, the pea composition comprises raffinose content of about 0.3% or less, stachyose content of about 1.5% or less, and/or sucrose content of about 3.0% or more. In some embodiments, the pea composition comprises a pea seed composition, a pea protein composition, a pea protein concentrate, a pea protein isolate. Pea flour, pea flake, pea white flake, textured pea protein, or a pea oil composition. In some embodiments, the pea composition comprises a mutated *GAS* and/or RS gene or homolog thereof or regulatory region thereof. In some embodiments, the pea composition comprises a mutated *Pisum sativum GAS* gene comprising an insertion, a substitution, or a deletion of one or more nucleotides of SEQ ID NO: 1, 2, 5, 6, 25, 37, or 38; and/or a mutated *Pisum sativum RS* gene comprising an insertion, a substitution, or a deletion of one or more nucleotides of SEQ ID NO: 3, 4, 7, 8, 31, 39, or 40. In some embodiments, the pea composition comprises a mutated *Pisum sativum GAS* gene comprising a G to A substitution of nucleotide 128 of SEQ ID NO: 5 or 25, or comprising a nucleic acid sequence of SEQ ID NO: 15 or 26; a mutated *Pisum sativum GAS* gene comprising a deletion of nucleotides 115-119 of SEQ ID NO: 25, or comprising a nucleic acid sequence of SEQ ID NO: 27; a mutated *Pisum sativum GAS* gene comprising a deletion of nucleotides 111-117 of SEQ ID NO: 25, or comprising a nucleic acid sequence of SEQ ID NO: 28; a mutated *Pisum sativum RS* gene comprising a G to A substitution of nucleotide 1044 of SEQ ID NO: 7 or 31, or comprising a nucleic acid sequence of SEQ ID NO: 16 or 32; a mutated *Pisum sativum RS* gene comprising a deletion of nucleotides 1246-1253 of SEQ ID NO: 31, or comprising a nucleic acid sequence of SEQ ID NO: 33; a mutated *Pisum sativum RS* gene comprising a deletion of nucleotides 1248-1254 of SEQ ID NO: 31, or comprising a nucleic acid sequence of SEQ ID NO: 34; a mutated *Pisum sativum* GAS protein comprising a G to D substitution of amino acid 43 of SEQ ID NO: 9, or comprising an amino acid sequence of SEQ ID NO: 17; a mutated *Pisum sativum* GAS protein encoded by the nucleic acid sequence of SEQ ID NO: 27, or comprising an amino acid sequence of SEQ ID NO: 29; a mutated *Pisum sativum* GAS protein encoded by the nucleic acid sequence of SEQ ID NO: 28, or comprising an amino acid sequence of SEQ ID NO: 30; a truncated *Pisum sativum* RS protein comprising a deletion of amino acids 348-798 of SEQ ID NO: 11, or comprising an amino acid sequence of SEQ ID NO: 18; a mutated *Pisum sativum* RS protein encoded by the nucleic acid sequence of SEQ ID NO: 33, or comprising an amino acid sequence of SEQ ID NO: 35; and/or a mutated *Pisum sativum* RS protein encoded by the nucleic acid sequence of SEQ ID NO: 34, or comprising an amino acid sequence of SEQ ID NO: 36.

In one aspect, provided herein is a nucleic acid molecule comprising a nucleic acid sequence of a mutated *GAS* or *RS* gene or coding sequence thereof. The mutation is located in a *GAS* or *RS* gene: comprising a nucleic acid sequence having at least 80% sequence identity to a nucleic acid sequence of any one of SEQ ID NOs: 1-8, 25, 31, 37-40, 42, and 50, wherein the nucleic acid sequence encodes a polypeptide that retains GAS or RS activity; comprising the nucleic acid sequence of any one of SEQ ID NOs: 1-8, 25, 31, 37-40, 42, and 50; encoding a polypeptide comprising an amino acid sequence having at least 80% sequence identity to an amino acid sequence of any one of SEQ ID NOs: 9-12, 41, 43, and 51, wherein the polypeptide retains GAS or RS activity; and/or encoding a polypeptide comprising an amino acid sequence of any one of SEQ ID NOs: 9-12, 41, 43, and 51. The mutation decreases level or activity of a GAS and/or RS protein encoded by the *GAS* or *RS* gene. In some embodiments, the nucleic acid sequence: has at least 80% identity to a nucleic acid sequence of SEQ ID NOs: 15, 16, 26-28, 32-34, and 44-46; comprises the nucleic acid sequence of SEQ ID NOs: 15, 16, 26-28, 32-34, and 44-46; encodes a polypeptide comprising an amino acid sequence having at least 80% sequence identity to an amino acid sequence of SEQ ID NOs: 17, 18, 29, 30, 35, 36, and 47-49; and/or encodes a polypeptide comprising an amino acid sequence of SEQ ID NOs: 17, 18, 29, 30, 35, 36, and 47-49.

In one aspect, the present disclosure provides a DNA construct comprising, in operable linkage, a promoter that is functional in a plant cell, and the nucleic acid molecule provided herein.

In one aspect, the present disclosure provides a cell comprising the nucleic acid molecule or the DNA construct provided herein. In some embodiments, the cell is a plant cell.

### BRIEF DESCRIPTION OF THE DRAWINGS

FIG. 1 depicts part of an example biosynthetic pathway of raffinose family oligosaccharides (RFOs) including raffinose and stachyose.
FIG. 2 depicts an expression profile of pea *GAS* (*PsGAS*) gene copies *Pisat. 03G015560* and *Pisat. 06G066360* in various tissues of pea. "fpkm" stands for Fragments Per Kilobase of transcript per Million mapped reads.
FIG. 3 depicts expression profile of pea *RS* (*PsRS*) gene copies *Pisat. 05G054130* and *Pisat.01G021000* in various tissues of pea. "fpkm" stands for Fragments Per Kilobase of transcript per Million mapped reads.
FIG. 4 depicts raffinose, stachyose, and sucrose content in M4 seeds of *PsGAS* G43D mutant, *PsRS* W348* mutant, and control pea plants.
FIG. 5 depicts raffinose and stachyose content in M5seeds of *PsGAS* G43D mutant, *PsRS* W348* mutant (siblings), and non-mutant parent pea plants.

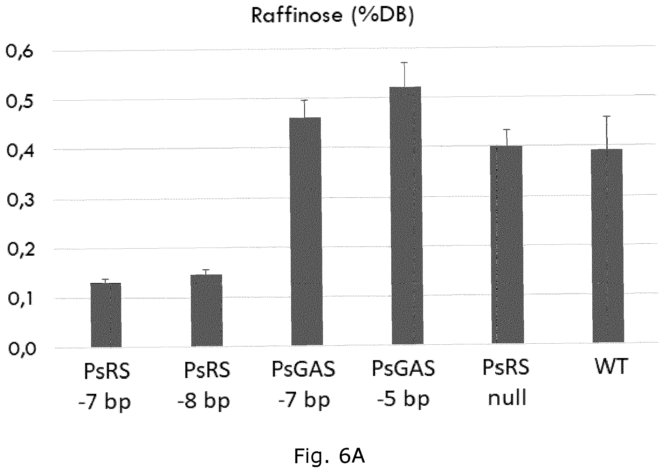

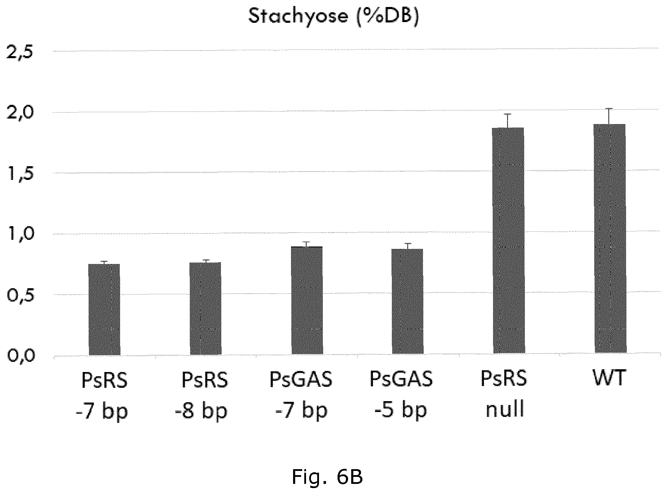

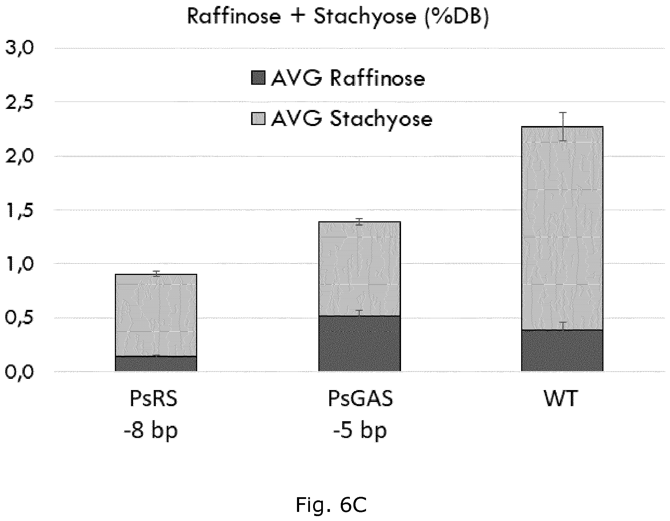

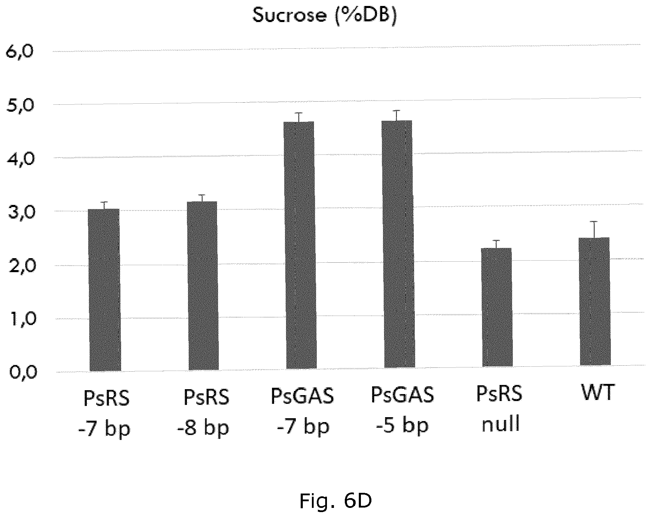

FIGs. 6A, 6B, and 6D depict raffinose content (FIG. 6A), stachyose content (FIG. 6B), and sucrose content (FIG. 6D) in T2 seeds of mutated pea plants having a -7 bp deletion in the *PsRS* gene (resulting in SEQ ID NO: 34, "PsRS -7 bp"), having a -8 bp deletion in the *PsRS* gene (resulting in SEQ ID NO: 33, "PsRS -8 bp"), having a -7 bp deletion in the *PsGAS* gene (resulting in SEQ ID NO: 28, "PsGAS -7 bp"), or having a -5 bp deletion in the *PsGAS* gene (resulting in SEQ ID NO: 27, "PsGAS -5 bp"), in *PsRS* null mutation plants ("PsRS null"), and a wild-type ("WT") plants. FIG. 6C depicts content of raffinose plus stachyose in the PsRS -8 bp, PsGAS -5 bp, and WT pea plant seeds. In FIGs. 6A-6D, each bar represents average ± SD of measurement of seeds harvested from 12 plants.
FIG. 7A depicts content of raffinose plus stachyose in flour and pea protein concentrate (PPC) produced from T2 seeds of the PsRS -8 bp, PsGAS -5 bp, and WT pea plants.
FIG. 7B depicts sucrose content in flour and pea protein concentrate (PPC) produced from T2 seeds of the PsRS -8 bp, PsGAS -5 bp, and WT pea plants.

### DETAILED DESCRIPTION OF THE INVENTION

The present disclosure now will be described more fully hereinafter. The disclosure may be embodied in many different forms and should not be construed as limited to the aspects set forth herein; rather, these aspects are provided so that this disclosure will satisfy applicable legal requirements.

### I. Definitions

Unless otherwise defined, all technical and scientific terms used herein have the same meaning as commonly understood by one of ordinary skill in the art to which this invention belongs. The terminology used in the description of the invention herein is for the purpose of describing particular embodiments only and is not intended to be limiting of the invention.

As used herein, "a," "an," or "the" can mean one or more than one. For example, "a" cell can mean a single cell or a multiplicity of cells. Further, the term "a plant" may include a plurality of plants.

As used herein, unless specifically indicated otherwise, the word "or" is used in the inclusive sense of "and/or" and not the exclusive sense of "either/or."

The term "about" or "approximately" usually means within 5%, or more preferably within 1%, of a given value or range.

The terms "comprises," "comprising," "includes," "including," "having" and their conjugates mean "including but not limited to."

Various embodiments of this disclosure may be presented in a range format. It should be noted that whenever a value or range of values of a parameter are recited, it is intended that values and ranges intermediate to the recited values are also part of this disclosure. It should be understood that the description in range format is merely for convenience and brevity and should not be construed as an inflexible limitation on the scope of the disclosure. Accordingly, the description of a range should be considered to have specifically disclosed all the possible subranges as well as individual numerical values within that range. For example, description of a range such as from 1-10 should be considered to have specifically disclosed subranges such as from 1 to 3, from 1 to 4, from 1 to 5, from 1 to 6, from 1 to 7, from 1 to 8, from 1 to 9, from 2 to 4, from 2 to 6, from 2 to 8, from 2 to 10, from 3 to 6, etc., as well as individual numbers within that range, for example, 1, 2, 3, 4, 5, 6, 7, 8, 9 and 10. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals there between. The recitation of a numerical range for a variable is intended to convey that the present disclosure may be practiced with the variable equal to any of the values within that range. Thus, for a variable which is inherently discrete, the variable can be equal to any integer value within the numerical range, including the end-points of the range. Similarly, for a variable which is inherently continuous, the variable can be equal to any real value within the numerical range, including the end-points of the range. As an example, and without limitation, a variable which is described as having values between 0 and 2 can take the values 0, 1 or 2 if the variable is inherently discrete, and can take the values 0.0, 0.1, 0.01, 0.001, or any other real values ≧0 and ≦2 if the variable is inherently continuous.

A "plant" refers to a whole plant, any part thereof, or a cell or tissue culture derived from a plant, comprising any of: whole plants, plant components or organs (e.g., leaves, stems, roots, embryos, pollen, ovules, seeds, leaves, flowers, branches, fruit, pulp, juice, kernels, ears, cobs, husks, stalks, root tips, anthers, etc.), plant tissues, seeds, plant cells, protoplasts and/or progeny of the same. A plant cell is a biological cell of a plant, taken from a plant or derived through culture of a cell taken from a plant. Grain is intended to mean the mature seed produced by commercial growers for purposes other than growing or reproducing the species. Progeny, variants, and mutants of the regenerated plants are also included within the scope of the invention.

As used herein, a "subject plant or plant cell" is one in which genetic alteration, such as a mutation, has been effected as to a gene of interest, or is a plant or plant cell which is descended from a plant or cell so altered and which comprises the alteration. As used herein, the term "mutated" or "genetically modified" or "transgenic" or "transformed" or "edited" plants, plant cells, plant tissues, plant parts or seeds refers plants, plant cells, plant tissues, plant parts or seeds that have been mutated by the methods of the present disclosure to include one or more mutations (e.g., insertions, substitutions, and/or deletions) in the genomic sequence.

As used herein, a "control plant" or "control plant part" or "control cell" or "control seed" refers to a plant or plant part or plant cell or seed that has not been subject to the methods and compositions described herein. A "control" or "control plant" or "control plant part" or "control cell" or "control seed" provides a reference point for measuring changes in phenotype of the subject plant or plant cell. A control plant or plant cell may comprise, for example: (a) a wild-type plant or cell, i.e., of the same genotype as the starting material for the genetic alteration which resulted in the subject plant or cell; (b) a plant or plant cell of the same genotype as the starting material but which has been transformed with a null construct (i.e. with a construct which has no known effect on the trait of interest, such as a construct comprising a marker gene); (c) a plant or plant cell which is a non-transformed segregant among progeny of a subject plant or plant cell; (d) a plant or plant cell genetically identical to the subject plant or plant cell but which is not exposed to conditions or stimuli (e.g., sucrose) that would induce expression of the gene of interest; or (e) the subject plant or plant cell itself, under conditions in which the gene of interest is not expressed. In certain instances, a control plant or plant population of the present disclosure is grown under the same environmental conditions (e.g., same or similar temperature, humidity, air quality, soil quality, water quality, and/or pH conditions) as a subject plant described herein. Similarly, a control protein/oil/seed composition can refer to a protein/oil/seed composition that is isolated or derived from a control plant. In specific embodiments, a control plant, plant part, or plant cell is a plant, plant part, or plant cell that does not have a mutated nucleotide sequence in a *GAS* and/or RS gene or a regulatory region of a *GAS* and/or RS gene.

Plant cells possess nuclear, plastid, and mitochondrial genomes. Accordingly, by "chromosome" or "chromosomal" is intended the nuclear, plastid, or mitochondrial genomic DNA. "Genome" as it applies to plant cells encompasses not only chromosomal DNA found within the nucleus, but organelle DNA found within subcellular components (e.g., mitochondria or plastids) of the cell. The compositions and methods disclosed herein are not limited to mutations made in the genomic DNA of the plant nucleus, but may be used to modify the sequence of the nuclear, plastid, and/or mitochondrial genome, or to modulate the expression of a gene or genes encoded by the nuclear, plastid, and/or mitochondrial genome. In certain embodiments, a mutation is created in the genomic DNA of an organelle (e.g. a plastid and/or a mitochondrion). In certain embodiments, a mutation is created in extrachromosomal nucleic acids (including RNA) of the plant, cell, or organelle of a plant. Nonlimiting examples include creating mutations in supernumerary chromosomes (e.g. B chromosomes), plasmids, and/or vector constructs used to deliver nucleic acids to a plant. It is anticipated that new nucleic acid forms will be developed and yet fall within the scope of the claimed invention when used with the teachings described herein.

As used herein, the term "gene" or "coding sequence," herein used interchangeably, refers to a functional nucleic acid unit encoding a protein, polypeptide, or peptide. As will be understood by those in the art, this functional term includes genomic sequences, cDNA sequences, and smaller engineered gene segments that express, or may be adapted to express proteins, polypeptides, domains, peptides, fusion proteins, and mutants. A gene may include a regulatory region, e.g., a promoter region or a 5'untranslated region, that regulates transcription or translation of the encoded gene. For example, a *"GAS* and/or *RS* gene" includes the coding region of the *GAS* and/or *RS* gene, and may also include the regulatory region (e.g., promoter, 5'UTR) of the *GAS* and/or *RS* gene. Further, a *"GAS* and/or *RS* gene" as used herein includes a homolog of a known a *GAS* and/or *RS* gene.

As used herein, the term a "nucleic acid," used interchangeably with a "nucleotide," refers to a molecule consisting of a nucleoside and a phosphate that serves as a component of DNA or RNA. For instance, nucleic acids include adenine, guanine, cytosine, uracil, and thymine.

As used herein, "allele" refers to an alternative nucleic acid sequence at a particular locus. The length of an allele can be as small as one nucleotide base. For example, a first allele can occur on one chromosome, while a second allele occurs on a second homologous chromosome, e.g., as occurs for different chromosomes of a heterozygous individual, or between different homozygous or heterozygous individuals in a population. "Locus" as used herein refers to a chromosome region or chromosomal region where a polymorphic nucleic acid, trait determinant, gene, or marker is located.

As used herein, a "mutation" is any change in a nucleic acid sequence. Nonlimiting examples comprise insertions, deletions, duplications, substitutions, inversions, and translocations of any nucleic acid sequence, regardless of how the mutation is brought about and regardless of how or whether the mutation alters the functions or interactions of the nucleic acid. For example and without limitation, a mutation may produce altered enzymatic activity of a ribozyme, altered base pairing between nucleic acids (e.g. RNA interference interactions, DNA-RNA binding, etc.), altered mRNA folding stability, and/or how a nucleic acid interacts with polypeptides (e.g. DNA-transcription factor interactions, RNA-ribosome interactions, gRNA-endonuclease reactions, etc.). A mutation might result in the production of proteins with altered amino acid sequences (e.g. missense mutations, nonsense mutations, frameshift mutations, etc.) and/or the production of proteins with the same amino acid sequence (e.g. silent mutations). Certain synonymous mutations may create no observed change in the plant while others that encode for an identical protein sequence nevertheless result in an altered plant phenotype (e.g. due to codon usage bias, altered secondary protein structures, etc.). Mutations may occur within coding regions (e.g., open reading frames) or outside of coding regions (e.g., within promoters, terminators, untranslated elements, or enhancers), and may affect, for example and without limitation, gene expression levels, gene expression profiles, protein sequences, and/or sequences encoding RNA elements such as tRNAs, ribozymes, ribosome components, and microRNAs.

Accordingly, "plant with mutation" or "plant part with mutation" or "plant cell with mutation" or "plant genome with mutation" refers to a plant, plant part, plant cell, or plant genome that contains a mutation (e.g., an insertion, a substitution, or a deletion) described in the present disclosure, such as a mutation in the nucleic acid sequence of a *GAS* and/or *RS* gene or a regulatory region of a *GAS* and/or *RS* gene. For example, as used herein, a plant, plant part, or plant cell with mutation may refer to a plant, plant part, or plant cell in which, or in an ancestor of which, at least one a *GAS* and/or *RS* gene or a regulatory region of the *GAS* and/or *RS* gene has been deliberately mutated such that the plant, plant part or plant cell expresses a mutated (e.g., truncated) GAS and/or RS or have a reduced expression level of the *GAS* and/or *RS* gene or GAS and/or RS. The mutated GAS and/or RS can have altered function, e.g., reduced function or loss-of-function, compared to a corresponding wild-type, or control, GAS and/or RS comprising no mutation.

"Genome editing" or "gene editing" as used herein refers to a type of genetic engineering by which one or more mutations (e.g., insertions, substitutions, deletions, modifications) are introduced at a specific location of the genome.

As used herein, the term "recombinant DNA construct," "recombinant construct," "expression cassette," "expression construct," "chimeric construct," "construct," and "recombinant DNA fragment" are used interchangeably herein and are single or double-stranded polynucleotides. A recombinant construct comprises an artificial combination of nucleic acid fragments, including, without limitation, regulatory and coding sequences that are not found together in nature. For example, a recombinant DNA construct may comprise regulatory sequences and coding sequences that are derived from different sources, or regulatory sequences and coding sequences derived from the same source and arranged in a manner different than that found in nature. Such a construct may be used by itself or may be used in conjunction with a vector.

An expression construct can permit transcription of a particular nucleic acid sequence in a host cell (e.g., a bacterial cell or a plant cell). An expression cassette may be part of a plasmid, viral genome, or nucleic acid fragment. Typically, an expression cassette includes a polynucleotide to be transcribed, operably linked to a promoter. "Operably linked" is intended to mean a functional linkage between two or more elements. For example, an operable linkage between a promoter of and a nucleic acid molecule is a functional link that allows for expression of the nucleic acid molecule. Operably linked elements may be contiguous or non-contiguous. When used to refer to the joining of two protein coding regions, by operably linked is intended that the coding regions are in the same reading frame. The cassette may additionally contain at least one additional gene to be co-transformed into the plant. Alternatively, the additional gene(s) can be provided on multiple expression cassettes or DNA constructs. The expression cassette may additionally contain selectable marker genes. Other elements that may be present in an expression cassette include those that enhance transcription (e.g., enhancers) and terminate transcription (e.g., terminators), as well as those that confer certain binding affinity or antigenicity to the recombinant protein produced from the expression cassette.

As used herein, "function" of a gene, a peptide, a protein, or a molecule refers to activity of a gene, a peptide, a protein, or a molecule.

"Introduced" in the context of inserting a nucleic acid molecule (e.g., a recombinant DNA construct) into a cell, means "transfection" or "transformation" or "transduction" and includes reference to the incorporation of a nucleic acid fragment into a plant cell where the nucleic acid fragment may be incorporated into the genome of the cell (e.g., nuclear chromosome, plasmid, plastid chromosome or mitochondrial chromosome), converted into an autonomous replicon, or transiently expressed (e.g., transfected mRNA).

As used herein with respect to a parameter, the term "increased" or "increasing" or "increase" refers to a detectable (e.g., at least about 5%, 10%, 15%, 20%, 25%, 30%, 35%, 40%, 45%, 50%, 55%, 60%, 65%, 70%, 75%, 80%, 85%, 90%, 95%, 96%, 97%, 98%, 99%, 100%, 120%, 150%, 200%, 300%, 400%, 500%, or more) positive change in the parameter from a comparison control, e.g., an established normal or reference level of the parameter, or an established standard control. Accordingly, the terms "increased," "increase," and the like encompass both a partial increase and a significant increase compared to a control.

As used herein with respect to a parameter, the term "decreased" or "decreasing" or "decrease" or "reduced" or "reducing" or "reduce" or "lower" or "loss" refers to a detectable (e.g., at least about 5%, 10%, 15%, 20%, 25%, 30%, 35%, 40%, 45%, 50%, 55%, 60%, 65%, 70%, 75%, 80%, 85%, 90%, 95%, 96%, 97%, 98%, 99%, or 100%) negative change in the parameter from a comparison control, e.g., an established normal or reference level of the parameter, or an established standard control. Accordingly, the terms "decreased," "reduced," and the like encompass both a partial reduction and a complete reduction compared to a control.

When reference is made to particular sequence listings, such reference is to be understood to also encompass sequences that substantially correspond to its complementary sequence as including minor sequence variations, resulting from, e.g., sequencing errors, cloning errors, or other alterations resulting in base substitution, base deletion or base addition, provided that the frequency of such variations is less than 1 in 50 nucleotides, alternatively, less than 1 in 100 nucleotides, alternatively, less than 1 in 200 nucleotides, alternatively, less than 1 in 500 nucleotides, alternatively, less than 1 in 1000 nucleotides, alternatively, less than 1 in 5,000 nucleotides, alternatively, less than 1 in 10,000 nucleotides.

As used herein, the term "polypeptide" refers to a linear organic polymer containing a large number of amino-acid residues bonded together by peptide bonds in a chain, forming part of (or the whole of) a protein molecule. The amino acid sequence of the polypeptide refers to the linear consecutive arrangement of the amino acids comprising the polypeptide, or a portion thereof.

As used herein the terms "polynucleotide," "polynucleotide sequence," "nucleic acid sequence," and "nucleic acid fragment" are used interchangeably and refer to a single or double stranded nucleic acid sequence which is isolated and provided in the form of an RNA sequence (e.g., an mRNA sequence), a complementary nucleic acid sequence (cDNA), a genomic nucleic acid sequence, a synthetic nucleic acid sequence, and/or a composite nucleic acid sequences (e.g., a combination of the above). The polynucleotides provided herein encompass all forms of sequences including, but not limited to, single-stranded forms, double-stranded forms, hairpins, stem-and-loop structures, and the like.

The term "isolated" refers to at least partially separated from the natural environment e.g., from a plant cell.

As used herein, the term "expression" or "expressing" refers to the transcription and/or translation of a particular nucleic acid sequence driven by a promoter.

As used herein, the terms "exogenous" or "heterologous" in reference to a nucleic acid sequence or amino acid sequence are intended to mean a sequence that is purely synthetic, that originates from a foreign species, or, if from the same species, is substantially modified from its native form in composition and/or genomic locus by deliberate human intervention. Thus, a heterologous nucleic acid sequence may not be naturally expressed within the plant (e.g., a nucleic acid sequence from a different species) or may have altered expression when compared to the corresponding wild type plant. An exogenous polynucleotide may be introduced into the plant in a stable or transient manner, so as to produce a ribonucleic acid (RNA) molecule and/or a polypeptide molecule. It should be noted that the exogenous polynucleotide may comprise a nucleic acid sequence which is identical or partially homologous to an endogenous nucleic acid sequence of the plant.

As used herein, by "endogenous" in reference to a gene or nucleic acid sequence or protein is intended a gene or nucleic acid sequence or protein that is naturally comprised within or expressed by a cell. Endogenous genes can include genes that naturally occur in the cell of a plant, but that have been modified in the genome of the cell without insertion or replacement of a heterologous gene that is from another plant species or another location within the genome of the modified cell.

As used herein, "fertilization" and/or "crossing" broadly includes bringing the genomes of gametes together to form zygotes but also broadly may include pollination, syngamy, fecundation and other processes related to sexual reproduction. Typically, a cross and/or fertilization occurs after pollen is transferred from one flower to another, but those of ordinary skill in the art will understand that plant breeders can leverage their understanding of fertilization and the overlapping steps of crossing, pollination, syngamy, and fecundation to circumvent certain steps of the plant life cycle and yet achieve equivalent outcomes, for example, a plant or cell of a pea cultivar described herein. In certain embodiments, a user of this innovation can generate a plant of the claimed invention by removing a genome from its host gamete cell before syngamy and inserting it into the nucleus of another cell. While this variation avoids the unnecessary steps of pollination and syngamy and produces a cell that may not satisfy certain definitions of a zygote, the process falls within the definition of fertilization and/or crossing as used herein when performed in conjunction with these teachings. In certain embodiments, the gametes are not different cell types (i.e. egg vs. sperm), but rather the same type and techniques are used to effect the combination of their genomes into a regenerable cell. Other embodiments of fertilization and/or crossing include circumstances where the gametes originate from the same parent plant, i.e. a "self' or "self-fertilization." While selfing a plant does not require the transfer of pollen from one plant to another, those of skill in the art will recognize that it nevertheless serves as an example of a cross, just as it serves as a type of fertilization. Thus, methods and compositions taught herein are not limited to certain techniques or steps that must be performed to create a plant or an offspring plant of the claimed invention, but rather include broadly any method that is substantially the same and/or results in compositions of the claimed invention.

"Homolog" or "homologous sequence" may refer to both orthologous and paralogous sequences. Paralogous sequence relates to gene-duplications within the genome of a species. Orthologous sequence relates to homologous genes in different organisms due to ancestral relationship. Thus, orthologs are evolutionary counterparts derived from a single ancestral gene in the last common ancestor of given two species and therefore have great likelihood of having the same function. One option to identify homologs (e.g., orthologs) in monocot plant species is by performing a reciprocal BLAST search. This may be done by a first blast involving blasting the sequence-of-interest against any sequence database, such as the publicly available NCBI database which may be found at: ncbi.nlm.nih.gov. If orthologs in rice were sought, the sequence-of-interest would be blasted against, for example, the 28,469 full-length cDNA clones from *Oryza sativa* Nipponbare available at NCBI. The blast results may be filtered. The full-length sequences of either the filtered results or the non-filtered results are then blasted back (second blast) against the sequences of the organism from which the sequence-of-interest is derived. The results of the first and second blasts are then compared. An ortholog is identified when the sequence resulting in the highest score (best hit) in the first blast identifies in the second blast the query sequence (the original sequence-of-interest) as the best hit. Using the same rational a paralog (homolog to a gene in the same organism) is found. In case of large sequence families, the ClustalW program may be used [ebi.ac.uk/Tools/clustalw2/index.html], followed by a neighbor-joining tree (wikipedia.org/wiki/Neighbor-joining) which helps visualizing the clustering.

In some embodiments, the term "homolog" as used herein, refers to functional homologs of genes. A functional homolog is a gene encoding a polypeptide that has sequence similarity to a polypeptide encoded by a reference gene, and the polypeptide encoded by the homolog carries out one or more of the biochemical or physiological function(s) of the polypeptide encoded by the reference gene. In general, it is preferred that functional homologs and/or polypeptides encoded by functional homologs share at least some degree of sequence identity with the reference gene or polypeptide encoded by the reference gene.

Homology (e.g., percent homology, sequence identity+sequence similarity) can be determined using any homology comparison software computing a pairwise sequence alignment.

As used herein, "sequence identity," "identity," "percent identity," "percentage similarity," "sequence similarity" and the like refer to a measure of the degree of similarity of two sequences based upon an alignment of the sequences that maximizes similarity between aligned amino acid residues or nucleotides, and which is a function of the number of identical or similar residues or nucleotides, the number of total residues or nucleotides, and the presence and length of gaps in the sequence alignment. A variety of algorithms and computer programs are available for determining sequence similarity using standard parameters. As used herein, sequence similarity is measured using the BLASTp program for amino acid sequences and the BLASTn program for nucleic acid sequences, both of which are available through the National Center for Biotechnology Information (www.ncbi.nlm.nih.gov/), and are described in, for example, Altschul et al. (1990), J. Mol. Biol. 215:403-410; Gish and States (1993), Nature Genet. 3:266-272; Madden et al. (1996), Meth. Enzymol.266:131-141; Altschul et al. (1997), Nucleic Acids Res. 25:3389-3402); Zhang et al. (2000), J. Comput. Biol. 7(1-2):203-14. As used herein, percent similarity of two amino acid sequences is the score based upon the following parameters for the BLASTp algorithm: word size=3; gap opening penalty=-11; gap extension penalty=-1; and scoring matrix=BLOSUM62. As used herein, percent similarity of two nucleic acid sequences is the score based upon the following parameters for the BLASTn algorithm: word size=11; gap opening penalty=-5; gap extension penalty=-2; match reward=1; and mismatch penalty=-3. When percentage of sequence identity is used in reference to proteins it is recognized that residue positions which are not identical often differ by conservative amino acid substitutions, where amino acid residues are substituted for other amino acid residues with similar chemical properties (e.g. charge or hydrophobicity) and therefore do not change the functional properties of the molecule. Where sequences differ in conservative substitutions, the percent sequence identity may be adjusted upwards to correct for the conservative nature of the substitution. Sequences which differ by such conservative substitutions are considered to have "sequence similarity" or "similarity." Means for making this adjustment are well-known to those of skill in the art. Typically this involves scoring a conservative substitution as a partial rather than a full mismatch, thereby increasing the percentage sequence identity. Thus, for example, where an identical amino acid is given a score of 1 and a non-conservative substitution is given a score of zero, a conservative substitution is given a score between zero and 1. The scoring of conservative substitutions is calculated, e.g., according to the algorithm of Henikoff S and Henikoff J G. (Proc Natl Acad Sci 89:10915-9 (1992)). Identity (e.g., percent homology) can be determined using any homology comparison software, including for example, the BlastN software of the National Center of Biotechnology Information (NCBI) such as by using default parameters.

According to some embodiments, the identity is a global identity, i.e., an identity over the entire amino acid or nucleic acid sequences of the invention and not over portions thereof.

According to some embodiments, the term "homology" or "homologous" refers to identity of two or more nucleic acid sequences; or identity of two or more amino acid sequences; or the identity of an amino acid sequence to one or more nucleic acid sequence. According to some embodiments, the homology is a global homology, e.g., a homology over the entire amino acid or nucleic acid sequences of the invention and not over portions thereof. The degree of homology or identity between two or more sequences can be determined using various known sequence comparison tools which are described in WO2014/102774.

As used herein, the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "population" refers to a set comprising any number, including one, of individuals, objects, or data from which samples are taken for evaluation.

The term can relate to a plurality of plants contacted with a mutagen or gene editing reagents, and introduced with a mutation. In such a case, not all plants of the population may carry the mutation, but a certain percentage or more of the population carry the mutation. The term can also relate to a breeding population of plants from which members are selected and crossed to produce progeny in a breeding program. A population of plants can include the progeny of a single breeding cross or a plurality of breeding crosses and can be either actual plants or plant derived material, or in silico representations of plants. The member of a population need not be identical to the population members selected for use in subsequent cycles of analyses, nor does it need to be identical to those population members ultimately selected to obtain a final progeny of plants. Often, a plant population is derived from a single biparental cross but can also derive from two or more crosses between the same or different parents. Although a population of plants can comprise any number of individuals, those of skill in the art will recognize that plant breeders commonly use population sizes ranging from one or two hundred individuals to several thousand, and that the highest performing 5-20% of a population is what is commonly selected to be used in subsequent crosses in order to improve the performance of subsequent generations of the population in a plant breeding program.

As used herein, the term "crop performance" is used synonymously with "plant performance" and refers to of how well a plant grows under a set of environmental conditions and cultivation practices. Crop performance can be measured by any metric a user associates with a crop's productivity (e.g., yield), appearance and/or robustness (e.g., color, morphology, height, biomass, maturation rate, etc.), product quality (e.g., fiber lint percent, fiber quality, seed protein content, seed carbohydrate content, etc.), cost of goods sold (e.g., the cost of creating a seed, plant, or plant product in a commercial, research, or industrial setting) and/or a plant's tolerance to disease (e.g., a response associated with deliberate or spontaneous infection by a pathogen) and/or environmental stress (e.g., drought, flooding, low nitrogen or other soil nutrients, wind, hail, temperature, day length, etc.). Crop performance can also be measured by determining a crop's commercial value and/or by determining the likelihood that a particular inbred, hybrid, or variety will become a commercial product, and/or by determining the likelihood that the offspring of an inbred, hybrid, or variety will become a commercial product. Crop performance can be a quantity (e.g., the volume or weight of seed or other plant product measured in liters or grams) or some other metric assigned to some aspect of a plant that can be represented on a scale (e.g., assigning a 1-10 value to a plant based on its disease tolerance).

A "microbe" will be understood to be a microorganism, i.e. a microscopic organism, which can be single celled or multicellular. Microorganisms are very diverse and include all the bacteria, archaea, protozoa, fungi, and algae, especially cells of plant pathogens and/or plant symbionts. Certain animals are also considered microbes, e.g. rotifers. In various embodiments, a microbe can be any of several different microscopic stages of a plant or animal. Microbes also include viruses, viroids, and prions, especially those which are pathogens or symbionts to crop plants. A "pathogen" as used herein refers to a microbe that causes disease or harmful effects on plant health.

A "fungus" includes any cell or tissue derived from a fungus, for example whole fungus, fungus components, organs, spores, hyphae, mycelium, and/or progeny of the same. A fungus cell is a biological cell of a fungus, taken from a fungus or derived through culture of a cell taken from a fungus.

A "pest" is any organism that can affect the performance of a plant in an undesirable way. Common pests include microbes, animals (e.g. insects and other herbivores), and/or plants (e.g. weeds). Thus, a pesticide is any substance that reduces the survivability and/or reproduction of a pest, e.g. fungicides, bactericides, insecticides, herbicides, and other toxins.

"Tolerance" or "improved tolerance" in a plant to disease conditions (e.g. growing in the presence of a pest) will be understood to mean an indication that the plant is less affected by the presence of pests and/or disease conditions with respect to yield, survivability and/or other relevant agronomic measures, compared to a less tolerant, more "susceptible" plant. Tolerance is a relative term, indicating that a "tolerant" plant survives and/or performs better in the presence of pests and/or disease conditions compared to other (less tolerant) plants (e.g., a different cultivar) grown in similar circumstances. As used in the art, "tolerance" is sometimes used interchangeably with "resistance," although resistance is sometimes used to indicate that a plant appears maximally tolerant to, or unaffected by, the presence of disease conditions. Plant breeders of ordinary skill in the art will appreciate that plant tolerance levels vary widely, often representing a spectrum of more-tolerant or less-tolerant phenotypes, and are thus trained to determine the relative tolerance of different plants, plant lines or plant families and recognize the phenotypic gradations of tolerance.

"Yield" as used herein is defined as the measurable produce of economic value from a crop. This may be defined in terms of quantity and/or quality. Yield is directly dependent on several factors, for example, the number and size of the organs, plant architecture (for example, the number of branches), seed production, leaf senescence and more. Root development, nutrient uptake, stress tolerance, photosynthetic carbon assimilation rates, and early vigor may also be important factors in determining yield. Optimizing the abovementioned factors may therefore contribute to increasing crop yield. Yield can be measured and expressed by any means known in the art. In specific embodiments, yield is measured by seed weight or volume in a given harvest area.

A plant, or its environment, can be contacted with a wide variety of "agriculture treatment agents." As used herein, an "agriculture treatment agent," or "treatment agent," or "agent" can refer to any exogenously provided compound that can be brought into contact with a plant tissue (e.g. a seed) or its environment that affects a plant's growth, development and/or performance, including agents that affect other organisms in the plant's environment when those effects subsequently alter a plant's performance, growth, and/or development (e.g. an insecticide that kills plant pathogens in the plant's environment, thereby improving the ability of the plant to tolerate the insect's presence). Agriculture treatment agents also include a broad range of chemicals and/or biological substances that are applied to seeds, in which case they are commonly referred to as seed treatments and/or seed dressings. Seed treatments are commonly applied as either a dry formulation or a wet slurry or liquid formulation prior to planting and, as used herein, generally include any agriculture treatment agent including growth regulators, micronutrients, nitrogen-fixing microbes, and/or inoculants. Agriculture treatment agents include pesticides (e.g. fungicides, insecticides, bactericides, etc.) hormones (abscisic acids, auxins, cytokinins, gibberellins, etc.) herbicides (e.g. glyphosate, atrazine, 2,4-D, dicamba, etc.), nutrients (e.g. a plant fertilizer), and/or a broad range of biological agents, for example a seed treatment inoculant comprising a microbe that improves crop performance, e.g. by promoting germination and/or root development. In certain embodiments, the agriculture treatment agent acts extracellularly within the plant tissue, such as interacting with receptors on the outer cell surface. In some embodiments, the agriculture treatment agent enters cells within the plant tissue. In certain embodiments, the agriculture treatment agent remains on the surface of the plant and/or the soil near the plant. In certain embodiments, the agriculture treatment agent is contained within a liquid. Such liquids include, but are not limited to, solutions, suspensions, emulsions, and colloidal dispersions. In some embodiments, liquids described herein will be of an aqueous nature. However, in various embodiments, such aqueous liquids that comprise water can also comprise water insoluble components, can comprise an insoluble component that is made soluble in water by addition of a surfactant, or can comprise any combination of soluble components and surfactants. In certain embodiments, the application of the agriculture treatment agent is controlled by encapsulating the agent within a coating, or capsule (e.g. microencapsulation). In certain embodiments, the agriculture treatment agent comprises a nanoparticle and/or the application of the agriculture treatment agent comprises the use of nanotechnology.

In certain embodiments, plants disclosed herein can be modified to exhibit at least one desired trait. The disclosed innovations are not limited to any set of traits that can be considered desirable, but nonlimiting examples include low RFO (e.g., raffinose, stachyose) content, high sucrose content, male sterility, herbicide tolerance, pest tolerance, disease tolerance, modified fatty acid metabolism, modified carbohydrate metabolism, modified seed yield, modified seed oil, modified seed protein, modified lodging resistance, modified shattering, modified iron-deficiency chlorosis, modified water use efficiency, and/or combinations thereof. Desired traits can also include traits that are deleterious to plant performance, for example, when a researcher desires that a plant exhibits such a trait in order to study its effects on plant performance.

In certain embodiments, a user can combine the teachings herein with high-density molecular marker profiles spanning substantially the entire plant genome (e.g., pea genome) to estimate the value of selecting certain candidates in a breeding program in a process commonly known as genomic selection.

The patent and scientific literature referred to herein establishes knowledge that is available to those of skill in the art. The issued US patents, allowed applications, published foreign applications, and references, including GenBank database sequences, which are cited herein are hereby incorporated by reference to the same extent as if each was specifically and individually indicated to be incorporated by reference.

All publications, patent applications, patents, and other references mentioned herein are incorporated by reference herein in their entirety.

### II. Overview of the Invention

Raffinose family oligosaccharides (RFOs) are α-1, 6-galactosyl derivatives of sucrose. Representative RFOs include raffinose, stachyose, verbascose, and ciceritol. RFOs are found in plants, abundant in the seeds, roots, and specialized storage organs such as tubers and leaves of many crops, including legumes. RFOs are poorly digested by monogastric animals due to the lack of α-galactosidase activity in the gut of the monogastric animals, including pigs, dogs, chickens, and humans. Decreased RFO content in plants, plant parts, and plant products can increase the metabolizable energy of the diet and/or reduce flatulence production, and can be an advantageous trait in the growing markets of food, beverages, and feed.

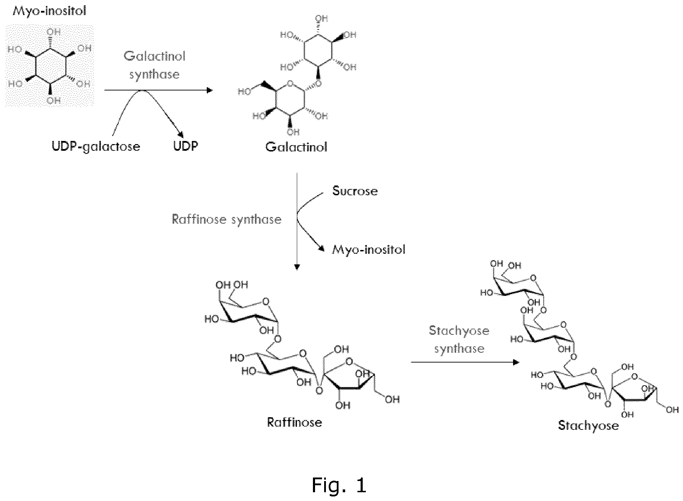

FIG. 1 depicts part of an example RFO biosynthesis pathway. In the galactinol-dependent RFO synthesis pathway, galactinol synthase (GAS) catalyzes the first and rate limiting step of RFO biosynthesis. GAS catalyzes the transfer of a galactosyl moiety from UDP-galactose to myo-inositol, forming galactinol. In pea, *Pisat. 03G015560* and *Pisat.06G066360* have been identified as *GAS* genes. In soybean, *Glyma.19G227800* and *Glyma.03G229800* have been identified as GAS genes.

The next step is catalyzed by the raffinose synthase (RS), which transfers a galactosyl moiety from galactinol to sucrose, forming raffinose, the first RFO in the biosynthetic pathway. Raffinose synthase (RS) control the accumulation of the RFOs. In pea, *Pisat.05G054130* and *Pisat.01G021000* have been identified as *RS* genes.

Following the formation of raffinose, larger RFOs (e.g., stachyose and verbascose) can be formed. The formation of stachyose is catalyzed by stachyose synthase, similar to the formation of raffinose, by a galactosyl moiety being transferred from galactinol. During this reaction, raffinose is the acceptor instead of sucrose. The verbascose formation is also similar, except stachyose is now the galactosyl acceptor. In peas, stachyose synthase is used as a multi-functional enzyme in synthesizing both stachyose and verbascose. Accordingly, decreasing the activity of GAS and/or RS in a plant or plant part can be one approach to generate advantageous traits, such as decreased RFO (e.g., raffinose, stachyose) content, and/or increased sucrose content. In addition to their role in RFO biosynthesis, GAS and/or RS may also play a role in regulating protein content in plants or plant parts (e.g., seeds).

Disclosed herein are plants or plant parts comprising a genetic mutation that increases GAS and/or RS activity compared to a control plant or plant part, as well as methods for making the plants or plant parts with decreased GAS and/or RS activity. Such plants or plant parts can have one or more insertions, substitutions, or deletions in at least one native (e.g., wild-type) *GAS* and/or *RS* gene or homolog thereof or in its regulatory region. The plants or plant parts can have a reduced expression of the *GAS* and/or *RS* gene or homolog thereof, reduced level or activity of a GAS and/or RS protein encoded by the *GAS* and/or *RS* gene or homolog thereof, decreased RFO (e.g., raffinose, stachyose) content, increased sucrose content, and/or increased protein content compared to a plant or plant part without the mutation.

Also disclosed herein are compositions and methods for producing plants, plant parts, or a population of plants or plant parts having decreased GAS and/or RS activity. One approach for decreasing GAS and/or RS activity is to introduce a genetic mutation that reduces GAS and/or RS activity. The methods disclosed herein can include introducing one or more insertions, substitutions, or deletions in at least one a *GAS* and/or *RS* gene or homolog thereof or regulatory region thereof in the genome of a plant, plant part, or plant cell, such that an expression level of the *GAS* and/or *RS* gene or homolog thereof is reduced, level or activity of a GAS and/or RS protein encoded by the *GAS* and/or *RS* gene or homolog thereof is reduced, RFO (e.g., raffinose, stachyose) content is reduced, sucrose content is increased, and/or protein content is increased in the plant, plant part, or plant cell compared to a plant, plant part, or plant cell without the mutation. The methods of the present disclosure can include contacting the plant or plant part with a mutagen, or introducing editing reagents (e.g., nuclease, guide RNA) into the plants or plant parts to introduce a mutation that reduces GAS and/or RS activity in a genome of the plant or plant part.

Also disclosed herein are a population of plants or plant parts (e.g., seeds) and plant products (e.g., seed compositions, protein and/or oil compositions, food and beverage products) produced from the plants, plant parts, or population of plants or plant parts of the present disclosure, having reduced GAS and/or RS activity, mutation in a *GAS* and/or *RS* gene or homolog thereof or regulatory region thereof, decreased RFO (e.g., raffinose, stachyose) content, increased sucrose content, and/or increased protein content compared to a control.

Further provided herein are nucleic acid molecules comprising a mutated *GAS* and/or *RS* gene or its regulatory region (e.g., mutated promoter or 5' UTR) with reduced GAS and/or RS activity, a DNA construct comprising the mutated *GAS* and/or *RS* gene operably linked to a functional promoter, and cells comprising the nucleic acid molecule or the DNA construct of the present disclosure.

### III. Plants with Reduced GAS and/or RS Activity

Plants and plant parts are provided herein having altered (e.g., reduced) GAS and/or RS level or activity as compared to a control plant or plant part. As used herein, "GAS activity" refers to an enzymatic activity to catalyze production of galactinol, e.g., by transferring a galactosyl moiety from UDP-galactose to myo-inositol. As used herein, "RS activity" refers to an enzymatic activity to catalyze production of raffinose, e.g., by transferring a galactosyl moiety from galactinol to sucrose. In particular aspects, plants and plant parts (e.g., seeds, leaves) disclosed herein have a genetic mutation that alters (e.g., increases) the GAS and/or RS activity. The plants or plant parts described herein having altered GAS and/or RS level or activity can comprise a genetic mutation or transgene that alters (e.g., reduces) GAS and/or RS level or activity, altered (e.g., reduced) expression levels of at least one a *GAS* and/or *RS* gene encoding GAS and/or RS, altered (e.g., increased) GAS and/or RS levels or activity, and/or altered (e.g., increased) protein content compared to a control plant or plant part.

Also provided herein is a population of plants and plant parts comprising the plants and plant parts described herein having altered (e.g., reduced) GAS and/or RS level or activity. In such population of plants or plant parts, having altered GAS and/or RS level or activity relative to a control population, not all individual plants or plant parts need to have altered (e.g., reduced) GAS and/or RS level or activity, genetic mutation that cause altered (e.g., reduced) GAS and/or RS level or activity, or phenotypes caused by the altered (e.g., reduced) GAS and/or RS activity (e.g., decreased RFO (e.g., raffinose, stachyose) content, increased sucrose content, and/or increased protein content, altered protein metabolism). In specific embodiments at least 80%, 81%, 82%, 83%, 84%, 85%, 86%, 87%, 88%, 89%, 90%, 91%, 92%, 93%, 94%, 95%, 96%, 97%, 98%, 99%, or more plants within a given plant population have a mutation that alters the GAS and/or RS level or activity.

The teachings herein are not limited to certain plant species, and it is envisioned that they can be modified to be useful for monocots, dicots, and/or substantially any crop and/or valuable plant type, including plants that can reproduce by self-fertilization and/or cross fertilization, hybrids, inbreds, varieties, and/or cultivars thereof. A plant or plant part of the present disclosure can be a legume, i.e., a plant belonging to the family *Fabaceae* (or *Leguminosae*), or a part (e.g., fruit or seed) of such a plant. When used as a dry grain, the seed of a legume is also called a pulse. Examples of legume include, without limitation, pea *(Pisum sativum),* soybean *(Glycine max),* beans (*Phaseolus* spp., *Vigna* spp.), common bean (*Phaseolus vulgaris*), mung bean (*Vigna radiata*), cowpea (*Vigna unguiculata*), adzuki bean (*Vigna angularis*), fava bean (*Viciafaba*), chickpea (*Cicer arietinum*), peanut *(Arachis hypogaea*), lentils (*Lens culinaris*, *Lens esculenta*), lupins (*Lupinus* spp.), white lupin (*Lupinus albus*), mesquite (*Prosopis* spp.), carob (*Ceratonia siliqua*), tamarind (*Tamarindus indica*), alfalfa (*Medicago sativa*), barrel medic (*Medicago truncatula*), birdsfood trefoil (*Lotus japonicus*), licorice (*Glycyrrhiza glabra*), and clover (*Trifolium* spp.). For example, a plant or plant part of the present disclosure can be *Glycine max* or a part of *Glycine max.* Additionally, a plant or plant part of the present disclosure can be a crop plant or part of a crop plant, including legumes. Examples of crop plants include, but are not limited to, corn (*Zea mays*), *Brassica* sp. (e.g., *B. napus, B. rapa, B. juncea*), particularly those *Brassica* species useful as sources of seed oil, pea *(Pisum sativum),* alfalfa (*Medicago sativa*), rice (*Oryza sativa*), rye (*Secale cereale*), sorghum (*Sorghum bicolor*, *Sorghum vulgare*), camelina (*Camelina sativa*), millet (e.g., pearl millet (*Pennisetum glaucum*), proso millet (*Panicum miliaceum*), foxtail millet (*Setaria italica*), finger millet (*Eleusine coracana*))*,* sunflower (*Helianthus annuus*), quinoa (*Chenopodium quinoa*), chicory (*Cichorium intybus*), lettuce (*Lactuca sativa*), safflower (*Carthamus tinctorius*), wheat (*Triticum aestivum*), soybean (*Glycine max*), tobacco (*Nicotiana* spp., e.g., *Nicotiana tabacum*, *Nicotiana sylvestris*), potato (*Solanum tuberosum*), tomato (*Solanum lycopersicum*), peanuts *(Arachis hypogaea*), cotton (*Gossypium barbadense*, *Gossypium hirsutum*), sweet potato (*Ipomoea batatus*), cassava (*Manihot esculenta*), coffee (*Coffea* spp.), coconut (*Cocos nucifera*), pineapple (*Ananas comosus*), citrus trees (*Citrus* spp.), cocoa (*Theobroma cacao*), tea (*Camellia sinensis*), banana (*Musa* spp.), avocado (*Persea americana*), fig (*Ficus casica*), guava (*Psidium guajava*), mango (*Mangifera indica*), grapes (*Vitis vinifera*, *Vitis riparia*), olive (*Olea europaea*), papaya (*Carica papaya*), cashew (*Anacardium occidentale*), macadamia (*Macadamia integrifolia*), almond (*Prunus amygdalus*), sugar beets (*Beta vulgaris*), sugarcane (*Saccharum* spp.), oil palm (*Elaeis guineensis*), poplar (*Populus* spp.), eucalyptus (*Eucalyptus* spp.), oats (*Avena sativa*), barley (*Hordeum vulgare*), vegetables, ornamentals, and conifers. Additionally, a plant or plant part of the present disclosure can be an oilseed plant (e.g., canola (*Brassica napus*), cotton (*Gossypium* sp.), camelina (*Camelina sativa*) and sunflower (*Helianthus* sp.)), or other species including wheat (*Triticum* sp., such as *Triticum aestivum* L. ssp. *Aestivum* (common or bread wheat), other subspecies of *Triticum aestivum, Triticum turgidum* L. ssp. Durum (durum wheat, also known as macaroni or hard wheat), *Triticum monococcum* L. ssp. *Monococcum* (cultivated einkorn or small spelt), *Triticum timopheevi* ssp. *Timopheevi*, *Triticum turgigum* L. ssp. Dicoccon (cultivated emmer), and other subspecies of *Triticum turgidum* (Feldman)), barley (*Hordeum vulgare*), maize (*Zea mays*), oats (*Avena sativa*), or hemp (*Cannabis sativa*). Additionally, a plant or plant part of the present disclosure can be a forage plant or part of a forage plant. Examples of forage plants include legumes and crop plants described herein as well as grass forages including *Agrostis* spp., *Lolium* spp., *Festuca* spp., *Poa* spp., and *Bromus* spp.

### A. Plants with altered level or activity of GAS and/or RS

Provided herein are plants or plant parts (e.g., seeds) comprising altered (e.g., decreased) galactinol synthase (GAS) and/or raffinose synthase (RS) activity compared to a control plant or plant part. Also provided herein is a population of plants or plant parts (e.g., seeds) comprising altered (e.g., reduced) activity of GAS and/or RS compared to a control population provided herein.

The genetic mutation that alters (e.g., decreases) the GAS and/or RS activity in the plants and plant parts provided herein can comprise one or more insertions, substitutions, or deletions in at least one native *GAS* and/or *RS* gene or homolog thereof, or in a regulatory region of at least one native *GAS* and/or *RS* gene or homolog thereof. The genetic mutation that alters (e.g., decreases) the GAS and/or RS activity can be located in at least one native *GAS* and/or *RS* gene or homolog thereof; in a regulatory region of the native *GAS* and/or *RS* gene or homolog thereof; a coding region, a non-coding region, or a regulatory region of any other gene; or at any other site in the genome of the plant or plant part.

A "native" gene, as used herein, refers to any gene having a wild-type nucleic acid sequence, e.g., a nucleic acid sequence that can be found in the genome of a plant existing in nature, and need not naturally occur within the plant, plant part, or plant cell comprising such native gene. For example, a transgenic *GAS* and/or *RS* gene located at a genomic site or in a plant in a non-naturally occurring matter is a "native" *GAS* and/or *RS* gene if its nucleic acid sequence can be found in a plant existing in nature.

A "regulatory region" of a gene, as used herein, refers to the region of a genome that controls expression of the gene. A regulatory region of a gene can include a genomic site where a RNA polymerase, a transcription factor, or other transcription modulators bind and interact to control mRNA synthesis of the gene, such as promoter regions, binding sites for transcription modulator proteins, and other genomic regions that contribute to regulation of transcription of the gene. A regulatory region of the gene can be located in the 5' untranslated region of the gene.

A control plant or plant part can be a plant or plant part to which a mutation provided herein has not been introduced, e.g., by methods of the present disclosure. Thus, a control plant or plant part (e.g., seeds, leaves) may express a native (e.g., wild-type) *GAS* and/or *RS* gene endogenously or transgenically. A control plant of the present disclosure may be grown under the same environmental conditions (e.g., same or similar temperature, humidity, air quality, soil quality, water quality, and/or pH conditions) as a plant with the mutation described herein. A plant, plant part (e.g., seeds, leaves), or a population of plants or plant parts of the present disclosure may have altered (e.g., decreased) expression levels of at least one *GAS* and/or *RS* gene or homolog thereof, altered (e.g., decreased) GAS and/or RS level or activity, and/or altered (e.g., increased) protein content as compared to a control plant, plant part, or population, when the plant, plant part, or population of plants or plant parts of the present disclosure is grown under the same environmental conditions as the control plant or plant part.

### 1. Plants with one or more mutations in at least one a GAS and/or RS gene, or its homolog, ortholog, or variant

In some aspects, the plants and plant parts of the present disclosure comprise decreased GAS and/or RS activity and a genetic mutation that decreases the GAS and/or RS activity. The genetic mutation can comprise one or more (e.g., about 1, 2, 3, 4, 5, 6, 7, 8, 9, 10, 11, 12, 13, 14, 15, 16, 17, 18, 19, 20, or more) insertions, substitutions, and/or deletions in at least one native *GAS* and/or *RS* gene or homolog thereof and/or in a regulatory region of said at least one native *GAS* and/or *RS* gene or homolog thereof in a genome of said plant or plant part. A plant or plant part described herein can comprise 1-2, 1-3, 1-4, 1-5, 2-5, 3-5, 4-5 (e.g., 1, 2, 3, 4, or 5) copies of *GAS* and/or *RS* gene, each encoding a GAS and/or RS. In particular, a plant or plant part described herein can comprise at least 2 genes encoding a GAS and/or RS, such as 2, 3, 4, or 5 genes that have less than 100% (e.g., less than 100%, 99%, 98%, 97%, 96%, 95%, 94%, 93%, 92%, 91%, 90%, 89%, 88%, 87%, 86%, or 85%) sequence identity to one another. The plant or plant part described herein can comprise one or more (e.g., about 1, 2, 3, 4, 5, 6, 7, 8, 9, 10, 11, 12, 13, 14, 15, 16, 17, 18, 19, 20, or more) insertions, substitutions, and/or deletions: in one *GAS* and/or *RS* gene or homolog; in a regulatory region of one *GAS* and/or *RS* gene or homolog; in more than one (e.g., 2, 3, 4, 5, 6, 7, 8, 9, 10), but not all *GAS* and/or *RS* genes or homologs; in regulatory regions of more than one (e.g., 2, 3, 4, 5, 6, 7, 8, 9, 10), but not all *GAS* and/or *RS* genes or homologs; in all *GAS* and/or *RS* genes or homologs; and/or in regulatory regions of all *GAS* and/or *RS* genes or homologs in the plant or plant part.

Each mutation can be heterozygous or homozygous. That is, the plants or plant parts described herein can comprise a certain mutation (e.g., comprising one or more insertions, substitutions, and/or deletions) in one allele or two (both) alleles of a *GAS* and/or *RS* gene/homolog or its regulatory region. All mutations in the plant or plant part can be homozygous; all mutations in the plant or plant part can be heterozygous; or mutations can comprise some heterozygous mutations in certain locations of the genome and some homozygous mutations in certain locations of the genome in the plant or plant part.
In some embodiments, the mutation is located at least partially in a *GAS* and/or *RS* gene or its regulatory region, and (i) the *GAS* and/or *RS* gene (before the mutation is located) comprises a nucleic acid sequence having at least 80% (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) sequence identity to a nucleic acid sequence of any one of SEQ ID NOs: 1-8, 25, 31, 37-40, 42, and 50, wherein the nucleic acid sequence encodes a polypeptide that retains GAS and/or RS activity; (ii) the *GAS* and/or *RS* gene (before the mutation is located) comprises the nucleic acid sequence of any one of SEQ ID NOs: 1-8, 25, 31, 37-40, 42, and 50; (iii) the *GAS* and/or *RS* gene (before the mutation is located) encodes a polypeptide comprising an amino acid sequence having at least 80% (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) sequence identity to an amino acid sequence of any one of SEQ ID NOs: 9-12, 41, 43, and 51, wherein the polypeptide retains GAS and/or RS activity; and/or (iv) the *GAS* and/or *RS* gene (before the mutation is located) encodes a polypeptide comprising an amino acid sequence of any one of SEQ ID NOs: 9-12, 41, 43, and 51. As used herein, where a mutation (e.g., an insertion, a substitution, or a deletion) is "at least partially" in a certain nucleotide region, the whole part of the insertion, substitution, or deletion can be within the certain nucleotide region, or alternatively, can span across the certain nucleotide region and a region outside the nucleotide region. For instance, where an insertion, a substitution, or a deletion is at least partially in an exon, the whole part of the insertion, the substitution, or the deletion can be within the exon, or can span across the exon and a region (e.g., an intron, a regulatory region) upstream or downstream of the exon.

In plants or plant parts provided herein comprising a mutation that decreases the GAS and/or RS activity, at least one (e.g., about 1, 2, 3, 4, 5, 6, 7, 8, 9, 10, 11, 12, 13, 14, 15, 16, 17, 18, 19, 20, or more) insertion, substitution, or deletion can be located in a nucleic acid region of a *Pisum sativum GAS* gene, e.g., *Pisat.03G015560, Pisat.06G066360, Psat3G018520,* or *Psat6g153600,* e.g., comprising the nucleic acid sequence of SEQ ID NO: 1, 2, 5, 6, 25, 37, or 38 and/or in a nucleic acid region of a *Pisum sativum RS* gene, e.g., *Pisat.05G054130, Pisat.01G021000, Psat0s2597g0160,* or *Psat1g055560,* e.g., comprising the nucleic acid sequence of SEQ ID NO: 3, 4, 7, 8, 31, 39, or 40. *Pisat.03G015560* corresponds to *Psat3G018520* in the pea reference genome Cameor. *Pisat.06G066360* corresponds to *Psat6g153600* in Cameor. *Pisat.05G054130* corresponds to *Psat0s2597g0160* in Cameor. *Pisat.01G021000* corresponds to *Psat1g055560* in Cameor. In plants or plant parts provided herein comprising a mutation that decreases the GAS and/or RS activity, at least one (e.g., about 1, 2, 3, 4, 5, 6, 7, 8, 9, 10, 11, 12, 13, 14, 15, 16, 17, 18, 19, 20, or more) insertion, substitution, or deletion can be located in a nucleic acid region of a *Glycine max GAS* gene, e.g., *Glyma.19G227800* or *Glyma.03G229800,* e.g., comprising the nucleic acid sequence of SEQ ID NO: 1, 2, 5, 6, 25, 37, or 38 and/or in a nucleic acid region of a *Pisum sativum RS* gene, e.g., *Pisat.05G054130, Pisat.01G021000, Psat0s2597g0160,* or *Psat1g055560,* e.g., comprising the nucleic acid sequence of SEQ ID NO: 42 or 50. For example, the plants or plant parts provided herein can (i) comprise a mutated *Pisum sativum GAS* gene comprising a G to A substitution of nucleotide 128 of SEQ ID NO: 5 or 25, or comprise a nucleic acid sequence of SEQ ID NO: 15 or 26; (ii) comprise a mutated *Pisum sativum GAS* gene comprising a deletion of nucleotides 115-119 of SEQ ID NO: 25, or comprise a nucleic acid sequence of SEQ ID NO: 27; (iii) comprise a mutated *Pisum sativum GAS* gene comprising a deletion of nucleotides 111-117 of SEQ ID NO: 25, or comprise a nucleic acid sequence of SEQ ID NO: 28; (iv) comprise a mutated *Pisum sativum RS* gene comprising a G to A substitution of nucleotide 1044 of SEQ ID NO: 7 or 31, or comprise a nucleic acid sequence of SEQ ID NO: 16 or 32; (v) comprise a mutated *Pisum sativum RS* gene comprising a deletion of nucleotides 1246-1253 of SEQ ID NO: 31, or comprise a nucleic acid sequence of SEQ ID NO: 3; (vi) comprise a mutated *Pisum sativum RS* gene comprising a deletion of nucleotides 1248-1254 of SEQ ID NO: 31, or comprise a nucleic acid sequence of SEQ ID NO: 34; (vii) comprise a mutated *Pisum sativum* GAS protein comprising a G to D substitution of amino acid 43 of SEQ ID NO: 9, or comprise an amino acid sequence of SEQ ID NO: 17; (viii) comprise a mutated *Pisum sativum* GAS protein encoded by the nucleic acid sequence of SEQ ID NO: 27, or comprise an amino acid sequence of SEQ ID NO: 29; (ix) comprise a mutated *Pisum sativum* GAS protein encoded by the nucleic acid sequence of SEQ ID NO: 28, or comprise an amino acid sequence of SEQ ID NO: 30; (x) comprise a truncated *Pisum sativum* RS protein comprising a deletion of amino acids 348-798 of SEQ ID NO: 11, or comprise an amino acid sequence of SEQ ID NO: 18; (xi) comprise a mutated *Pisum sativum* RS protein encoded by the nucleic acid sequence of SEQ ID NO: 33, or comprise an amino acid sequence of SEQ ID NO: 35; (xii) a mutated *Pisum sativum* RS protein encoded by the nucleic acid sequence of SEQ ID NO: 34, or comprising an amino acid sequence of SEQ ID NO: 36; (xiii) a mutated *Glycine max GAS* gene comprising a deletion of nucleotides 197-209 of SEQ ID NO: 42, or comprising a nucleic acid sequence of SEQ ID NO: 44; (xiv) a mutated *Glycine max GAS* gene comprising a deletion of nucleotides 199-209 of SEQ ID NO: 42, or comprising a nucleic acid sequence of SEQ ID NO: 45; (xv) a mutated *Glycine max GAS* gene comprising a deletion of nucleotide 204 of SEQ ID NO: 42, or comprising a nucleic acid sequence of SEQ ID NO: 46; (xvi) a mutated *Glycine max* GAS protein encoded by the nucleic acid sequence of SEQ ID NO: 44, or comprising an amino acid sequence of SEQ ID NO: 47; (xvii) a mutated *Glycine max* GAS protein encoded by the nucleic acid sequence of SEQ ID NO: 45, or comprising an amino acid sequence of SEQ ID NO: 48; and/or (xviii) a mutated *Glycine max* GAS protein encoded by the nucleic acid sequence of SEQ ID NO: 46, or comprising an amino acid sequence of SEQ ID NO: 49. Additionally or alternatively, the plants or plant pats provided herein can comprise a mutation in the *PsGAS* gene comprising the nucleic acid sequence of SEQ ID NO: 1, 2, 5, 6, 25, 37, or 38 at or near the binding site of a *PsGAS* guide RNA (e.g., SEQ ID NO: 13), and/or a mutation in the *PsRS* gene comprising the nucleic acid sequence of SEQ ID NO: 3, 4, 7, 8, 31, 39, or 40 at or near the binding site of a *PsRS* guide RNA (SEQ ID NO: 14); and/or a mutation in the *GmGAS* gene comprising the nucleic acid sequence of SEQ ID NO: 42 or 50 at or near the binding site of a *GmGAS* guide RNA (SEQ ID NO: 52).

The mutation that decreases the GAS and/or RS activity in the plant or plant part disclosed herein can comprise an out-of-frame mutation of one or both alleles of at least one (e.g., one, more than one but not all, or all) *GAS* and/or RS gene or homolog thereof, such as a deletion of nucleotides 115-119 of *Pisum sativum* GAS gene SEQ ID NO: 25, resulting in a nucleic acid sequence of SEQ ID NO: 27 and/or a mutated *Ps*GAS protein having an amino acid sequence of SEQ ID NO: 29; a deletion of nucleotides 111-117 of *Pisum sativum* GAS gene SEQ ID NO: 25, resulting in a nucleic acid sequence of SEQ ID NO: 28 and/or a mutated *Ps*GAS protein having an amino acid sequence of SEQ ID NO: 30; a deletion of nucleotides 1246-1253 of *Pisum sativum* RS gene SEQ ID NO: 31, resulting in a nucleic acid sequence of SEQ ID NO: 33 and/or a mutated *Ps*RS protein having an amino acid sequence of SEQ ID NO: 35; a deletion of nucleotides 1248-1254 of *Pisum sativum* RS gene SEQ ID NO: 31, resulting in a nucleic acid sequence of SEQ ID NO: 34 and/or a mutated *Ps*RS protein having an amino acid sequence of SEQ ID NO: 36; a deletion of nucleotides 197-209 of *Glycine max* GAS gene SEQ ID NO: 42, resulting in a nucleic acid sequence of SEQ ID NO: 44 and/or a mutated *Gm*GAS protein having an amino acid sequence of SEQ ID NO: 47; a deletion of nucleotides 199-209 of *Glycine max* GAS gene SEQ ID NO: 42, resulting in a nucleic acid sequence of SEQ ID NO: 45 and/or a mutated *Gm*GAS protein having an amino acid sequence of SEQ ID NO: 48; a deletion of nucleotide 204 of SEQ ID NO: 42, resulting in a nucleic acid sequence of SEQ ID NO: 46 and/or a mutated *Gm*GAS protein having an amino acid sequence of SEQ ID NO: 49. Alternatively, the mutation in the plant or plant part can comprise an in-frame mutation, e.g., a missense mutation (such as a G128A substitution in the *PsGAS* gene resulting in SEQ ID NO: 15 or 26, resulting in a G43D substitution in the *Ps*GAS protein set forth as SEQ ID NO: 17), or a nonsense mutation (such as a G1044A substitution in the *PsRS* gene resulting in SEQ ID NO: 16 or 32, resulting in a W348* mutation in the *Ps*RS protein set forth as SEQ ID NO: 18), of one or both alleles of at least one (e.g., one, more than one but not all, or all) *GAS* and/or *RS* gene or homolog thereof.

A plant or plant part of the present disclosure can have a genetic mutation that decreases the GAS and/or RS activity in a gene that is a homolog, ortholog, or variant of a *GAS* and/or *RS* gene disclosed herein and expresses a functional GAS and/or RS, or in a regulatory region of such homolog, ortholog, or variant of a *GAS* and/or *RS* gene. By "orthologs" is intended genes derived from a common ancestral gene and found in different species as a result of speciation. Genes found in different species are considered orthologs when their nucleic acid sequences and/or their encoded protein sequences share at least 75%, 80%, 85%, 90%, 91%, 92%, 93%, 94%, 95%, 96%, 97%, 98%, 99%, or greater sequence identity. Functions of orthologs are often highly conserved among species. Thus, plants or plant parts comprising polynucleotides that have GAS and/or RS activity and share at least 75% sequence identity to the sequences disclosed herein are encompassed by the present disclosure and can have a genetic mutation that decreases the GAS and/or RS activity.

Variant sequences (e.g., homologs, orthologs) can be isolated by PCR. Methods for designing PCR primers and PCR cloning are generally known in the art and are disclosed in Sambrook et al. (1989) Molecular Cloning: A Laboratory Manual (2d ed., Cold Spring Harbor Laboratory Press, Plainview, New York). See also Innis et al., eds. (1990) PCR Protocols: A Guide to Methods and Applications (Academic Press, New York); Innis and Gelfand, eds. (1995) PCR Strategies (Academic Press, New York); and Innis and Gelfand, eds. (1999) PCR Methods Manual (Academic Press, New York). Variant sequences (e.g., homologs, orthologs) may also be identified by analysis of existing databases of sequenced genomes. In this manner, variant sequences encoding GAS and/or RS can be identified and used in the methods of the present disclosure. The variant sequences will retain the GAS and/or RS activity.

In certain instances, mutations in any *GAS* and/or *RS* gene in a plant, plant part, population of plants or plant parts, or plant product (e.g., seed composition, plant protein composition) can be identified by a diagnostic method described herein, e.g., PCR or quantitative PCR. Such diagnostic methods may comprise use of primers for detecting mutation in a *GAS* and/or *RS* gene. For example, a forward primer and a reverse primer can be used for detection of a mutation in the *Pisum sativum GAS* gene at or near the binding site of a *PsGAS* guide RNA (e.g., SEQ ID NO: 13), e.g., a mutation generated by introducing a nuclease and a *PsGAS* guide RNA (e.g., SEQ ID NO: 13) into the plant or plant part. A forward primer (e.g., SEQ ID NO: 23) and a reverse primer (e.g., SEQ ID NO: 24) can be used for detection of a mutation in the *Pisum sativum RS* gene at or near the binding site of the *PsRS* guide RNA (e.g., SEQ ID NO: 14), e.g., a mutation generated by introducing *PsRS* guide RNA (e.g., SEQ ID NO: 14) into the plant or plant part. A forward primer (e.g., SEQ ID NO: 19), a reverse primer (e.g., SEQ ID NO: 20), and/or a probe can be used for detection of a mutation in the *Pisum sativum GAS* gene at or near nucleotide 128 of SEQ ID NO: 5 or 25, e.g., to detect a G128A mutation in SEQ ID NO: 5 or 25 or a mutation that results in a G43D mutation in SEQ ID NO: 9. A forward primer (e.g., SEQ ID NO: 21), a reverse primer (e.g., SEQ ID NO: 22), and/or a probe can be used for detection of a mutation in the *Pisum sativum RS* gene at or near nucleotide 1044 of SEQ ID NO: 7 or 31, e.g., to detect a G1044A mutation in SEQ ID NO: 7 or 31 or a mutation that results in a W348* mutation in SEQ ID NO: 11.

In certain instances, a kit comprising a set of primers can be used for detecting mutation of *GAS* and/or *RS* genes in plants, plant parts, or plant product (e.g., seed composition, plant protein composition). For example, a kit comprising a forward primer and a reverse primer can be used for detection of a mutation in *PsGAS* in plants, plant parts, or plant products (e.g., seed composition, plant protein compositions) near the binding site of the *PsGAS* guide RNA (e.g., SEQ ID NO: 13). A kit comprising a forward primer (e.g., SEQ ID NO: 23) and a reverse primer (e.g., SEQ ID NO: 24) can be used for detection of a mutation in *PsRS* in plants, plant parts, or plant products (e.g., seed composition, plant protein compositions) at or near the binding site of the *PsRS* guide RNA (e.g., SEQ ID NO: 14). A kit comprising a forward primer (e.g., SEQ ID NO: 19), a reverse primer (e.g., SEQ ID NO: 20), and/or a probe can be used for detection of a mutation in the *Pisum sativum GAS* gene at or near nucleotide 128 of SEQ ID NO: 5 or 25, e.g., to detect a G128A mutation in SEQ ID NO: 5 or 25or a mutation that results in a G43D mutation in SEQ ID NO: 9. A kit comprising a forward primer (e.g., SEQ ID NO: 21), a reverse primer (e.g., SEQ ID NO: 22), and/or a probe can be used for detection of a mutation in the *Pisum sativum RS* gene at or near nucleotide 1044 of SEQ ID NO: 7 or 31, e.g., to detect a G1044A mutation in SEQ ID NO: 7 or 31 or a mutation that results in a W348* mutation in SEQ ID NO: 11.

In some embodiments, the mutations, e.g., one or more insertions, substitutions, or deletions are integrated into the plant genome and the plant or the plant part is stably transformed. In other embodiments, the one or more mutations are not integrated into the plant genome and the plant or the plant part is transiently transformed.

The *GAS* and/or *RS* gene with mutation can be an endogenous copy of the gene, and/or an exogenous copy of the gene that was introduced into the plants or plant parts.

Also provided herein is a population of plants or plant parts (e.g., seeds) comprising the plants and plant parts having a genetic mutation that decreases the GAS and/or RS level or activity described herein. Not all individual plants or plant parts need to have the genetic mutation that decreases the GAS and/or RS level or activity. In specific embodiments at least 80%, 81%, 82%, 83%, 84%, 85%, 86%, 87%, 88%, 89%, 90%, 91%, 92%, 93%, 94%, 95%, 96%, 97%, 98%, 99%, or more plants within a given plant population have a mutation that alters the GAS and/or RS level or activity.

One or more mutations (e.g., insertions, substitutions, or deletions) located in at least one *GAS* and/or *RS* gene or homolog or in a regulatory region of such *GAS* and/or *RS* gene or homolog in the genome of the plant or plant part can reduce the expression levels of the *GAS* and/or RS gene or homolog, reduce level or activity of a GAS and/or RS protein encoded by the *GAS* and/or *RS* gene or homolog, reduce GAS and/or RS activity, and/or increase protein content in plant or plant part (e.g., seeds) relative to a control plant or plant part, e.g., when grown under the same environmental condition, as further described in the present disclosure.

### 2. Plants with one or more mutations in regulatory region of a GAS and/or RS gene

The plants or plant parts described herein can comprise a mutation that decreases the GAS and/or RS activity [e.g., one or more (e.g., about 1, 2, 3, 4, 5, 6, 7, 8, 9, 10, 11, 12, 13, 14, 15, 16, 17, 18, 19, 20, or more) insertions, substitutions, and/or deletions] in a regulatory region of at least one (e.g., one, more than one but not all, or all) *GAS* and/or *RS* gene. The *GAS* and/or *RS* gene can be an endogenous copy of the gene, and/or an exogenous copy of the gene that was introduced into the plants or plant parts. The regulatory region having the mutation can comprise a promoter region, 5' untranslated region (5'UTR), a binding site (e.g., an enhancer sequence) for a transcription modulator protein (e.g., transcription factor), or other genomic regions that contribute to regulation of transcription or translation of at least one (e.g., one, more than one but not all, or all) *GAS* and/or *RS* gene to confer to the plant or plant part altered (e.g., reduced) RFO content and/or altered (e.g., increased) sucrose content. Where an insertion, a substitution, or a deletion is "at least partially" in a regulatory region, the whole part of the insertion, the substitution, or the deletion can be within the regulatory region, or can span across the regulatory region and a region upstream or downstream of the regulatory region (e.g., exons, introns).

In some embodiments, the mutation is in a promoter region of at least one (e.g., one, more than one but not all, or all) *GAS* and/or *RS* gene. As used herein, a "promoter" refers to an upstream regulatory region of DNA prior to the ATG of a native gene, having a transcription initiation activity (e.g., function) for said gene and other downstream genes. "Transcription initiation" as used herein refers to a phase or a process during which the first nucleotides in the RNA chain are synthesized. It is a multistep process that starts with formation of a complex between a RNA polymerase holoenzyme and a DNA template at the promoter, and ends with dissociation of the core polymerase from the promoter after the synthesis of approximately first nine nucleotides. A promoter sequence can include a 5' untranslated region (5'UTR), including intronic sequences, in addition to a core promoter that contains a TATA box capable of directing RNA polymerase II (pol II) to initiate RNA synthesis at the appropriate transcription initiation site for a particular polynucleotide sequence of interest. A promoter may additionally comprise other recognition sequences positioned upstream of the TATA box, and well as within the 5'UTR intron, which influence the transcription initiation rate. The one or more insertions, substitutions, and/or deletions in the promoter region of the *GAS* and/or *RS* gene can alter the transcription initiation activity of the promoter. For example, the modified promoter can reduce transcription of the operably linked nucleic acid molecule (e.g., the *GAS* and/or *RS* gene), initiate transcription in a developmentally-regulated or temporally-regulated manner, initiate transcription in a cell-specific, cell-preferred, tissue-specific, or tissue-preferred manner, or initiate transcription in an inducible manner. A deletion, a substitution, or an insertion, e.g., introduction of a heterologous promoter sequence, a cis-acting factor, a motif or a partial sequence from any promoter, including those described elsewhere in the present disclosure, can be introduced into the promoter region of the *GAS* and/or *RS* gene to confer an altered (e.g., reduced) transcription initiation function according to the present disclosure. The insertion, substitution, or deletion can comprise insertion, substitution, or deletion of one or more (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, or more) nucleotides. The substitute can be a cisgenic substitute, a transgenic substitute, or both. The mutation of a promoter region can comprise correction of the promoter sequence by: (i) detection of one or more polymorphism or mutation that enhances the activity of the promoter sequence; and (ii) correction of the promoter sequences by deletion, modification, and/or correction of the polymorphism or mutation. In some embodiments, the mutation is in the upstream region of a promoter region of at least one (e.g., one, more than one but not all, or all) *GAS* and/or *RS* gene.

In some embodiments, a mutation is at least partially located in 5'UTR of one or more (e.g., one, more than one but not all, or all) *GAS* and/or *RS* gene. As used herein, a "5'UTR," used interchangeably with a 5' untranslated region, a leader sequence, or a transcript leader, refers the region of a genomic DNA or mRNA from the transcription initiation site to the translation initiation codon (e.g., between the promoter and the translation initiation codon). The 5'UTR regulates translation of a main coding sequence of the mRNA by various mechanisms including forming complex secondary structure (e.g., pre-initiation complex regulation, closed-loop regulation) or being translated into a polypeptide that regulates translation of the main coding sequence (reinitiation of translation, cis- and trans-regulation).

In some embodiments, the plant or plant part provided herein comprises a mutation that is at least partially located in the regulatory region (e.g., promoter region or 5'UTR) of at least one (e.g., one, more than one but not all, or all) *GAS* and/or *RS* gene at or near one or more transcriptional regulator (e.g., transcriptional enhancer) binding domains. Mutation at or near the transcriptional regulator binding site can alter (e.g., decrease) binding of a transcription factor (e.g., transcriptional enhancer) and alter (e.g., decrease) level or activity of a *GAS* and/or *RS* gene or encoded protein.

In some embodiments, the plant or plant part of the present disclosure comprises a deletion of one or more nucleotides at least partially in the promoter and/or 5'UTR of a *Pisum sativum GAS* or *RS* gene.

In some embodiments, a mutation is located in the gene encoding (or regulating expression of) one or more transcription factors that regulates expression of a *GAS* and/or *RS* gene. A "transcription factor" as used herein refers to a protein (other than an RNA polymerase) that regulates transcription of a target gene. A transcription factor has DNA-binding domains to bind to specific genomic sequences such as an enhancer sequence or a promoter sequence. In some instances, a transcription factor binds to a promoter sequence near the transcription initiation site and regulate formation of the transcription initiation complex. A transcription factor can also bind to regulatory sequences, such as enhancer sequences, and modulate transcription of the target gene. The mutation in the gene encoding (or regulating expression of) a transcription factor can modulate expression or function of the transcription factor and reduce expression levels of the *GAS* and/or *RS* gene, e.g., by inhibiting transcription initiation of the *GAS* and/or *RS* gene promoter. In some embodiments, the mutation modifies or inserts transcription factor binding sites or enhancer elements that regulates *GAS* and/or *RS* gene expression into the regulatory region of the *GAS* and/or *RS* gene.

In some embodiments, the mutation inserts a part or whole of one or more negative regulatory elements of the *GAS* and/or *RS* gene into the genome of a plant cell or plant part. A "negative regulatory element" of a gene, as used herein, refers to a nucleic acid molecule that suppresses expression or GAS and/or RS activity gene, e.g., by suppressing transcription activity of the promoter. The negative regulatory sequence of the gene can be in a cis location or in a trans location. Negative regulatory elements of the one or more *GAS* and/or *RS* genes can also include upstream open reading frames (uORFs). In some instances, a negative regulatory element can be inserted in a region upstream of the *GAS* and/or *RS* gene in order to inhibit the expression and/or function of the gene.

The insertion, substitution, or deletion that is at least partially in the promoter, 5' UTR, the gene encoding (or regulating expression of) one or more transcription factors that regulates expression of a *GAS* and/or *RS* gene, or other regulatory region of a *GAS* and/or *RS* gene can comprise insertion, substitution, or deletion of one or more (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, or more) nucleotides. The substitute can be a cisgenic substitute, a transgenic substitute, or both.

### 3. Plants with reduced GAS and/or RS activity

The plants, plant parts (e.g., seeds, leaves), or plant products (e.g., seed composition, plant protein composition) of the present disclosure can comprise reduced activity of GAS and/or RS compared to a control plant, plant part, or plant product. Also provided herein is a population of plants or plant parts (e.g., seeds) comprising the plants and plant parts of the present disclosure, which has reduced GAS and/or RS activity compared to a control (e.g., wild-type) population of plants or plant parts.

In particular, the GAS and/or RS activity in the plant, plant part, population of plants or plant parts, or plant product of the present disclosure can be reduced by about 10-100%, 20-100%, 30-100%, 40-100%, 50-100%, 60-100%, 70-100%, 80-100%, 20-90%, 30-90%, 40-90%, 50-90%, 60-90%, 70-90%, or 100% (e.g., by about 10-20%, 20-30%, 30-40%, 40-50%, 50-60%, 60-70%, 70-80%, 80-90%, or 90-99%, 100%), e.g., by about 10%, 15%, 20%, 25%, 30%, 35%, 40%, 45%, 50%, 55%, 60%, 65%, 70%, 75%, 80%, 85%, 90%, 95%, 99%, or 100%, or at least 10%, 15%, 20%, 25%, 30%, 35%, 40%, 45%, 50%, 55%, 60%, 65%, 70%, 75%, 80%, 85%, 90%, 95%, 99%, or 100%, as compared to a control plant, plant part, population, or plant product.

GAS and/or RS activity in a plant, plant part, or plant product can be measured by in the plant sample by one or more standard methods for measuring enzymatic activity in a plant sample. For example, GAS activity can be determined by contacting UDP-galactose and myo-inositol with a sample obtained from a plant, plant part, or plant product and measuring the level of galactinol, e.g., by high performance liquid chromatography with refractive index detection (HPLC-RI), ion chromatography, size exclusion chromatography with refractive index detection (HPSEC-RI), liquid chromatography with mass spectrometry (LC-MS), derivatization and gas chromatography (GC), or ion chromatography with pulsed amperometric detection (HPAEC-PAD). RS activity can be determined by contacting galactinol with a sample obtained from a plant, plant part, or plant product and measuring the level of raffinose, e.g., by HPLC-RI, HPSEC-RI, LC-MS, GC, HPAEC-PAD.

### 4. Plants with reduced expression level of GAS and/or RS gene or GAS and/or RS protein

The plant, plant part (e.g., seeds, leaves), or plant product (e.g., seed composition, plant protein composition) of the present disclosure, e.g., comprising one or more insertions, substitutions, or deletions in at least one native *GAS* and/or *RS* gene or homolog or in a regulatory region of such *GAS* and/or *RS* gene or homolog can have reduced expression level of the *GAS* and/or *RS* gene(s) or homolog as compared to the expression level of the *GAS* and/or *RS* gene or homolog in a control plant, plant part, a population of plants or plant parts, or plant product, e.g., a plant, plant part, a population of plants or plant parts, or plant product without such mutation. Also provided herein is a population of plants or plant parts (e.g., seeds) comprising the plants and plant parts of the present disclosure, which has reduced expression level of *GAS* and/or *RS* gene(s) or GAS and/or RS compared to a control (e.g., wild-type) population of plants or plant parts.

In particular, the expression levels of *GAS* and/or *RS* gene or homolog in the plant, plant part, a population of plants or plant parts, or plant product (e.g., seed composition, plant protein composition) of the present disclosure can be reduced by about 10-100%, 20-100%, 30-100%, 40-100%, 50-100%, 60-100%, 70-100%, 80-100%, 20-90%, 30-90%, 40-90%, 50-90%, 60-90%, 70-90%, or 100% (e.g., by about 10-20%, 20-30%, 30-40%, 40-50%, 50-60%, 60-70%, 70-80%, 80-90%, 90-99%, or 100%), e.g., by about 10%, 15%, 20%, 25%, 30%, 35%, 40%, 45%, 50%, 55%, 60%, 65%, 70%, 75%, 80%, 85%, 90%, 95%, 99%, or 100%, or at least 10%, 15%, 20%, 25%, 30%, 35%, 40%, 45%, 50%, 55%, 60%, 65%, 70%, 75%, 80%, 85%, 90%, 95%, 99%, or 100%, as compared to a control plant, plant part, a population of plants or plant parts, or plant product. In specific embodiments, expression levels of *GAS* and/or *RS* gene(s) or homolog in the plant, plant part, a population of plants or plant parts, or plant product of the present disclosure are null or undetectable; or alternatively, are reduced, but is not completely eliminated, i.e., reduced by more than 0% and less than 100% as compared to the expression level of the *GAS* and/or *RS* gene or homolog in a control plant, plant part, a population of plants or plant parts, or plant product. Expression levels of the *GAS* and/or *RS* gene or homolog can be measured by any standard methods for measuring mRNA levels of a gene, including quantitative RT-PCR, northern blot, and serial analysis of gene expression (SAGE). Expression levels of the *GAS* and/or *RS* gene or homolog in a plant, plant part, a population of plants or plant parts, or plant product can also be measured by any standard methods for measuring protein levels, including western blot analysis, ELISA, or dot blot analysis of a protein sample obtained from a plant, plant part, a population of plants or plant parts, or plant product using an antibody directed to the GAS and/or RS encoded by the *GAS* and/or *RS* gene.

The plant, plant part (e.g., seeds, leaves), or plant product (e.g., seed composition, plant protein composition) of the present disclosure, e.g., comprising one or more insertions, substitutions, or deletions in at least one native *GAS* and/or *RS* gene or homolog or in a regulatory region of such *GAS* and/or *RS* gene or homolog can have reduced expression of the GAS and/or RS, e.g., the GAS and/or RS encoded by the *GAS* and/or *RS* gene or homolog (having the mutation in the gene or in its regulatory region), as compared to the expression level of the GAS and/or RS in a control plant, plant part, a population of plants or plant parts, or plant product, e.g., a plant, plant part, a population of plants or plant parts, or plant product without such mutation. In particular, the expression levels of a full length GAS and/or RS in the plant, plant part, a population of plants or plant parts, or plant product of the present disclosure can be reduced as compared to a control plant, plant part, a population of plants or plant parts, or plant product. A "full-length" GAS and/or RS, as used herein, refers to a GAS and/or RS comprising the complete amino acid sequence of a wild-type GAS and/or RS, e.g., encoded by a native *GAS* and/or *RS* gene. A plant, plant part, a population of plants or plant parts, or plant product that contains a mutated *GAS* and/or *RS* gene can have reduced expression of full-length GAS and/or RS as compared to a control plant, plant part, a population of plants or plant parts, or plant product, e.g., a plant, plant part, a population of plants or plant parts, or plant product without such mutation, e.g., a plant, plant part, a population of plants or plant parts, or plant product comprising a native (e.g., wild-type) *GAS* and/or *RS* gene. In some embodiments, in the plant, plant part, a population of plants or plant parts, or plant product of the present disclosure [e.g., comprising one or more insertions, substitutions, or deletions in at least one native *GAS* and/or *RS* gene or homolog or in a regulatory region of such *GAS* and/or *RS* gene or homolog], expression of GAS and/or RS, e.g., full length GAS and/or RS, e.g., encoded by the *GAS* and/or *RS* gene is reduced by about 10-100%, 20-100%, 30-100%, 40-100%, 50-100%, 60-100%, 70-100%, 80-100%, 20-90%, 30-90%, 40-90%, 50-90%, 60-90%, 70-90%, or 100% (e.g., by about 10-20%, 20-30%, 30-40%, 40-50%, 50-60%, 60-70%, 70-80%, 80-90%, 90-99%, or 100%), e.g., by about 10%, 15%, 20%, 25%, 30%, 35%, 40%, 45%, 50%, 55%, 60%, 65%, 70%, 75%, 80%, 85%, 90%, 95%, 99%, or 100%, or at least 10%, 15%, 20%, 25%, 30%, 35%, 40%, 45%, 50%, 55%, 60%, 65%, 70%, 75%, 80%, 85%, 90%, 95%, 99%, or 100%, as compared to expression of GAS and/or RS, e.g., full length GAS and/or RS in a control plant, plant part, a population of plants or plant parts, or plant product. In specific embodiments, expression of GAS and/or RS, e.g., full length GAS and/or RS in the plant, plant part, a population of plants or plant parts, or plant product of the present disclosure is completely eliminated; or alternatively, reduced, but is not completely eliminated, i.e., reduced by more than 0% and less than 100%, as compared to a control plant, plant part, a population of plants or plant parts, or plant product. Expression of a GAS and/or RS, such as a full length GAS and/or RS, in a plant, plant part, a population of plants or plant parts, or plant product can be determined by one or more standard methods of determining protein levels. For example, expression of a GAS and/or RS can be determined by western blot analysis, ELISA, or dot blot analysis of a protein sample obtained from a plant, plant part, a population of plants or plant parts, or plant product using an antibody directed to the GAS and/or RS, e.g., the full-length GAS and/or RS.

### 5. Plants with loss-of-function or reduced function of GAS and/or RS

The plant, plant part (e.g., seeds, leaves), or plant product (e.g., seed composition, plant protein composition) of the present disclosure, e.g., comprising one or more insertions, substitutions, or deletions in at least one native *GAS* and/or *RS* gene or homolog or in a regulatory region of such *GAS* and/or *RS* gene or homolog can have loss-of-function or reduced function in the GAS and/or RS, e.g., loss of GAS and/or RS activity or reduced GAS and/or RS activity, as compared to the GAS and/or RS in a control plant, plant part, or plant product. Also provided herein is a population of plants or plant parts (e.g., seeds) comprising the plants and plant parts of the present disclosure, which has loss-of-function or reduced function of the GAS and/or RS compared to a control (e.g., wild-type) population of plants or plant parts. A control plant, plant part, a population of plants or plant parts, or plant product can be a plant, plant part, a population of plants or plant parts, or plant product without the mutation, or a plant, plant part, a population of plants or plant parts, or plant product having wild-type GAS and/or RS activity. The GAS and/or RS with loss-of-function or reduced function can comprise a mutation compared to a wild-type GAS and/or RS that causes loss or reduction of GAS and/or RS function. In some embodiments, the function or GAS and/or RS activity encoded by the *GAS* and/or *RS* gene or homolog having a mutation (e.g., one or more insertions, substitutions, or deletions) in the gene or its regulatory region is reduced by about 10-100%, 20-100%, 30-100%, 40-100%, 50-100%, 60-100%, 70-100%, 80-100%, 20-90%, 30-90%, 40-90%, 50-90%, 60-90%, 70-90%, or 100% (e.g., by about 10-20%, 20-30%, 30-40%, 40-50%, 50-60%, 60-70%, 70-80%, 80-90%, 90-99%, or 100%), e.g., by about 10%, 15%, 20%, 25%, 30%, 35%, 40%, 45%, 50%, 55%, 60%, 65%, 70%, 75%, 80%, 85%, 90%, 95%, 99%, or 100%, or at least 10%, 15%, 20%, 25%, 30%, 35%, 40%, 45%, 50%, 55%, 60%, 65%, 70%, 75%, 80%, 85%, 90%, 95%, 99%, or 100%, as compared to function or activity of a control GAS and/or RS encoded by a control *GAS* and/or *RS* gene or homolog without such mutation. In specific embodiments, the function or GAS and/or RS activity in the plant, plant part, a population of plants or plant parts, or plant product of the present disclosure is completely eliminated; or alternatively, reduced, but is not completely eliminated, i.e., reduced by more than 0% and less than 100%, as compared to a control plant, plant part, a population of plants or plant parts, or plant product.

GAS and/or RS activity in a plant, plant part, population of plants or plant parts, or plant product can be determined by standard methods for measuring enzymatic activity in a plant sample. For example, GAS activity can be determined by contacting UDP-galactose and myo-inositol with a sample obtained from a plant, plant part, or plant product and measuring the level of galactinol, e.g., by high performance liquid chromatography with refractive index detection (HPLC-RI), ion chromatography, size exclusion chromatography with refractive index detection (HPSEC-RI), liquid chromatography with mass spectrometry (LC-MS), derivatization and gas chromatography (GC), or ion chromatography with pulsed amperometric detection (HPAEC-PAD). RS activity can be determined by contacting galactinol with a sample obtained from a plant, plant part, or plant product and measuring the level of raffinose, e.g., by HPLC-RI, HPSEC-RI, LC-MS, GC, HPAEC-PAD.

### 6. Plants with decreased RFO (e.g., raffinose, stachyose) content and/or increased sucrose content

The plant, plant part (e.g., seeds, leaves), or plant product (e.g., seed composition, plant protein composition) of the present disclosure, e.g., comprising a mutation that decreases GAS and/or RS activity, e.g., comprising one or more insertions, substitutions, or deletions in at least one native *GAS* and/or *RS* gene or homolog or in a regulatory region of such *GAS* and/or *RS* gene or homolog, can have decreased RFO (e.g., raffinose, stachyose) content and/or increased sucrose content as compared to a control plant, plant part, or plant product, e.g., without such mutation. Also provided herein is a population of plants or plant parts (e.g., seeds) comprising the plants and plant parts of the present disclosure, which has decreased RFO (e.g., raffinose, stachyose) content and/or increased sucrose content as compared to a control population. In the population provided herein, not all individual plants or plant parts need to have decreased RFO (e.g., raffinose, stachyose) content and/or increased sucrose content. In specific embodiments, at least 80%, 81%, 82%, 83%, 84%, 85%, 86%, 87%, 88%, 89%, 90%, 91%, 92%, 93%, 94%, 95%, 96%, 97%, 98%, 99%, or more plants within a given plant population have decreased RFO (e.g., raffinose, stachyose) content and/or increased sucrose content as compared to a control plant or plant part.

A control plant, plant part, a population of plants or plant parts, or plant product can comprise a plant or plant part to which a mutation provided herein has not been introduced, e.g., by methods of the present disclosure. Thus, a control plant, plant part, a population of plants or plant parts, or plant product may express a native (e.g., wild-type) *GAS* and/or *RS* gene endogenously or transgenically, and/or may have a wild-type GAS and/or RS activity. One skilled in the art can select an appropriate control. A plant, plant part, a population of plants or plant parts, or plant product of the present disclosure may have decreased RFO (e.g., raffinose, stachyose) content and/or increased sucrose content as compared to a control plant, plant part, a population of plants or plant parts, or plant product, when the plant or plant part of the present disclosure is grown under the same environmental conditions (e.g., same or similar temperature, humidity, air quality, soil quality, water quality, and/or pH conditions) as the control plant or plant part.

In some embodiments, the raffinose and/or stachyose content in the plant, plant part, population of plants or plant parts, and/or plant product (e.g., plant protein and/or oil composition) disclosed herein is reduced by about 10-100%, 20-100%, 30-100%, 40-100%, 50-100%, 60-100%, 70-100%, 80-100%, 20-90%, 30-90%, 40-90%, 50-90%, 60-90%, or 70-90% (e.g., by about 10-20%, 20-30%, 30-40%, 40-50%, 50-60%, 60-70%, 70-80%, 80-90%, or 90-100%), by about 10%, 15%, 20%, 25%, 30%, 35%, 40%, 45%, 50%, 55%, 60%, 65%, 70%, 75%, 80%, 85%, 90%, 95%, or 100%, or at least 10%, 15%, 20%, 25%, 30%, 35%, 40%, 45%, 50%, 55%, 60%, 65%, 70%, 75%, 80%, 85%, 90%, 95%, or 100% as compared to a control plant, plant part, population, or plant product. In specific embodiments, raffinose content in the plant, plant part, population of plants or plant parts, and/or plant product provided herein is reduced by about 15%, at least about 15%, about 85%, at least about 85%, or at least about 15-85%, such that the plant, plant part, population, or plant product comprises raffinose content that is about 85%, 85% or less, about 15%, 15% or less, or about 15%-85% or less as compared to a control plant, plant part, population, or plant product. In specific embodiments, stachyose content in the plant, plant part, population of plants or plant parts, and/or plant product provided herein is reduced by about 50%, at last about 50%, about 70%, at least about 70%, about 80%, at least about 80%, at least about 50-80%, or at least about 70-80%, such that the plant, plant part, population, or plant product comprises stachyose content that is about 50%, 50% or less, 30%, 30% or less, about 20%, 20% or less, about 20-50% or less, or about 20-30% or less as compared to a control plant, plant part, population, or plant product.

In certain cases, seeds of a reference variety of pea cultivar may contain about 0.8-0.9% dry weight of raffinose and about 4.5-5.0% dry weight of stachyose. Seeds of a reference variety of soybean cultivar may have raffinose/stachyose content similar to pea cultivars, e.g., about 0.8-0.9% dry weight of raffinose and about 4.0-4.5% dry weight of stachyose. In contrast, the seeds of the plant, plant part, population of plants or plant parts (e.g., pea), or a population of seeds (e.g., pea) provided herein can have raffinose content of about 0.7% or less, 0.6% or less, 0.5% or less, 0.4% or less, 0.3% or less, 0.2% or less, 0.1% or less; about 0.1-0.7%, 0.1-0.6%, 0.1-0.5%, 0.1-0.4%, 0.1-0.3%, 0.1-0.2%, 0.2-0.3%, 0.3-0.4%, 0.4-0.5%, 0.5-0.6%, 0.6-0.7%, 0.5-0.7%, 0.4-0.7%, 0.3-0.7%, or 0.2-0.7%; or about 0.7%, 0.6%, 0.5%, 0.4%, 0.3%, 0.2%, or 0.1% dry weight. The seeds of the plant, plant part, population of plants or plant parts (e.g., pea), or a population of seeds (e.g., pea) provided herein can have stachyose content of about 1.5% or less, 1.4% or less, 1.3% or less, 1.2% or less, 1.1% or less, 1.0% or less, 0.9% or less, 0.8% or less; about 0.6-1.5%, 0.6-1.4%, 0.6-1.3%, 0.6-1.2%, 0.6-1.1%, 0.6-1.0%, 0.6-0.9%, 0.9-1.0%, 1.0-1.1%, 1.1-1.2%, 1.2-1.3%, 1.3-1.4%, 1.2-1.4%, 1.1-1.4%, 1.0-1.4%, 0.9-1.4%, 0.7%-1.5%, 0.8%-1.5%, 0.9%-1.5%, 1.0%-1.5%; or about 1.5%, 1.4%, 1.3%, 1.2%, 1.1%, 1.0%, 0.9%, or 0.8% dry weight.

In some embodiments, the sucrose content in the plant, plant part, population of plants or plant parts, and/or plant product disclosed herein is increased by about 5-100%, 10-100%, 20-100%, 30-100%, 40-100%, 50-100%, 60-100%, 70-100%, 80-100%, 20-90%, 30-90%, 40-90%, 50-90%, 60-90%, 70-90%, 100-150%, 150-200%, or more than 200%, e.g., by about 10%, 15%, 20%, 25%, 30%, 35%, 40%, 45%, 50%, 55%, 60%, 65%, 70%, 75%, 80%, 85%, 90%, 95%, 100%, 150%, 200%, or more, or at least 10%, 15%, 20%, 25%, 30%, 35%, 40%, 45%, 50%, 55%, 60%, 65%, 70%, 75%, 80%, 85%, 90%, 95%, 100%, 150%, 200%, or more in the plants or plant parts of the present disclosure as compared to a control plant or plant part. In specific embodiments, sucrose content in the plant, plant part, population of plants or plant parts, and/or plant product provided herein is increased by about 10%, at least about 10%, about 80%, at least about 80%, or at least about 10-80%, as compared to a control plant, plant part, population, or plant product. Without wishing to be bound by theory, seeds of a reference variety of pea cultivar may contain about 2.4-3.4% dry weight of sucrose. In contrast, the seeds of the plant, plant part, population of plants or plant parts (e.g., pea), or a population of seeds (e.g., pea) provided herein can have stachyose content of about 3.0% or more, 3.5% or more, 3.7% or more, 4.0% or more, 4.5% or more, 5.0% or more, 5.5% or more, 6.0% or more, 6.2% or more; about 3.0-6.2%, 3.7-6.2%, 4.0-6.2%, 4.5-6.2%, 5.0-6.2%, 5.5-6.2%, 6.0-6.2%, 3.7-4.0%, 4.0-4.5%, 4.5-5.0%, 5.0-5.5%, 5.5-6.2%, 6.0-6.2%, 5.0-6.2%, 4.5-6.2%, 4.0-6.2%, 3.5-6.2%, 3.0-6.2%, 3.5-6.2%; or about 3.0%, 3.7%, 4.0%, 4.5%, 5.0%, 5.5%, 6.0%, or 6.2% dry weight.

Content of raffinose, stachyose, and sucrose in a plant, plant part, population of plants or plant parts, or plant product can be measured by any standard methods, including spectroscopy (near infrared spectroscopy), refractometry, high performance liquid chromatography with refractive index detection (HPLC-RI), ion chromatography, size exclusion chromatography with refractive index detection (HPSEC-RI), liquid chromatography with mass spectrometry (LC-MS), derivatization and gas chromatography (GC), and ion chromatography with pulsed amperometric detection (HPAEC-PAD).

In specific embodiments, the plant, plant part, or a population of plants or plant parts of the present disclosure have the trait of decreased RFO (e.g., raffinose, stachyose) content and/or increased sucrose content as compared to a control plant, plant part, population of plants or plant parts, or plant product, without a significant decrease in yield or germination rate. In some embodiments, a reduction in yield or germination rate in the plant, plant part, or population of plants or plant parts of the present disclosure, having decreased RFO (e.g., raffinose, stachyose) content and/or increased sucrose content, is no more than about 0.5%, 1.0%, 1.5%, 2.0%, 2.5%, 3.0%, 3.5%, 4.0%, 4.5%, or about 5%, 6%, 7%, 8%, 9%, or 10%, e.g., no more than about 0-5%, 0.5-4.5%, 0.5-4%, 1-5%, 1-4%, 2-5%, 2-4%, 0.5-10%, 0.5-8%, 1-10%, 2-10%, 3-10%, 4-10%, 5-10%, 6-10%, 7-10%, or 8-10% as compared to a control plant, plant part, or population of plants or plant parts. Yield and germination rate can be measured and expressed by any means known in the art. In specific embodiments, yield is measured by seed weight or volume of seeds, fruits, leaves, or whole plants harvested from a given harvest area. Germination rate can be measured as percent of germinated plants over total seeds that were seeded.

In specific embodiments, provided herein are seeds and a population of seeds with decreased GAS and/or RS activity provided herein, having decreased RFO (e.g., raffinose, stachyose) content and/or increased sucrose content as compared to control seeds or a population of seeds. For example, provided herein are pea seeds or a population of pea seeds with decreased GAS and/or RS activity, decreased RFO content, and/or increased sucrose content, having seed raffinose content of about 0.7% or less, seed stachyose content of about 1.4% or less, and/or seed sucrose content of about 3.7% or more.

### 7. Plants with increased protein content

The plant, plant part (e.g., seeds, leaves), or plant product (e.g., seed composition, plant protein composition) of the present disclosure, e.g., comprising a mutation that decreases GAS and/or RS activity, e.g., comprising one or more insertions, substitutions, or deletions in at least one native *GAS* and/or *RS* gene or homolog or in a regulatory region of the *GAS* and/or *RS* gene or homolog, can have increased protein content as compared to a control plant, plant part, or plant product, e.g., without such mutation. Also provided herein is a population of plants or plant parts (e.g., seeds) comprising the plants and plant parts of the present disclosure, which has increased protein content and/or white flake protein content as compared to a control population.

A control plant, plant part, a population of plants or plant parts, or plant product can comprise a plant or plant part to which a mutation provided herein has not been introduced, e.g., by methods of the present disclosure. Thus, a control plant, plant part, a population of plants or plant parts, or plant product may express a native (e.g., wild-type) *GAS* and/or *RS* gene endogenously or transgenically, and/or may have a wild-type GAS or RS polypeptide activity. A plant, plant part, a population of plants or plant parts, or plant product of the present disclosure may have increased protein content as compared to a control plant, plant part, a population of plants or plant parts, or plant product, when the plant or plant part of the present disclosure is grown under the same environmental conditions (e.g., same or similar temperature, humidity, air quality, soil quality, water quality, and/or pH conditions) as the control plant or plant part.

In some embodiments, total protein content can be increased by about 0.1-0.5%, 0.5-1.0%, 1-10%, 10-100%, 20-100%, 30-100%, 40-100%, 50-100%, 60-100%, 70-100%, 80-100%, 20-90%, 30-90%, 40-90%, 50-90%, 60-90%, 70-90%, 100-1000%, 200-1000%, 300-1000%, 400-1000%, 500-1000%, 600-1000%, 700-1000%, 800-1000%, 200-900%, 300-900%, 400-900%, 500-900%, 600-900%, 700-900%, or more than 1000% (e.g., by about 10-20%, 20-30%, 30-40%, 40-50%, 50-60%, 60-70%, 70-80%, 80-90%, 90-100%, 100-200%, 200-300%, 300-400%, 400-500%, 500-600%, 600-700%, 700-800%, 800-900%, 900-1000%, or more than 1000%), e.g., by about 0.1%, 0.5%, 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 100%, 200%, 300%, 400%, 500%, 600%, 700%, 800%, 900%, 1000%, or more, or at least 10%, 15%, 20%, 25%, 30%, 35%, 40%, 45%, 50%, 55%, 60%, 65%, 70%, 75%, 80%, 85%, 90%, 95%, 100%, 200%, 300%, 400%, 500%, 600%, 700%, 800%, 900%, 1000%, or more in the plants, plant parts of the present disclosure as compared to a control plant or plant part. In specific embodiments, protein content in the plant, plant part, population of plants or plant parts, and/or plant product provided herein is increased by about 5%, at least about 5%, about 10%, at least about 10%, about 20%, at least about 20%, or at least about 5-20%, as compared to a control plant, plant part, population, or plant product. In some embodiments, total protein content, as expressed by % dry weight, in the plant, plant part, population of plant or plant parts, or plant product provided herein is greater than that in control plant, plant part, or population, and the difference (by subtraction) is about 0.25-10%, 0.5-10%, 0.75-10%, 1.0-10%, 1.5-10%, 2-10%, 2.5-10%, 3-10%, 3.5-10%, 4-10%, 4.5-10%, 5-10%, 6-10%, 7-10%, 8-10%, 9-10%, or more than 10% (e.g., by about 0.25-0.5%, 0.5-0.75%, 0.75-1.0%, 1.0-1.5%, 1.5-2.0%, 2.0-2.5%, 2.5-3.0%, 3.0-3.5%, 3.5-4.0%, 4.0-4.5%, 4.5-5.0%, 5-6%, 6-7%, 7-8%, or 8-9%, 9-10%, or more than 10%), by about 0.25%, 0.5%, 0.75%, 1.0%, 1.5%, 2.0%, 2.5%, 3.0%, 3.5%, 4.0%, 4.5%, 5%, 6%, 7%, 8%, 9%, 10%, or more, or at least 0.25%, 0.5%, 0.75%, 1.0%, 1.5%, 2.0%, 2.5%, 3.0%, 3.5%, 4.0%, 4.5%, 5%, 6%, 7%, 8%, 9%, 10%, or more % dry weight. In specific embodiments, protein content in the plant, plant part, population of plants or plant parts, and/or plant product provided herein is increased by about 2%, at least about 2%, about 5%, at least about 5%, about 10%, at least about 10%, or at least about 5-20% dry weight (by subtraction) as compared to a control plant, plant part, population, or plant product.

In some embodiments, provided herein are seeds or a population of seeds having seed protein content greater than control seeds or a control population of seeds (e.g., control seeds or population having a native GAS and/or RS protein, reference seeds or population, commodity seeds or population). The seeds can be legume seeds, e.g., pea seeds. Without wishing to be bound by theory, typical pea cultivars average approximately 20-30% protein in the seed in dry weight (Meng & Cloutier, 2014 Microencapsulation in the Food Industry: A Practical Implementation Guide § 20.5). In contrast, the pea seeds or population of pea seeds provided herein can have seed protein content of at least 35%, 36%, 37%, 38%, 39%, 40%, 41%, 42%, 43%, 44%, 45%, 46%, 47%, 48%, 49%, 50%, or more by dry weight.

Protein content in a plant, plant part, plant product, or a population of plants or plant parts can be measured by standard methods for measuring total and specific amino acids in a plant sample, for example by high performance liquid chromatography (HPLC), spectrophotometer, mass spectrometry (MS), and combination thereof. Protein content in a plant sample can be measured by standard methods, for example by protein extraction and quantitation (e.g., BCA protein assay, Lowry protein assay, Bradford protein assay), spectroscopy, near-infrared reflectance (NIR) (e.g., analyzing 700 - 2500 nm), and nuclear magnetic resonance spectrometry (NMR). In specific embodiments, protein content is measured by the Dumas method, by combusting samples at a high temperature in the presence of high-purity oxygen, analyzing the gas from combustion for nitrogen content using a thermal conductivity detector, and calculating the amount of protein present in the sample using a conversion factor. The industry standard conversion factor for pea is 6.25.

In specific embodiments, provided herein are seeds and a population of seeds with decreased GAS and/or RS activity provided herein, having increased protein content as compared to control seeds or a population of seeds. For example, provided herein are pea seeds or a population of pea seeds with decreased GAS and/or RS activity and increased protein content, having seed protein content of about 40% or more.

The plant, plant part, a population of plants or plant parts, or plant product of the present disclosure can have decreased RFO content as well as increased protein content as compared to a control plant, plant part, population of plants or plant parts, or plant product. For example, the plant, plant part, population of plants or plant parts, and/or plant product provided herein can have raffinose content that is reduced by about 15%, at least about 15%, about 85%, at least about 85%, or at least about 15-85%, such that the plant, plant part, population, or plant product comprises raffinose content that is about 85%, 85% or less, about 15%, 15% or less, or about 15%-85% or less as compared to a control plant, plant part, population, or plant product; and/or stachyose content that is reduced by about 50%, at least about 50%, about 70%, at least about 70%, about 80%, at least about 80%, at least about 50-80%, or at least about 70-80%, such that the plant, plant part, population, or plant product comprises stachyose content that is about 50%, 50% or less, 30%, 30% or less, about 20%, 20% or less, about 20-50% or less, or about 20-30% or less as compared to a control plant, plant part, population, or plant product; and/or sucrose content that is increased by about 10%, at least about 10%, about 80%, at least about 80%, or at least about 10-80%, as compared to a control plant, plant part, population, or plant product; as well as protein content that is increased by about 5%, at least about 5%, about 10%, at least about 10%, about 20%, at least about 20%, or at least about 5-20% as compared to a control plant, plant part, population, or plant product.

In specific embodiments, provided herein are seeds and a population of seeds with decreased GAS and/or RS activity, having decreased RFO (e.g., raffinose, stachyose) content (and/or increased sucrose content), as well as increased protein content, as compared to control seeds or a population of seeds. For example, provided herein are pea seeds or a population of pea seeds with decreased GAS and/or RS activity, decreased RFO content (and/or increased sucrose content), as well as increased protein content, having seed raffinose content of about 0.7% or less, seed stachyose content of about 1.4% or less, and/or seed sucrose content of about 3.7%; as well as seed protein content of 40% or more.

### B. Plant parts and plant products

The present disclosure provides plant parts and plant products obtained from the plant of the present disclosure. A "plant product," as used herein, refers to any composition derived from the plant or plant part, including any oil products, sugar products, fiber products, protein products (such as protein concentrate, protein isolate, flake, or other protein product), seed hulls, meal, or flour, for a food, feed, aqua, or industrial product, plant extract (e.g., sweetener, antioxidants, alkaloids, etc.), plant concentrate (e.g., whole plant concentrate or plant part concentrate), plant powder (e.g., formulated powder, such as formulated plant part powder (e.g., seed flour)), plant biomass (e.g., dried biomass, such as crushed and/or powdered biomass), grains, plant protein composition, plant oil composition, and food and beverage products containing plant compositions (e.g., plant parts, plant extract, plant concentrate, plant powder, plant protein, plant oil, and plant biomass) described herein. Plant parts and plant products provided herein can be intended for human or animal consumption.

As used herein, a "protein product" or "protein composition" refers to any protein composition or product isolated, extracted, and/or produced from plants or plant parts (e.g., seed) and includes isolates, concentrates, and flours, e.g., pea/soy protein composition, pea/soy protein concentrate (PPC/SPC), pea/soy protein isolate (PPI/SPI), pea/soy flour, flake, white flake, texturized vegetable protein (TVP), or textured pea/soy protein (PSP/TSP)). Plant protein compositions of the present disclosure can be a concentrated protein solution (e.g., pea protein concentrate solution) in which the protein is in a higher concentration than the protein in the plant from which the protein composition is derived. The protein composition can comprise multiple proteins as a result of the extraction or isolation process. In specific embodiments, the protein composition can further comprise stabilizers, excipients, drying agents, desiccating agents, anti-caking agents, or any other ingredient to make the protein fit for the intended purpose. The protein composition can be a solid, liquid, gel, or aerosol and can be formulated as a powder. The protein composition can be extracted in a powder form from a plant and can be processed and produced in different ways, such as: *(i) as an isolate* - through the process of wet fractionation, which has the highest protein concentration; *(ii) as a concentrate* - through the process of dry fractionation, which are lower in protein concentration; and/or *(iii) in textured form* - when it is used in food products as a substitute for other products, such as meat substitution (e.g. a "meat" patty). Protein isolate can be derived from defatted plant flour with a high solubility in water, as measured by the nitrogen solubility index (NSI). The aqueous extraction is carried out at a pH below 9. The extract is clarified to remove the insoluble material and the supernatant liquid Is acidified to a pH range of 4-5. The precipitated protein-curd is collected and separated from the whey by centrifuge. The curd can be neutralized with alkali to form the sodium proteinate salt before drying. Protein concentrate can be produced by immobilizing the plant globulin proteins while allowing the soluble carbohydrates, whey proteins, and salts to be leached from the defatted flakes or flour. The protein is retained by one or more of several treatments: leaching with 20-80% aqueous alcohol/solvent, leaching with aqueous acids in the isoelectric zone of minimum protein solubility, pH 4-5; leaching with chilled water (which may involve calcium or magnesium cations), and leaching with hot water of heat-treated defatted protein meal/flour (e.g., pea/soy meal/flour). Any of the process provided herein can result in a product that is 70% protein, 20% carbohydrates (2.7 to 5% crude fiber), 6% ash and about 1% oil, but the solubility may differ. As an example, one ton (t) of defatted flakes can yield about 750 kg of plant protein concentrate. "Texturized vegetable protein" (TVP), "Textured vegetable protein," also referred to as "textured pea/soy protein" (TPP/TSP), pea/soy meat, or pea/soya chunks refers to a defatted plant (e.g., pea, soy) flour product, a by-product of extracting plant (e.g., pea, soybean) oil. It can be used as a meat analogue or meat extender. It is quick to cook, with a protein content comparable to certain meats. TVP can be produced from any protein-rich seed meal left over from vegetable oil production. A wide range of pulse seeds such as lentils, peas, soybeans, and fava beans, or peanut may be used for TVP production. TVP can be made from high protein (e.g., 50%) plant isolate, flour, or concentrate, and can also be made from cottonseed, wheat, and oats. It is extruded into various shapes (chunks, flakes, nuggets, grains, and strips) and sizes, exiting the nozzle while still hot and expanding as it does so. The defatted thermoplastic proteins are heated to 150-200 °C, which denatures them into a fibrous, insoluble, porous network that can soak up as much as three times its weight in liquids. As the pressurized molten protein mixture exits the extruder, the sudden drop in pressure causes rapid expansion into a puffy solid that is then dried. As much as 50% protein when dry, TVP can be rehydrated at a 2:1 ratio, which drops the percentage of protein to an approximation of ground meat at 16%. TVP can be used as a meat substitute. When cooked together, TVP can help retain more nutrients from the meat by absorbing juices normally lost. Also provided herein are methods of isolating, extracting, or preparing any of the protein compositions or protein products provided herein from plants or plant parts.

In specific embodiments, the plant protein compositions provided herein are obtained from a pea plant *(Pisum sativum)* that contains a mutation that decreases GAS and/or RS activity, e.g., one or more insertions, substitutions, or deletions in at least one native *GAS* and/or *RS* gene or homolog or in a regulatory region of such *GAS* and/or *RS* gene or homolog.

Food and/or beverage products of the present disclosure can contain plant compositions, e.g., seed composition, plant protein compositions of the present disclosure. Food and/or beverage products can be meant for human or animal consumption. Food and/or beverage products of the present disclosure can include animal feed, shakes (e.g., protein shakes), health drinks, alternative meat products (e.g., meatless burger patties, meatless sausages), alternative egg products (e.g., eggless mayo), non-dairy products (e.g., non-dairy whipped toppings, non-dairy milk, non-dairy creamer, non-dairy milk shakes, non-diary ice cream), energy bars (e.g., protein energy bars), infant formula, baby foods, cereals, baked goods, edamame, tofu, and tempeh.

Plant parts (e.g., seeds) and plant products (e.g., plant biomass, seed compositions, protein compositions, food and/or beverage products) as disclosed herein can be meant for consumption by agricultural animals or for use as feed in an agriculture or aquaculture system. In specific embodiments, plant parts and plant products include animal feed (e.g., roughages - forage, hay, silage; concentrates - cereal grains, pea cake) intended for consumption by bovine, porcine, poultry, lambs, goats, or any other agricultural animal. In some embodiments, plant parts and plant products include aquaculture feed for any type of fish or aquatic animal in a farmed or wild environment including, without limitation, trout, carp, catfish, salmon, tilapia, crab, lobster, shrimp, oysters, clams, mussels, and scallops.

Seeds of the present disclosure include a representative sample of seeds from a plant of the present disclosure. A plant or plant part of the present disclosure can be a crop plant, a forage plant, or part of a crop plant or forage plant.

As provided herein, the plant parts, population of plant parts, and plant products (e.g., seed compositions, plant protein compositions, and plant-based food/beverage products) of the present disclosure can contain a mutation that decreases GAS and/or RS activity, e.g., one or more insertions, substitutions, or deletions in at least one native *GAS* and/or *RS* gene or homolog or in a regulatory region of such *GAS* and/or *RS* gene or homolog (e.g., a missense mutation in the *PsGAS* gene, a nonsense mutation in the *PsRS* gene). The plant parts, population of plant parts, and plant products of the present disclosure can have reduced GAS and/or RS activity, reduced expression level of the *GAS* and/or *RS* gene or homolog, reduced expression level of the GAS and/or RS (e.g., the full-length GAS and/or RS), loss of function or reduced function or GAS and/or RS activity, decreased RFO (e.g., raffinose, stachyose) content and/or increased sucrose content as compared to a control plant part, population, or plant product, e.g., without the mutation, comprising a native (e.g., wild-type) *GAS* and/or *RS* gene or GAS and/or RS, or comprising wild-type GAS and/or RS activity. For example, the plant parts, population of plant parts, and plant products provided herein can have decreased GAS and/or RS activity provided herein, decreased RFO (e.g., raffinose, stachyose) content, increased sucrose content, and/or increased protein content, as compared to control plant parts, population of plant parts, and plant products. For example, pea seeds or pea seed compositions (such as flour or pea protein concentrate) provided herein can have decreased GAS and/or RS activity, decreased RFO content, increased sucrose content, and/or increased protein content, with raffinose content of about 0.7% or less, or 0.3% or less, seed stachyose content of about 1.5% or less, 1.4% or less, sucrose content of about 3.0% or more, or 3.7% or more. Further, pea seeds or pea seed compositions provided herein can have protein content of 40% or more. In specific embodiments, the plant parts, population of plant parts, and plant products having decreased GAS and/or RS activity have decreased RFO (e.g., raffinose, stachyose) content (and/or increased sucrose content) as well as increased protein content, as compared to control seeds or a population of seeds, such as pea seeds or pea seed compositions with decreased GAS and/or RS activity, decreased RFO content (and/or increased sucrose content), as well as increased protein content, having seed raffinose content of about 0.7% or less or 0.3% or less, seed stachyose content of about 1.5% or less or 1.4% or less, and/or seed sucrose content of about 3.0% or more or 3.7% or more; and seed protein content of 40% or more.

Also provided herein is a pea composition comprising low RFO content and/or high protein content relative to a reference pea composition. In specific embodiments, provided herein is a pea composition comprising raffinose content of about 0.7% or less or 0.3% or less, stachyose content of about 1.5% or less or 1.4% or less, and sucrose content of about 3.0% or more or 3.7% or more dry weight of total content. In specific embodiments, the pea composition can comprise raffinose content of about 0.3% or less, stachyose content of about 1.5% or less, and sucrose content of about 3.0% or more dry weight of total content. The pea composition can further have protein content of about 40% or more dry weight of total content. In further specific embodiments, the pea composition comprises (i) raffinose content of about 0.7% or less or 0.3% or less, stachyose content of about 1.5% or less or 1.4% or less, and/or sucrose content of about 3.0% or more or 3.7% or more, and (ii) protein content of about 40% or more dry weight of total content. The pea composition can comprise pea flour, a pea protein concentrate, a pea seed composition, a pea protein composition, a pea protein isolate, pea flake, pea white flake, textured pea protein, or a pea oil composition.

The pea composition provided herein can comprise a mutated *GAS* and/or *RS* gene or homolog thereof or regulatory region thereof. For example, the pea composition comprises a mutated *Pisum sativum GAS gene* comprising an insertion, a substitution, or a deletion of one or more nucleotides of SEQ ID NO: 1, 2, 5, 6, 25, 37, or 38; and/or a mutated *Pisum sativum RS* gene comprising an insertion, a substitution, or a deletion of one or more nucleotides of SEQ ID NO: 3, 4, 7, 8, 31, 39, or 40. The pea composition can comprise a mutated *Pisum sativum GAS* gene comprising a G to A substitution of nucleotide 128 of SEQ ID NO: 5 or 25, or comprising a nucleic acid sequence of SEQ ID NO: 15 or 26; a mutated *Pisum sativum GAS* gene comprising a deletion of nucleotides 115-119 of SEQ ID NO: 25, or comprising a nucleic acid sequence of SEQ ID NO: 27; a mutated *Pisum sativum GAS* gene comprising a deletion of nucleotides 111-117 of SEQ ID NO: 25, or comprising a nucleic acid sequence of SEQ ID NO: 28; a mutated *Pisum sativum RS* gene comprising a G to A substitution of nucleotide 1044 of SEQ ID NO: 7 or 31, or comprising a nucleic acid sequence of SEQ ID NO: 16 or 32; a mutated *Pisum sativum RS* gene comprising a deletion of nucleotides 1246-1253 of SEQ ID NO: 31, or comprising a nucleic acid sequence of SEQ ID NO: 33; a mutated *Pisum sativum RS* gene comprising a deletion of nucleotides 1248-1254 of SEQ ID NO: 31, or comprising a nucleic acid sequence of SEQ ID NO: 34; a mutated *Pisum sativum* GAS protein comprising a G to D substitution of amino acid 43 of SEQ ID NO: 9, or comprising an amino acid sequence of SEQ ID NO: 17; a mutated *Pisum sativum* GAS protein encoded by the nucleic acid sequence of SEQ ID NO: 27, or comprising an amino acid sequence of SEQ ID NO: 29; a mutated *Pisum sativum* GAS protein encoded by the nucleic acid sequence of SEQ ID NO: 28, or comprising an amino acid sequence of SEQ ID NO: 30; a truncated *Pisum sativum* RS protein comprising a deletion of amino acids 348-798 of SEQ ID NO: 11, or comprising an amino acid sequence of SEQ ID NO: 18; a mutated *Pisum sativum* RS protein encoded by the nucleic acid sequence of SEQ ID NO: 33, or comprising an amino acid sequence of SEQ ID NO: 35; and/or a mutated *Pisum sativum* RS protein encoded by the nucleic acid sequence of SEQ ID NO: 34, or comprising an amino acid sequence of SEQ ID NO: 36. The presence of such mutations in a pea composition can be detected by any standard method for detecting a mutation in a sample, such as PCR or quantitative PCR using primers (e.g., a forward primer SEQ ID NO: 23 and a reverse primer SEQ ID NO: 24, a forward primer SEQ ID NO: 19 and a reverse primer SEQ ID NO: 20, or a forward primer SEQ ID NO: 21 and a reverse primer SEQ ID NO: 22), and/or a probe for detecting mutation in a *GAS* and/or RS gene.

### IV. Decreasing GAS and/or RS Activity in Plants

Methods are provided herein for altering (e.g., increasing) protein content in a plant or plant part. In some aspects, the methods comprise reducing galactinol synthase (GAS) and/or raffinose synthase (RS) activity in the plant or plant part, by, e.g., reducing levels or galactinol synthase (GAS) and/or raffinose synthase (RS) activity. Levels or activity of GAS and/or RS in a plant or plant part can be reduced by any methods known in the art for reducing protein activity or reducing gene expression, including the methods provided herein.

In some aspects, the methods comprise introducing a genetic mutation that alters (e.g., decreases) galactinol synthase (GAS) and/or raffinose synthase (RS) activity into a plant or plant part. The method can further comprise introducing the genetic mutation that alters (e.g., decreases) GAS and/or RS activity into a plant cell, and regenerating a plant or plant part from the plant cell (e.g., transformed plant cell). The methods provided herein can alter (e.g., decrease) GAS and/or RS level or activity, alter (e.g., decrease) expression levels of at least one *GAS* and/or *RS* gene encoding GAS and/or RS, alter (e.g., decrease) GAS and/or RS levels or activity, and/or alter (e.g., increase) protein content in the plant or plant part compared to a control plant or plant part. A control plant or plant part can be a plant or plant part to which a mutation provided herein has not been introduced, e.g., by methods of the present disclosure. Thus, a control plant or plant part (e.g., seeds, leaves) may express a native (e.g., wild-type) *GAS* and/or *RS* gene endogenously or transgenically. A control plant of the present disclosure may be grown under the same environmental conditions (e.g., same or similar temperature, humidity, air quality, soil quality, water quality, and/or pH conditions) as a plant to which the mutation is introduced according to the methods provided herein. Also provided herein are plants, plant parts (e.g., seeds, leaves), a population of plants or plant parts, or plant product (e.g., seed composition, plant protein compositions) produced according to the methods of the present disclosure. Such plants, plant parts, a population of plants or plant parts, or plant products may have the mutation that decreases GAS and/or RS activity, altered (e.g., decreased) expression levels of at least one *GAS* and/or *RS* gene or homolog thereof, altered (e.g., decreased) GAS and/or RS levels or activity, and/or altered (e.g., increased) protein content as compared to a control plant or plant part, when the plant or plant part of the present disclosure is grown under the same environmental conditions as the control plant or plant part.

### A. Altering expression or function of GAS and/or RS gene or polypeptide in plants

Provided herein are compositions and methods for altering (e.g., increasing) protein content in a plant or plant part by introducing a genetic mutation that alters (e.g., decreases) galactinol synthase (GAS) and/or raffinose synthase (RS) activity into a plant or plant part. The method can further comprise introducing the genetic mutation that alters (e.g., decreases) GAS and/or RS activity into a plant cell, and regenerating a plant or plant part from the plant cell (e.g., transformed plant cell). The genetic mutation that is introduced into the plant or plant part according to the methods provided herein can comprise one or more insertions, substitutions, or deletions into the genome of the plant or plant part. The genetic mutation that alters (e.g., decreases) the GAS and/or RS activity can be introduced into at least one native *GAS* and/or *RS* gene or homolog thereof; a regulatory region of the native *GAS* and/or *RS* gene or homolog thereof; in a coding region, a non-coding region, or a regulatory region of any other gene; or at any other site in the genome of the plant or plant part. A "native" gene refers to any gene having a wild-type nucleic acid sequence, e.g., a nucleic acid sequence that can be found in the genome of a plant existing in nature, including a gene that does not naturally occur within the plant, plant part, or plant cell comprising the gene. For example, a transgenic *GAS* and/or *RS* gene located at a genomic site or in a plant in a non-naturally occurring matter is a "native" *GAS* and/or *RS* gene if its nucleic acid sequence can be found in a plant existing in nature.

### 1. Introducing mutation to GAS and/or RS gene, or its homolog. ortholog, or variant

In some aspects, the methods provided herein comprise introducing a genetic mutation that decreases the GAS and/or RS activity into a plant or plant part. The genetic mutation that is introduced into the plant or plant part can comprise one or more (e.g., about 1, 2, 3, 4, 5, 6, 7, 8, 9, 10, 11, 12, 13, 14, 15, 16, 17, 18, 19, 20, or more) insertions, substitutions, and/or deletions in at least one native *GAS* and/or *RS* gene or homolog thereof and/or in a regulatory region of said at least one native *GAS* and/or *RS* gene or homolog thereof in a genome of said plant or plant part. A plant or plant part described herein can comprise 1-2, 1-3, 1-4, 1-5, 2-5, 3-5, 4-5 (e.g., 1, 2, 3, 4, or 5) copies of *GAS* and/or *RS* gene, each encoding a GAS and/or RS. In particular, the plant or plant part to which the mutation is introduced according to the methods can comprise at least 2 genes encoding a GAS and/or RS, such as 2, 3, 4, 5, 6, 7, 8, 9, or 10 genes that have less than 100% (e.g., less than 100%, 99%, 98%, 97%, 96%, 95%, 94%, 93%, 92%, 91%, 90%, 89%, 88%, 87%, 86%, 8, or 315%) sequence identity to one another. The methods can comprise introducing one or more (e.g., about 1, 2, 3, 4, 5, 6, 7, 8, 9, 10, 11, 12, 13, 14, 15, 16, 17, 18, 19, 20, or more) insertions, substitutions, and/or deletions: into one *GAS* and/or *RS* gene or homolog; into a regulatory region of one *GAS* and/or *RS* gene or homolog; into more than one (e.g., 2, 3, 4, 5, 6, 7, 8, 9, 10), but not all *GAS* and/or *RS* genes or homologs; into regulatory regions of more than one (e.g., 2, 3, 4, 5, 6, 7, 8, 9, 10), but not all *GAS* and/or *RS* genes or homologs; into all *GAS* and/or *RS* genes or homologs; and/or into regulatory regions of all *GAS* and/or *RS* genes or homologs in the plant or plant part.

Each mutation that is introduced into the plant or plant part can be heterozygous or homozygous. That is, the method can introduce a certain mutation (e.g., comprising one or more insertions, substitutions, and/or deletions) in one allele or two (both) alleles of a *GAS* and/or *RS* gene/homolog or its regulatory region. All mutations introduced into the plant or plant part can be homozygous; all mutations introduced into the plant or plant part can be heterozygous; or mutations can comprise some heterozygous mutations in certain locations of the genome and some homozygous mutations in certain locations of the genome in the plant or plant part.

In some embodiments, the mutation is introduced at least partially into a *GAS* and/or *RS* gene or its regulatory region, and (i) the *GAS* and/or RS gene comprises a nucleic acid sequence having at least 80% (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) sequence identity to a nucleic acid sequence of any one of SEQ ID NOs: 1-8, 25, 31, 37-40, 42, and 50, wherein the nucleic acid sequence encodes a polypeptide that retains GAS and/or RS activity; (ii) the *GAS* and/or *RS* gene comprises the nucleic acid sequence of any one of SEQ ID NOs: 1-8, 25, 31, 37-40, 42, and 50; (iii) the *GAS* and/or *RS* gene encodes a polypeptide comprising an amino acid sequence having at least 80% (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) sequence identity to an amino acid sequence of any one of SEQ ID NOs: 9-12, 41, 43, and 51, wherein the polypeptide retains GAS and/or RS activity; and/or (iv) the *GAS* and/or RS gene encodes a polypeptide comprising an amino acid sequence of any one of SEQ ID NOs: 9-12, 41, 43, and 51. In specific embodiments, the mutation that decreases the GAS and/or RS activity is introduced into one or two alleles of one or more (e.g., one, more than one but not all, or all) copies of *Pisum sativum GAS* or *RS* gene, and/or a regulatory region thereof.

The methods provided herein can include introducing at least one (e.g., about 1, 2, 3, 4, 5, 6, 7, 8, 9, 10, 11, 12, 13, 14, 15, 16, 17, 18, 19, 20, or more) insertion, substitution, or deletion at least partially into in a coding region of *Pisum sativum GAS* or *RS* gene in the plant or plant part. For instance, where an insertion, a substitution, or a deletion is at least partially in an exon, the whole part of the insertion, the substitution, or the deletion can be within the exon, or can span across the exon and a region (e.g., an intron, a regulatory region) upstream or downstream of the exon. For example, at least one (e.g., about 1, 2, 3, 4, 5, 6, 7, 8, 9, 10, 11, 12, 13, 14, 15, 16, 17, 18, 19, 20, or more) insertion, substitution, or deletion can be introduced into a nucleic acid region of a *Pisum sativum GAS* gene comprising the nucleic acid sequence of SEQ ID NO: 1, 2, 5, 6, 25, 37, or 38, and/or in a nucleic acid region of a *Pisum sativum RS* gene comprising the nucleic acid sequence of SEQ ID NO: 3, 4, 7, 8, 31, 39, or 40, and/or in a *Glycine max GAS* gene comprising the nucleic acid sequence of SEQ ID NO: 42 or 50. For example, a G to A substitution of nucleotide 128, or other mutation, can be introduced into SEQ ID NO: 5 or 25, to produce a nucleic acid sequence of SEQ ID NO: 15 or 26, or a G to D substitution of amino acid 43 of SEQ ID NO: 9 resulting in an amino acid sequence of SEQ ID NO: 17. A G to A substitution of nucleotide 1044, or other mutation can be introduced into SEQ ID NO: 7 or 31, to produce a nucleic acid sequence of 16 or 32, or a deletion of amino acids 348-798 of SEQ ID NO: 11, resulting in an amino acid sequence of SEQ ID NO: 18. Additionally or alternatively, a mutation can be introduced into the *PsGAS* gene comprising the nucleic acid sequence of SEQ ID NO: 1, 2, 5, 6, 25, 37, or 38 at or near the binding site of a *PsGAS* guide RNA (e.g., SEQ ID NO: 13). A mutation can be introduced into the *PsRS* gene comprising the nucleic acid sequence of SEQ ID NO: 3, 4, 7, 8, 31, 39, or 40 at or near the binding site of a *PsRS* guide RNA (SEQ ID NO: 14); and/or a mutation in the *GmGAS* gene comprising the nucleic acid sequence of SEQ ID NO: 42 or 50 at or near the binding site of a *GmGAS* guide RNA (SEQ ID NO: 52). For example, a deletion of nucleotides 115-119 can be introduced into a *Pisum sativum GAS* gene SEQ ID NO: 25, to produce a nucleic acid sequence of SEQ ID NO: 27 or a mutated *Ps*GAS protein having an amino acid sequence of SEQ ID NO: 29; a deletion of nucleotides 111-117 can be introduced into a *Pisum sativum GAS* gene SEQ ID NO: 25, to produce a nucleic acid sequence of SEQ ID NO: 28 or a mutated *Ps*GAS protein having an amino acid sequence of SEQ ID NO: 30; a deletion of nucleotides 1246-1253 can be introduced into a *Pisum sativum RS* gene SEQ ID NO: 31, to produce a nucleic acid sequence of SEQ ID NO: 33 or a mutated *Ps*RS protein having an amino acid sequence of SEQ ID NO: 35; a deletion of nucleotides 1248-1254 can be introduced into a *Pisum sativum RS* gene SEQ ID NO: 31, to produce a nucleic acid sequence of SEQ ID NO: 34 or a mutated *Ps*RS protein having an amino acid sequence of SEQ ID NO: 36; a deletion of nucleotides 197-209 can be introduced into *Glycine max* GAS gene SEQ ID NO: 42, to produce a nucleic acid sequence of SEQ ID NO: 44 or a mutated *Gm*GAS protein having an amino acid sequence of SEQ ID NO: 47; a deletion of nucleotides 199-209 can be introduced into *Glycine max* GAS gene SEQ ID NO: 42, to produce a nucleic acid sequence of SEQ ID NO: 45 or a mutated *Gm*GAS protein having an amino acid sequence of SEQ ID NO: 48; a deletion of nucleotide 204 cab be introduced into SEQ ID NO: 42, resulting in a nucleic acid sequence of SEQ ID NO: 46 or a mutated *Gm*GAS protein having an amino acid sequence of SEQ ID NO: 49.

The mutation introduced into the plant or plant part according to the methods of the present disclosure can comprise an out-of-frame mutation of one or both alleles of at least one (e.g., one, more than one but not all, or all) *GAS* and/or *RS* gene or homolog thereof such as a deletion of nucleotides 115-119 of *Pisum sativum* GAS gene SEQ ID NO: 25, resulting in a nucleic acid sequence of SEQ ID NO: 27 and/or a mutated *Ps*GAS protein having an amino acid sequence of SEQ ID NO: 29; a deletion of nucleotides 111-117 of *Pisum sativum* GAS gene SEQ ID NO: 25, resulting in a nucleic acid sequence of SEQ ID NO: 28 and/or a mutated *Ps*GAS protein having an amino acid sequence of SEQ ID NO: 30; a deletion of nucleotides 1246-1253 of *Pisum sativum* RS gene SEQ ID NO: 31, resulting in a nucleic acid sequence of SEQ ID NO: 33 and/or a mutated *Ps*RS protein having an amino acid sequence of SEQ ID NO: 35; a deletion of nucleotides 1248-1254 of *Pisum sativum* RS gene SEQ ID NO: 31, resulting in a nucleic acid sequence of SEQ ID NO: 34 and/or a mutated *Ps*RS protein having an amino acid sequence of SEQ ID NO: 36; a deletion of nucleotides 197-209 of *Glycine max* GAS gene SEQ ID NO: 42, resulting in a nucleic acid sequence of SEQ ID NO: 44 and/or a mutated *Gm*GAS protein having an amino acid sequence of SEQ ID NO: 47; a deletion of nucleotides 199-209 of *Glycine max* GAS gene SEQ ID NO: 42, resulting in a nucleic acid sequence of SEQ ID NO: 45 and/or a mutated *Gm*GAS protein having an amino acid sequence of SEQ ID NO: 48; a deletion of nucleotide 204 of SEQ ID NO: 42, resulting in a nucleic acid sequence of SEQ ID NO: 46 and/or a mutated *Gm*GAS protein having an amino acid sequence of SEQ ID NO: 49. Alternatively, the mutation introduced into the plant or plant part according to the methods can comprise an in-frame mutation, e.g., a missense mutation (such as a G128A substitution in the *PsGAS* gene set forth as SEQ ID NO: 5 or 25, resulting in a G43D substitution in the *Ps*GAS protein set forth as SEQ ID NO: 9), or a nonsense mutation (such as a G1044A substitution in the *PsRS* gene set forth as SEQ ID NO: 7 or 31, resulting in a W348* mutation in the *Ps*RS protein set forth as SEQ ID NO: 11), of one or both alleles of at least one (e.g., one, more than one but not all, or all) *GAS* and/or *RS* gene or homolog thereof.

A genetic mutation that decreases the GAS and/or RS activity can be introduced into a gene that is a homolog, ortholog, or variant of a *GAS* and/or *RS* gene disclosed herein and expresses a GAS and/or RS with GAS and/or RS function, or in a regulatory region of such homolog, ortholog, or variant of a *GAS* and/or *RS* gene, according to the methods provided herein.

Variant sequences (e.g., homologs, orthologs) can be isolated by PCR. In this manner, variant sequences encoding GAS and/or RS can be identified and used in the methods of the present disclosure. The variant sequences will retain the GAS and/or RS activity.

In certain instances, mutations introduced into any *GAS* and/or *RS* gene or its regulatory region in a plant, plant part, a population of plants or plant parts, or plant product (e.g., seed composition, plant protein composition) according to the methods provided herein can be identified by a diagnostic method described herein. Such diagnostic methods may comprise use of primers for detecting mutation in a *GAS* and/or *RS* gene. For example, a forward primer and a reverse primer can be used for detection of a mutation in the *Pisum sativum GAS* gene at or near the binding site of a *PsGAS* guide RNA (e.g., SEQ ID NO: 13), e.g., a mutation generated by introducing a nuclease and a *PsGAS* guide RNA (e.g., SEQ ID NO: 13) into the plant or plant part. A forward primer (e.g., SEQ ID NO: 23) and a reverse primer (e.g., SEQ ID NO: 24) can be used for detection of a mutation in the *Pisum sativum RS* gene at or near the binding site of the *PsRS* guide RNA (e.g., SEQ ID NO: 14), e.g., a mutation generated by introducing *PsRS* guide RNA (e.g., SEQ ID NO: 14) into the plant or plant part. A forward primer (e.g., SEQ ID NO: 19), a reverse primer (e.g., SEQ ID NO: 20), and/or a probe can be used for detection of a mutation in the *Pisum sativum GAS* gene at or near nucleotide 128 of SEQ ID NO: 5 or 25, e.g., to detect a G128A mutation in SEQ ID NO: 5 or 25 or a mutation that results in a G43D mutation in SEQ ID NO: 9. A forward primer (e.g., SEQ ID NO: 21), a reverse primer (e.g., SEQ ID NO: 22), and/or a probe can be used for detection of a mutation in the *Pisum sativum RS* gene at or near nucleotide 1044 of SEQ ID NO: 7 or 31, e.g., to detect a G1044A mutation in SEQ ID NO: 7 or 31 or a mutation that results in a W348* mutation in SEQ ID NO: 11.

In some embodiments, the one or more mutations are integrated into the plant genome and the plant or the plant part is stably transformed according to the methods. In other embodiments, the one or more mutations are not integrated into the plant genome and wherein the plant or the plant part is transiently transformed according to the methods.

Introducing one or mutations insertions, substitutions, or deletions into at least one *GAS* and/or *RS* gene or homolog or in a regulatory region of such *GAS* and/or *RS* gene or homolog in the genome of the plant or plant part can reduce the expression levels of the *GAS* and/or RS gene or homolog, reduce level or activity of a GAS and/or RS protein encoded by the *GAS* and/or *RS* gene or homolog, reduce GAS and/or RS activity, and/or increase protein content in the plant, plant part, or a population of plants or plant parts relative to a control plant or plant part, e.g., when grown under the same environmental condition, as further described in the present disclosure.

### 2. Introducing regulatory modifications

The methods described herein can comprise introducing a mutation that decreases the GAS and/or RS activity, e.g., one or more (e.g., about 1, 2, 3, 4, 5, 6, 7, 8, 9, 10, 11, 12, 13, 14, 15, 16, 17, 18, 19, 20, or more) insertions, substitutions, and/or deletions into a regulatory region of at least one (e.g., one, more than one but not all, or all) *GAS* and/or *RS* gene. A "regulatory region" of a gene can include a promoter region, 5'UTR, a genomic site where a RNA polymerase, a transcription factor, or other transcription modulators bind and interact to control mRNA synthesis of the gene, such as a binding site (e.g., enhancer sequence) for transcription modulator proteins (e.g., transcription factors), and other genomic regions that contribute to regulation of transcription of the gene. A regulatory region of the gene can be located in the 5' untranslated region of the gene.

For example, one or more insertions, substitutions, and/or deletions can be introduced into a promoter region, a transcription modulator protein (e.g., transcription factor) binding site, or other regulatory regions of at least one (e.g., one, more than one but not all, or all) *GAS* and/or *RS* gene to confer to the plant or plant part an altered (e.g., reduced) RFO content and/or altered (e.g., increased) sucrose content.

In some embodiments, the methods provided herein include introducing a mutation into a promoter region of at least one (e.g., one, more than one but not all, or all) *GAS* and/or *RS* gene. The one or more insertions, substitutions, and/or deletions in the promoter region of the *GAS* and/or *RS* gene can alter the transcription initiation activity of the promoter. For example, the modified promoter can reduce transcription of the operably linked nucleic acid molecule (e.g., the *GAS* and/or *RS* gene), initiate transcription in a developmentally-regulated or temporally-regulated manner, initiate transcription in a cell-specific, cell-preferred, tissue-specific, or tissue-preferred manner, or initiate transcription in an inducible manner. A deletion, a substitution, or an insertion, e.g., introduction of a heterologous promoter sequence, a cis-acting factor, a motif or a partial sequence from any promoter, including those described elsewhere in the present disclosure, can be introduced into the promoter region of the *GAS* and/or *RS* gene to confer an altered (e.g., reduced) transcription initiation function according to the present disclosure.

The promoter sequence of one or more *GAS* and/or *RS* genes can be inactivated by insertion of one or more (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, or more) nucleotides. Additionally or alternatively, the promoter sequence of one or more of *GAS* and/or *RS* genes can be inactivated by deletion of one or more (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, or more) nucleotides. The promoter sequence of one or more *GAS* and/or *RS* genes can also be inactivated by replacement of the promoter sequence with one or more substitutes. In particular, the substitute can be a cisgenic substitute, a transgenic substitute, or both.

In some instances, the promoter sequence of one or more *GAS* and/or *RS* genes is inactivated by correction of the promoter sequence. A promoter sequence may be corrected by deletion, modification, and/or correction of one or more polymorphisms or mutations that would otherwise enhance the activity of the promoter sequence. In particular, the promoter sequence of one or more *GAS* and/or *RS* genes can be inactivated by: (i) detection of one or more polymorphism or mutation that enhances the activity of the promoter sequence; and (ii) correction of the promoter sequences by deletion, modification, and/or correction of the polymorphism or mutation.

In some instances, the promoter sequence of one or more *GAS* and/or *RS* genes is inactivated by insertion, deletion, and/or modification of one or more (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, or more) upstream nucleotide sequences.

In some instances, the promoter sequence of one or more *GAS* and/or *RS* genes is inactivated by addition, insertion, and/or engineering of cis-acting factors that interact with and modify the promoter sequence.

In some embodiments, a mutation is introduced to locate at least partially in 5'UTR of one or more (e.g., one, more than one but not all, or all) *GAS* and/or *RS* gene, wherein the 5'UTR regulates translation of the main coding sequence (reinitiation of translation, cis- and trans-regulation).

In some embodiments, the method provided herein introduces mutation comprising a deletion of one or more nucleotides at least partially in the promoter and/or 5'UTR of a *Pisum sativum GAS* or *RS* gene.

Function and/or expression of the one or more *GAS* and/or *RS* genes can also be decreased or inhibited by modulation (e.g., increase or decrease) of expression of one or more transcription factor genes. For example, modulation of expression of the one or more transcription factor genes can inactivate or inhibit transcription initiation activity of the promoter of the one or more of *GAS* and/or *RS* genes and/or inhibit expression of the one or more *GAS* and/or *RS* genes.

Function and/or expression of the one or more *GAS* and/or *RS* genes can also be decreased by insertion, modification, and/or engineering of transcription factor binding sites or enhancer elements. For example, insertion of new transcription factor binding sites or enhancer elements can decrease function and/or expression of *GAS* and/or *RS* genes. Alternatively, modification and/or engineering of existing transcription factor binding sites or enhancer elements can decrease function and/or expression of *GAS* and/or *RS* genes.

Function and/or expression of the one or more *GAS* and/or *RS* genes can also be decreased or inhibited by insertion of one or more negative regulatory elements of the gene. For example, to inhibit the expression and/or function of the *GAS* and/or *RS* gene, a part or whole of one or more negative regulatory elements of the *GAS* and/or *RS* gene can be inserted in the genome of a plant cell or plant part. The negative regulatory sequence of the gene can be in a cis location. Alternatively, the negative regulatory sequence of the gene may be in a trans location. Negative regulatory elements of the one or more *GAS* and/or *RS* genes can also include upstream open reading frames (uORFs). In some instances, a negative regulatory sequence can be inserted in a region upstream of the *GAS* and/or *RS* gene in order to inhibit the expression and/or function of the gene.

### 3. RNA interference

GAS and/or RS level or activity in a plant or plant part can be altered by inhibiting or silencing the expression of the *GAS* and/or *RS* gene. Methods of the present disclosure can inhibit expression of the *GAS* and/or *RS* gene in a plant or plant part by RNA interference (RNAi). RNA interference is a biological process in which double-stranded RNA (dsRNA) molecules are involved in sequence-specific suppression of gene expression through translation or transcriptional repression. RNAi can be conducted using two types of small RNA molecules - microRNA (miRNA) and small interfering RNA (siRNA). RNAs are the direct products of genes, and these small RNAs can direct enzyme complexes to degrade messenger RNA (mRNA) molecules and thus decrease their activity by preventing translation, via post-transcriptional gene silencing. Moreover, transcription can be inhibited via the pre-transcriptional silencing mechanism of RNA interference, through which an enzyme complex catalyzes DNA methylation at genomic positions complementary to complexed siRNA or miRNA.

Provided herein are methods for suppressing the expression of a *GAS* and/or *RS* gene by using siRNA and/or miRNA molecules that are directed to the *GAS* and/or *RS* gene or its mRNA transcript. In particular, methods of the present disclosure can inhibit or silence the *GAS* and/or *RS* gene in the genome of cells or parts of a plant by RNA interference, using siRNA and/or miRNA molecules that are directed to the *GAS* and/or *RS* gene.

siRNA and/or miRNA molecules for use in the present methods can be complementary to about 1-23, 2-23, 3-23, 4-23, 5-23, 6-23, 7-23, 8-23, 9-23, or 10-23 (e.g., about 1, 2, 3, 4, 5, 6, 7, 8, 9, 10, 11, 12, 13, 14, 15, 16, 17, 18, 19, 20, 21, 22, or 23) nucleotides of the *GAS* and/or *RS* gene, or the corresponding RNA transcripts.

In some embodiments, the siRNA and/or miRNA molecules can be complementary to a nucleotide region that comprises a nucleic acid sequence having at least 75% (75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100%) sequence identity to the nucleic acid sequence of any one of SEQ ID NOs: 1-8, 25, 31, 37-40, 42, and 50. For example, the siRNA and/or miRNA molecules can be complementary to a nucleotide region that comprises the nucleic acid sequence of any one of SEQ ID NOs: 1-8, 25, 31, 37-40, 42, and 50.

### 4. Reducing GAS and/or RS activity

The methods of the present disclosure (e.g., introducing mutations into a *GAS* and/or *RS* gene or its regulatory region; RNAi; modification of transcriptional regulation of the *GAS* and/or *RS* gene; insertion of a regulatory element) can reduce activity of GAS and/or RS in plants, plant parts (e.g., seeds, leaves), a population of plants or plant parts, or plant products (e.g., seed composition, plant protein composition) compared to a control plant, plant part, a population of plants or plant parts, or plant product. In particular, methods provided herein can reduce the GAS and/or RS activity in the plant, plant part, a population of plants or plant parts, or plant product by about 10-100%, 20-100%, 30-100%, 40-100%, 50-100%, 60-100%, 70-100%, 80-100%, 20-90%, 30-90%, 40-90%, 50-90%, 60-90%, 70-90%, or 100% (e.g., by about 10-20%, 20-30%, 30-40%, 40-50%, 50-60%, 60-70%, 70-80%, 80-90%, or 90-99%, 100%), e.g., by about 10%, 15%, 20%, 25%, 30%, 35%, 40%, 45%, 50%, 55%, 60%, 65%, 70%, 75%, 80%, 85%, 90%, 95%, 99%, or 100%, or at least 10%, 15%, 20%, 25%, 30%, 35%, 40%, 45%, 50%, 55%, 60%, 65%, 70%, 75%, 80%, 85%, 90%, 95%, 99%, or 100%, as compared to a control plant, plant part, a population of plants or plant parts, or plant product.

GAS and/or RS activity in a plant, plant part, or plant product can be measured by in the plant sample by one or more standard methods for measuring enzymatic activity in a plant sample. For example, GAS activity can be determined by contacting UDP-galactose and myo-inositol with a sample obtained from a plant, plant part, or plant product and measuring the level of galactinol, e.g., by high performance liquid chromatography with refractive index detection (HPLC-RI), ion chromatography, size exclusion chromatography with refractive index detection (HPSEC-RI), liquid chromatography with mass spectrometry (LC-MS), derivatization and gas chromatography (GC), or ion chromatography with pulsed amperometric detection (HPAEC-PAD). RS activity can be determined by contacting galactinol with a sample obtained from a plant, plant part, or plant product and measuring the level of raffinose, e.g., by HPLC-RI, HPSEC-RI, LC-MS, GC, HPAEC-PAD.

### 5. Reducing expression level of GAS and/or RS gene or GAS and/or RS protein

The methods provided herein (e.g., introducing mutations into a *GAS* and/or *RS* gene or its regulatory region; RNAi; modification of transcriptional regulation of the *GAS* and/or *RS* gene; insertion of a regulatory element) can reduce the expression levels of *GAS* and/or *RS* gene or homolog in the plant, plant part, population of plants or plant parts, or plant product (e.g., seed composition, plant protein composition) by about 10-100%, 20-100%, 30-100%, 40-100%, 50-100%, 60-100%, 70-100%, 80-100%, 20-90%, 30-90%, 40-90%, 50-90%, 60-90%, 70-90%, or 100% (e.g., by about 10-20%, 20-30%, 30-40%, 40-50%, 50-60%, 60-70%, 70-80%, 80-90%, 90-99%, or 100%), e.g., by about 10%, 15%, 20%, 25%, 30%, 35%, 40%, 45%, 50%, 55%, 60%, 65%, 70%, 75%, 80%, 85%, 90%, 95%, 99%, or 100%, or at least 10%, 15%, 20%, 25%, 30%, 35%, 40%, 45%, 50%, 55%, 60%, 65%, 70%, 75%, 80%, 85%, 90%, 95%, 99%, or 100%, as compared to the expression level of the *GAS* and/or *RS* gene or homolog in a control plant, plant part, population of plants or plant parts, or plant product. The methods can reduce expression levels of *GAS* and/or *RS* gene(s) or homolog in the plant, plant part, a population of plants or plant parts, or plant product of the present disclosure to null or an undetectable level; or alternatively, by more than 0% and less than 100%. Expression levels of the *GAS* and/or *RS* gene or homolog can be measured by any standard methods for measuring mRNA levels of a gene, including quantitative RT-PCR, northern blot, and serial analysis of gene expression (SAGE). Expression levels of the *GAS* and/or *RS* gene or homolog in a plant, plant part, a population of plants or plant parts, or plant product can also be measured by any standard methods for measuring protein levels, including western blot analysis, ELISA, or dot blot analysis of a protein sample obtained from a plant, plant part, a population of plants or plant parts, or plant product using an antibody directed to the GAS and/or RS encoded by the *GAS* and/or *RS* gene.

The methods of the present disclosure (e.g., introducing mutations into a *GAS* and/or *RS* gene or its regulatory region; RNAi; modification of transcriptional regulation of the *GAS* and/or *RS* gene; insertion of a regulatory element) can reduce expression levels of the GAS and/or RS, e.g., the GAS and/or RS encoded by the *GAS* and/or *RS* gene or homolog (having the mutation in the gene or in its regulatory region) in the plant, plant part (e.g., seeds, leaves), a population of plants or plant parts, and plant product (e.g., seed composition, plant protein compositions), as compared to the expression level of the GAS and/or RS in a control plant, plant part, a population of plants or plant parts, or plant product, e.g., a plant, plant part, a population of plants or plant parts, or plant product without such mutation. In particular, the methods provided herein can reduce the expression levels of a full length GAS and/or RS having the complete amino acid sequence of a wild-type GAS and/or RS, e.g., encoded by a native *GAS* and/or *RS* gene in the plant, plant part, a population of plants or plant parts, or plant product (e.g., seed composition, plant protein composition) as compared to a control plant, plant part, a population of plants or plant parts, or plant product. The methods provided herein can introduce a mutation into at least one *GAS* and/or *RS* gene or its regulatory regions in the plant or plant part, which can reduce expression of full-length GAS and/or RS in the plant, plant part, a population of plants or plant parts, or plant product (e.g., seed composition, plant protein composition) as compared to a control plant, plant part, a population of plants or plant parts, or plant product, e.g., product without such mutation, e.g., comprising a native (e.g., wild-type) *GAS* and/or *RS* gene. In particular, the methods provided herein, e.g., introducing one or more insertions, substitutions, or deletions in at least one native *GAS* and/or *RS* gene or homolog or in a regulatory region of such *GAS* and/or *RS* gene or homolog, can reduce expression levels of GAS and/or RS, e.g., full length GAS and/or RS, e.g., encoded by the *GAS* and/or RS gene by about 10-100%, 20-100%, 30-100%, 40-100%, 50-100%, 60-100%, 70-100%, 80-100%, 20-90%, 30-90%, 40-90%, 50-90%, 60-90%, 70-90%, or 100% (e.g., by about 10-20%, 20-30%, 30-40%, 40-50%, 50-60%, 60-70%, 70-80%, 80-90%, 90-99%, or 100%), e.g., by about 10%, 15%, 20%, 25%, 30%, 35%, 40%, 45%, 50%, 55%, 60%, 65%, 70%, 75%, 80%, 85%, 90%, 95%, 99%, or 100%, or at least 10%, 15%, 20%, 25%, 30%, 35%, 40%, 45%, 50%, 55%, 60%, 65%, 70%, 75%, 80%, 85%, 90%, 95%, 99%, or 100%, as compared to expression of GAS and/or RS in a control plant, plant part, a population of plants or plant parts, or plant product. In specific embodiments, the methods completely eliminates expression of the GAS and/or RS; in other specific embodiments, the method decreases, but does not completely eliminate, the expression levels of GAS and/or RS in the plant, plant part, a population of plants or plant parts, or plant product provided herein, i.e., decrease the GAS and/or RS expression levels by more than 0% and less than 100% as compared to a control plant, plant part, a population of plants or plant parts, or plant product. Expression of a GAS and/or RS, such as a full length GAS and/or RS, in a plant, plant part, or plant product can be determined by one or more standard methods of determining protein levels. For example, expression of a GAS and/or RS can be determined by western blot analysis, ELISA, or dot blot analysis of a protein sample obtained from a plant, plant part, or plant product using an antibody directed to the GAS and/or RS, e.g., the full-length GAS and/or RS.

### 6. Reducing or eliminating activity of GAS and/or RS

The methods of the present disclosure (e.g., introducing mutations into a *GAS* and/or *RS* gene or its regulatory region; RNAi; modification of transcriptional regulation of the *GAS* and/or *RS* gene; insertion of a regulatory element) can reduce or eliminate (e.g., reduce to zero) function in the GAS and/or RS, e.g., reduce or eliminate GAS and/or RS activity, as compared to the GAS and/or RS in a control plant, plant part, a population of plants or plant parts, or plant product. A control plant, plant part, a population of plants or plant parts, or plant product can be a plant, plant part, a population of plants or plant parts, or plant product without the mutation, or a plant, plant part, a population of plants or plant parts, or plant product having wild-type GAS and/or RS activity. The methods disclosed herein can produce a GAS and/or RS with loss-of-function or reduced function having a mutation compared to a wild-type GAS and/or RS that causes loss or reduction of GAS and/or RS function. In some embodiments, the methods provided herein can reduce the function of the GAS and/or RS encoded by the *GAS* and/or *RS* gene or homolog to which a mutation (e.g., one or more insertions, substitutions, or deletions) has been introduced in the gene or its regulatory region by about 10-100%, 20-100%, 30-100%, 40-100%, 50-100%, 60-100%, 70-100%, 80-100%, 20-90%, 30-90%, 40-90%, 50-90%, 60-90%, 70-90%, or 100% (e.g., by about 10-20%, 20-30%, 30-40%, 40-50%, 50-60%, 60-70%, 70-80%, 80-90%, 90-99%, or 100%), e.g., by about 10%, 15%, 20%, 25%, 30%, 35%, 40%, 45%, 50%, 55%, 60%, 65%, 70%, 75%, 80%, 85%, 90%, 95%, 99%, or 100%, or at least 10%, 15%, 20%, 25%, 30%, 35%, 40%, 45%, 50%, 55%, 60%, 65%, 70%, 75%, 80%, 85%, 90%, 95%, 99%, or 100% as compared to a control GAS and/or RS encoded by a control *GAS* and/or *RS* gene or homolog without such mutation. In some embodiments, the methods provided herein can reduce the GAS and/or RS activity in the plant, plant part, a population of plants or plant parts, or plant product to which the mutation (e.g., one or more insertions, substitutions, or deletions) has been introduced by about 10-100%, 20-100%, 30-100%, 40-100%, 50-100%, 60-100%, 70-100%, 80-100%, 20-90%, 30-90%, 40-90%, 50-90%, 60-90%, 70-90%, or 100% (e.g., by about 10-20%, 20-30%, 30-40%, 40-50%, 50-60%, 60-70%, 70-80%, 80-90%, 90-99%, or 100%), e.g., by about 10%, 15%, 20%, 25%, 30%, 35%, 40%, 45%, 50%, 55%, 60%, 65%, 70%, 75%, 80%, 85%, 90%, 95%, 99%, or 100%, or at least 10%, 15%, 20%, 25%, 30%, 35%, 40%, 45%, 50%, 55%, 60%, 65%, 70%, 75%, 80%, 85%, 90%, 95%, 99%, or 100% as compared to a control plant, plant part, or plant product, e.g., a plant, plant part, or plant product without such mutation. In specific embodiments, the method completely eliminates (to 0%) the function or GAS and/or RS activity; in other specific embodiments, the method decreases, but does not completely eliminate, the function or activity of GAS and/or RS in the plant, plant part, a population of plants or plant parts, or plant product provided herein, i.e., decrease the GAS and/or RS function or activity by more than 0% and less than 100% as compared to a control plant, plant part, a population of plants or plant parts, or plant product.

GAS and/or RS activity in a plant, plant part, population of plants or plant parts, or plant product can be determined by standard methods for measuring enzymatic activity in a plant sample. For example, GAS activity can be determined by contacting UDP-galactose and myo-inositol with a sample obtained from a plant, plant part, or plant product and measuring the level of galactinol, e.g., by high performance liquid chromatography with refractive index detection (HPLC-RI), ion chromatography, size exclusion chromatography with refractive index detection (HPSEC-RI), liquid chromatography with mass spectrometry (LC-MS), derivatization and gas chromatography (GC), or ion chromatography with pulsed amperometric detection (HPAEC-PAD). RS activity can be determined by contacting galactinol with a sample obtained from a plant, plant part, or plant product and measuring the level of raffinose, e.g., by HPLC-RI, HPSEC-RI, LC-MS, GC, HPAEC-PAD.

### B. Introducing mutations into the genome of plant cells

Introducing one or more mutations into the plant genome, e.g., into at least one *GAS* and/or *RS* gene or its regulatory region, and modulating the level or activity of GAS and/or RS in a plant or plant part may be achieved in any method of creating a change in a nucleic acid of a plant. For example, one or more mutations can be introduced into the plant genome, e.g., into at least one *GAS* and/or *RS* gene (e.g., *PsGAS, PsRS*) or its regulatory region by contacting the plant or plant part (e.g., seeds) with a mutagen. A "mutagen" as used herein refers to an agent (e.g., a physical or chemical agent) that, upon exposure to an organism or a genetic material (e.g., DNA), introduces a mutation into the genetic material. Physical mutagens that can be used in the methods provided herein include electromagnetic radiation, such as gamma rays, X rays, and UV light, and particle radiation, such as fast and thermal neutrons, beta and alpha particles. Fast neutron mutagenesis is conducted by treating the seeds with ionizing radiation and has been effective in plant mutation breeding. It creates large DNA fragment deletions resulting to loss of multiple candidate genes, serious damage to chromosomes, and large segmental duplications (Bolon et al. 2014 Genetics 198:967-981; Oladosu et al. 2016 Biotechnol. Biotechnol. Equip. 30:1-16). The deletions provide gene knock outs and phenotypic variations such as chlorophyll deficiency (Campbell et al. 2015 G3 5:123-131), dwarfism (Hwang et al. 2014 Euphytica 203:399-408), hyper nodulation, and chimeras (Bolon et al. 2011 Plant Physiol 156:240-253).

Chemical mutagens react with DNA and lead to faulty base pairing. Chemical mutagens that can be used in the methods provided herein include alkylating agents. Alkylating agents include ethyl methanesulfonate (EMS), N-ethyl-N-nitrosourea (ENU), nitrogen mustards, mitomycin, methyl methane sulfonate (MMS), diethyl sulfate, and nitrosoguanidine. EMS produces random mutations in genetic material by nucleotide substitution, particularly through G:C to A:T transitions induced by guanine alkylation, typically producing point mutations. ENU produces mutations by transferring the ethyl group of ENU to nucleobases (usually thymine) in nucleic acids. Chemical mutagens that can be used in the methods provided herein also include DNA intercalating agents, such as acridine orange, ethidium bromide (EtBr), proflavin, and daunorubicin. Chemical mutagens that can be used in the methods provided herein further include base analogues, such as halouracils and uridine derivatives, e.g., 5-bromodeoxyuridine (BrdU), which mimic a particular nucleobase in nucleic acid and are misread by the replicating machinery as a normal base. Following incorporation into DNA, they form non-Watson pairing with the DNA. BrdU is capable of inducing point mutations by substituting thymine residues and pairing with guanine instead of adenine. Chemical mutagens that can be used in the methods provided herein also include nitrous acid, hydroxyl amine, and sodium azide, which can modify the bases by deamination, thus modifying the regular base pairing. Nitrous acid deaminates adenine, guanine, and cytosine substituting adenine to hypoxanthine, guanine to xanthine, and cytosine to uracil. These substitutions induce AT to GC transitions leading to faulty base pairing. In specific embodiments, the methods provided herein includes introducing a mutation that reduces GAS and/or RS activity into a plant or plant part by contacting the plant or plant part by EMS and/or ENU, such as contacting the plant or plant part (e.g., seeds) concurrently with EMS and ENU. Mutations produced by a chemical mutagen (e.g., EMS or ENU) can be detected for instance by next generation sequencing and TILLING (Targeting Induced Local Lesion IN Genome). Chemically mutagenized population can be analyzed using two approaches: (1) forward genetics, in which apparent phenotypes are characterized before the underlying gene is identified, and (2) reverse genetics, in which mutations in the genes of interest are detected first and later linked to a specific function or phenotype (Peters et al. 2003 Trends Plant Sci. 8:484-491).

Additionally or alternatively, one or more mutations can be introduced into the plant genome, e.g., into at least one *GAS* and/or *RS* gene (e.g., *Pisum sativum GAS* or *RS,* e.g., *Pisat.03G015560*, *Pisat.06G066360, Pisat.05G054130*, *Pisat.01G021000*, *Glycine max GAS* or *RS,* e.g., *Glyma.03G229800*, *Glyma.19G227800*) or its regulatory region through the use of precise genome-editing technologies to modulate the expression of the endogenous or transgenic sequence. In this manner, a nucleic acid sequence can be inserted, substituted, or deleted proximal to or within a native plant sequence corresponding to at least one *GAS* and/or *RS* gene through the use of methods available in the art. Such methods include, but are not limited to, use of a nuclease designed against the plant target genomic sequence of interest (D'Halluin et al 2013 Plant Biotechnol J 11: 933-941), such as the Type II CRISPR system, the Type V CRISPR system, the CRISPR-Cas9 system, the CRISPR-Cas12a (Cpfl) system, the transcription activator-like effector nuclease (TALEN) system, the zinc finger nuclease (ZFN) system, and other technologies for precise editing of genomes [Feng et al. 2013 Cell Research 23:1229-1232, Podevin et al. 2013 Trends Biotechnology 31: 375-383, Wei et al. 2013 J Gen Genomics 40:281-289, Zhang et al (2013) WO 2013/026740, Zetsche et al. 2015 Cell 163:759-771]; *Natronobacterium gregoryi Argonaute-mediated* DNA insertion (Gao et al. 2016 Nat Biotechnol doi:10.1038/nbt.3547); Cre-lox site-specific recombination (Dale et al. 1995 Plant J 7:649-659; Lyznik, et al. 2007 Transgenic Plant J 1:1-9; FLP-FRT recombination (Li et al. 2009 Plant Physiol 151:1087-1095); Bxb1-mediated integration (Yau et al. 2011 Plant J 701:147-166); zinc-finger mediated integration (Wright et al. 2005 Plant J 44:693-705); Cai et al. 2009 Plant Mol Biol 69:699-709); and homologous recombination (Lieberman-Lazarovich and Levy 2011 Methods MolBiol 701: 51-65; Puchta 2002 Plant Mol Biol 48:173-182). Reagents and compositions that can be used for introducing one or more mutations into plants or plant parts according to the methods of the present disclosure are herein described.

### 1. Editing reagent

Inserting, substituting, or deleting one or more nucleotides at a precise location of interest in at least one *GAS* and/or *RS* gene and/or a regulatory region of the *GAS* and/or *RS* gene in a plant or plant part may be achieved by introducing into the plant or plant part a system (e.g., a gene editing system), reagents (e.g., editing reagents), or a construct for introducing mutations at the target site of interest in a genome of a plant cell. A "gene editing system," "editing system," "gene editing reagent," and "editing reagent" as used herein, refer to a set of one or more molecules or a construct comprising or encoding the one or more molecules for introducing one or more mutations in the genome. An exemplary gene editing system or editing reagents comprise a nuclease and/or a guide RNA. Also disclosed herein is a construct (e.g., a DNA construct, a recombinant DNA construct) for introducing one or more mutations in plants or plant parts. A construct can comprise an editing system or polynucleotides encoding editing reagents (e.g., nuclease, guide RNA, base editor) each operably linked to a promoter.

As used herein, the terms "nuclease" or "endonuclease" refers to naturally-occurring or engineered enzymes, which cleave a phosphodiester bond within a polynucleotide chain. Nucleases that can be used in precise genome-editing technologies to modulate the expression of the native sequence (e.g., at least one *GAS* and/or *RS* gene and/or a regulatory region of the *GAS* and/or *RS* gene) include, but are not limited to, meganucleases designed against the plant genomic sequence of interest (D'Halluin et al (2013) Plant Biotechnol J 11: 933-941); Cas9 endonuclease; Cas12a (Cpfl) endonuclease; ortholog of Cas 12a endonuclease; Cms1 endonuclease; transcription activator-like effector nucleases (TALENs); zinc finger nucleases (ZFNs); and a deactivated CRISPR nuclease (e.g., a deactivated Cas9, Cas12a, or Cms1 endonuclease) fused to a transcriptional regulatory element (Piatek et al. (2015) Plant Biotechnol J 13:578-589). In some embodiments, the editing system or the editing reagents comprise a zinc finger nuclease (ZFN), a transcription activator-like effector nuclease (TALEN), and/or a clustered regularly interspaced short palindromic repeats (CRISPR) nuclease. In some embodiments, the editing reagents comprise a CRISPR nuclease. In some embodiments, the CRISPR nuclease is a Cas12a nuclease, herein used interchangeably with a Cpf1 nuclease, e.g., a McCpf1 nuclease. In some embodiments, the CRISPR nuclease is a Cas12a nuclease ortholog, e.g., Lb5Cas12a, CMaCas12a, BsCas12a, BoCas12a, MlCas12a, Mb2Cas12a, TsCas12a, and MAD7 endonucleases.

A nuclease system can introduce insertion, substitution, or deletion of genetic elements at a predefined genomic locus by causing a double-strand break at said predefined genomic locus and, optionally, providing an appropriate DNA template for insertion. This strategy is well-understood and has been demonstrated previously to insert a transgene at a predefined location in the cotton genome (D'Halluin et al. 2013 Plant Biotechnol. J. 11: 933-941). For example, a Cas12a (Cpfl) endonuclease coupled with a guide RNA (gRNA) designed against the genomic sequence of interest (i.e., at least one *GAS* and/or *RS* gene and/or a regulatory region of the *GAS* and/or *RS* gene) can be used (i.e., a CRISPR-Cas 12a system). Alternatively, a Cas9 endonuclease coupled with a gRNA designed against the genomic sequence of interest (a CRISPR-Cas9 system), or a Cms1 endonuclease coupled with a gRNA designed against the genomic sequence of interest (a CRISPR-Cms1) can be used. Other nuclease systems for use with the methods of the present invention include the CRISPR systems (e.g., Type I, Type II, Type III, Type IV, and/or Type V CRISPR systems (Makarova et al 2020 Nat Rev Microbiol 18:67-83)) with their corresponding gRNA(s), the TALEN system, the ZFN system, the meganuclease system, and the like. Alternatively, a deactivated CRISPR nuclease (e.g., a deactivated Cas9, Cas12a, or Cms1 endonuclease) fused to a transcriptional regulatory element can be targeted to the regulatory region (e.g., upstream regulatory region) of at least one *GAS* and/or *RS* gene, thereby modulating the transcription of the *GAS* and/or *RS* gene (Piatek et al. 2015 Plant Biotechnol J 13:578-589). Site-specific introduction of mutations of plant cells by biolistic introduction of a ribonucleoprotein comprising a nuclease and suitable guide RNA has been demonstrated (Svitashev et al. 2016 Nat Commun doi: 10.1038/ncomms13274), and is herein incorporated by reference. For example, a CRISPR system comprises a CRISPR nuclease (e.g., CRISPR-associated (Cas) endonuclease or variant or ortholog thereof, such as Cas12a or Cas12a ortholog) and a guide RNA. A CRISPR nuclease associates with a guide RNA that directs nucleic acid cleavage by the associated endonuclease by hybridizing to a recognition site in a polynucleotide. The guide RNA directs the nuclease to the target site and the endonuclease cleaves DNA at the target site. The guide RNA comprises a direct repeat and a guide sequence, which is complementary to the target recognition site. In certain embodiments, the CRISPR system further comprises a tracrRNA (trans-activating CRISPR RNA) that is complementary (fully or partially) to the direct repeat sequence present on the guide RNA. The CRISPR-Cas12a system may comprise at least one guide RNA (gRNA) operatively arranged with the ortholog endonuclease for genomic editing of a target DNA binding the gRNA. The system may comprise a CRISPR-Cas12a expression system encoding the Cas12a ortholog nucleases and crRNAs (CRISPR RNAs) for forming gRNAs that are coactive with the Cas12a nucleases. A "TALEN" nuclease is an endonuclease comprising a DNA-binding domain comprising a plurality of TAL domain repeats fused to a nuclease domain or an active portion thereof from an endonuclease or exonuclease, including but not limited to a restriction endonuclease, homing endonuclease, and yeast HO endonuclease. A "zinc finger nuclease" or "ZFN" refers to a chimeric protein comprising a zinc finger DNA-binding domain fused to a nuclease domain from an endonuclease or exonuclease, including but not limited to a restriction endonuclease, homing endonuclease, and yeast HO endonuclease.

The editing system, editing reagents, or construct described herein can comprise one or more guide RNAs (gRNAs). "Guide RNA" as used herein refers to a RNA molecule that function as guides for RNA- or DNA-targeting enzymes, e.g., nucleases. To introduce one or more mutations into at least one *GAS* and/or *RS* gene and/or the promoter region of the *GAS* and/or *RS* gene, antisense constructions, complementary to at least a portion of the sequence of the *GAS* and/or *RS* gene messenger RNA (mRNA), *GAS* and/or *RS* gene, or regulatory region of the *GAS* and/or *RS* gene can be constructed. Antisense nucleotides are designed to hybridize with the corresponding mRNA or genomic nucleic acid sequence. Modifications of the antisense sequences may be made as long as the sequences hybridize to and interfere with expression of the corresponding mRNA or genomic sequence. In this manner, antisense constructions having at least 75%, optimally 80%, more optimally 85%, 90%, 95% or greater sequence identity to the corresponding sequences to be edited may be used. Furthermore, portions of the antisense nucleotides may be used to disrupt the expression of the target gene.

Accordingly, a gene editing system, editing reagents, or a construct of the present disclosure can contain a guide RNA (gRNA) cassette, comprising one or more gRNAs or encoding one or more gRNAs, to drive mutations at the locus of at least one *GAS* and/or *RS* gene or the regulatory region of the *GAS* and/or *RS* gene. The one or more gRNAs can be designed to specifically target a regulatory region (e.g., promoter, 5'UTR) of a *GAS* and/or *RS* gene, or exons or introns of a *GAS* and/or *RS* gene.

For example, the gRNA can be specific to a nucleic acid sequence having at least 80% (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) sequence identity to the nucleic acid sequence of any one of SEQ ID NOs: 1-8, 25, 31, 37-40, 42, and 50. The gRNA can be specific to the nucleic acid sequence of any one of SEQ ID NOs: 1-8, 25, 31, 37-40, 42, and 50 and/or can drive a deletion at least partially in the 5' regulatory region (e.g., promoter, 5'UTR), exons, and/or introns of the *Pisum sativum GAS* and/or *RS* gene (e.g., *Pisat.03G015560*, *Pisat.06G066360*, *Pisat.05G054130*, *Pisat.01G021000*) or active homolog thereof. In particular instances, the gRNA targets the *PsGAS* gene and its targeting region is encoded by a nucleic acid sequence having at least 75% (75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100%) sequence identity with SEQ ID NO: 13. The targeting region of the gRNA can be encoded by the nucleic acid sequence of SEQ ID NO: 13. In particular instances, the gRNA targets the *PsRS* gene and its targeting region is encoded by a nucleic acid sequence having at least 75% (75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100%) sequence identity with SEQ ID NO: 14. The targeting region of the gRNA can be encoded by the nucleic acid sequence of SEQ ID NO: 14. In particular instances, the gRNA targets the *GmGAS* gene and its targeting region is encoded by a nucleic acid sequence having at least 75% (75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100%) sequence identity with SEQ ID NO: 52. The targeting region of the gRNA can be encoded by the nucleic acid sequence of SEQ ID NO: 52. The gRNA can facilitate binding of an RNA guided nuclease that cleaves a region of at least one a *GAS* and/or *RS* gene or a regulatory region of the *GAS* and/or *RS* gene, and cause non-homologous end joining or homology-directed repair to introduce a mutation at the cleavage site.

The methods provided herein can comprise introducing into the plant, plant part, or plant cell two or more (e.g., 2, 3, 4, 5, 6, 7, 8, 9, 10, or more) gRNAs specific to a nucleic acid sequence having at least 75% (75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100%) sequence identity to the nucleic acid sequence of any one of SEQ ID NOs: 1-8, 25, 31, 37-40, 42, and 50. The two or more gRNA can be specific to the nucleic acid sequence of any one of SEQ ID NOs: 1-8, 25, 31, 37-40, 42, and 50 (e.g., have a targeting region encoded by a nucleic acid sequence having at least 80% sequence identity with the nucleic acid sequence of SEQ ID NO: 13 and/or 14) and/or can drive one or more deletions at least partially in the 5' regulatory region (e.g., promoter, 5'UTR), exons, and/or introns of the *Pisum sativum GAS* and/or RS gene (e.g., *Pisat.03G015560*, *Pisat.06G066360, Pisat.05G054130*, *Pisat.01G021000*), or active homolog thereof in the plant, plant part, or plant cell. In some instances, introducing two or more gRNAs along with other editing reagents (e.g., nuclease) into the plant, plant part, or plant cell increases sequence diversity of mutations (e.g., insertions, substitutions, deletions) generated at or near the target site, as compared to introducing one gRNA.

In some instances, a gRNA may comprise a targeting region (i.e., spacer) that is complementary to a targeted sequence as well as another region that allows the gRNA to form a complex with a nuclease (e.g., a CRISPR nuclease) of interest. The targeting region (i.e. spacer) of a gRNA that binds to the region of at least one *GAS* and/or *RS* gene or a regulatory region of the *GAS* and/or *RS* gene for use in the method described herein above can be about 100-300 nucleotides long with the targeting region therein about 10-40 nucleotides long (e.g., 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 nucleotides long). For example, the targeting region of a gRNA for use in the method described herein may be 24 nucleotides in length. In some embodiments, the targeting region of a gRNA is encoded by a nucleic acid sequence comprising a nucleic acid sequence having at least 75% (e.g., 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100%) sequence identity to the nucleic acid sequence of SEQ ID NO: 13, 14, or 52. In particular instances, the targeting region of a gRNA for use in the method described herein is encoded by a nucleic acid sequence comprising the nucleic acid sequence of SEQ ID NO: 13, 14, or 52. The methods provided herein can comprise introducing into the plant, plant part, or plant cell one or more (e.g., 1, 2, 3, 4, 5, 6, 7, 8, 9, 10, or more) gRNAs, at least one of which comprising a nucleic acid sequence encoded by a nucleic acid sequence that shares at least 80% (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) sequence identity with the nucleic acid sequence of SEQ ID NO: 13, 14, or 52, or a nucleic acid sequence of SEQ ID NO: 13, 14, or 52.

The gRNA or a combination of two or more gRNAs provided herein can introduce a mutation at least partially in the 5' regulatory region (e.g., promoter, 5'UTR) or the coding region (e.g., exons, introns) of a *Pisum sativum GAS* and/or *RS* gene, e.g., *Pisat.03G015560*, *Pisat.06G066360, Pisat.05G054130*, *Pisat.01G021000*, e.g., comprising a nucleic acid sequence of any one of SEQ ID NOs: 1-8, 25, 31, and 37-40, or a *Glycine max GAS* gene, e.g., *Glyma.19G227800*, *Glyma.03G229800*, comprising a nucleic acid sequence of SEQ ID NO: 42 and 50, in the plant, plant part, or plant cell. For example, the one or more gRNAs provided herein can direct a nuclease to a specific target site at a genomic region (e.g., of a *Pisum sativum GAS* and/or *RS* gene, a *Glycine max GAS* and/or *RS* gene) and introduce a mutation at the garget site into the plant, plant part, or plant cell.

In some embodiments, a gene editing efficiency of the one or more gRNAs is greater than 0.5% (e.g., 0.5%, 1%, 1.5%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 99%, or 100%). In specific embodiments, the methods do not introduce mutations into at least one allele comprising at least one *GAS* and/or *RS* gene and its regulatory region. In some embodiments, the methods introduce mutations into all alleles each comprising a *GAS* and/or *RS* gene and its regulatory region.

Editing system or editing reagents can also include base editing components. For example, cytosine base editing (CBE) reagents, which change a C-G base pair to a T-A base pair, comprise a single guide RNA, a nuclease (e.g., dCas9, CAS9 nickase), a cytidine deaminase (e.g., APOBEC1), and a uracil DNA glycosylase inhibitor (UGI). Adenine base editing (ABE) reagents, which change an A-T base pair to a G-C base pair comprise a deaminase, (TadA), a nuclease (e.g., dCas or Cas nickase), and a guide RNA.

The gene editing system (e.g., CRISPR-Cas12a system), editing reagents, or a construct of the present disclosure can comprise at least one CRISPR RNA (crRNA) regulatory element operably linked to at least one nucleotide sequence encoding a crRNA for producing gRNA for targeting a target sequence, and at least one regulatory element, which may be the same as or different from the crRNA regulatory element, operably linked to a nucleotide sequence encoding the endonuclease, for generation of a CRISPR editing structure (e.g., CRISPR-Cas12a editing structure) by which the gRNA targets the target sequence and the CRISPR endonuclease cleaves a target DNA to alter gene expression in the cell, and wherein the CRISPR-associated nuclease, and the gRNA, do not naturally occur together. In such system, the at least one crRNA regulatory element may comprise one or more than one RNA polymerase II (Pol II) promoter, or alternatively, a single transcript unit (STU) regulatory element, or one or more of ZmUbi, OsU6, OsU3, and U6 promoters.

The methods described herein, comprising introducing into such plant a non-naturally occurring heterologous CRISPR-Cas12a genomic editing system of a type as variously described herein, can cause the editing reagents to introduce mutations in at least one *GAS* and/or *RS* gene or a regulatory region of the *GAS* and/or *RS* gene and alter the level or activity of a GAS and/or RS protein gene or GAS and/or RS. The gene editing system (e.g., the CRISPR-Cas12a system) can target PAM sites such as TTN, TTV, TTTV, NTTV, TATV, TATG, TATA, YTTN, GTTA, and/or GTTC.

Such methods of introducing mutations into plants, plant parts, or plant cells may be carried out at moderate temperatures, e.g., below 25°C. and above temperature producing freezing or frost damage of the plant. The methods provided herein may be performed on a wide variety of plants. In particular embodiments, the methods provided herein can be carried out to introduce mutations into the *Pisum sativum* plant at one or more *GAS* and/or *RS* genes or a regulatory region of the *GAS* and/or *RS* gene.

Methods disclosed herein are not limited to certain techniques of mutagenesis. Any method of creating a change in a nucleic acid of a plant can be used in conjunction with the disclosed invention, including the use of chemical mutagens (e.g. EMS, ENU, methanesulfonate, sodium azide, aminopurine, etc.), genome/gene editing techniques (e.g. CRISPR-like technologies, TALENs, zinc finger nucleases, and meganucleases), physical mutagens (e.g., ionizing radiation (e.g. ultraviolet, gamma rays)), T-DNA, transposons, temperature alterations, long-term seed storage, tissue culture conditions, targeting induced local lesions in a genome, sequence-targeted and/or random recombinases, etc. It is anticipated that new methods of creating a mutation in a nucleic acid of a plant will be developed and yet fall within the scope of the claimed invention when used with the teachings described herein. Any editing system or editing reagents for use in any genome-editing methods including those described herein can be expressed in a plant or plant part.

### 2. Promoter

As used herein, "promoter" refers to a regulatory region of DNA that is capable of driving expression of a sequence in a plant or plant cell. A number of promoters may be used in the practice of the disclosure, e.g., to express editing reagents in plants, plant parts, or plant cells. The promoter may have a constitutive expression profile. Constitutive promoters include the CaMV 35S promoter (Odell et al. (1985) Nature 313:810-812); rice actin (McElroy et al. (1990) Plant Cell 2:163-171); ubiquitin (Christensen et al. (1989) Plant Mol. Biol. 12:619-632 and Christensen et al. (1992) Plant Mol. Biol. 18:675-689); pEMU (Last et al. (1991) Theor. Appl. Genet. 81:581-588); MAS (Velten et al. (1984) EMBO J. 3:2723-2730); ALS promoter (U.S. Patent No. 5,659,026), and the like.

Alternatively, promoters for use in the methods of the present disclosure can be tissue-preferred promoters. Tissue-preferred promoters include Yamamoto et al. (1997) Plant J. 12(2):255-265; Kawamata et al. (1997) Plant Cell Physiol. 38(7):792-803; Hansen et al. (1997) Mol. Gen Genet. 254(3):337-343; Russell et al. (1997) Transgenic Res. 6(2):157-168; Rinehart et al. (1996) Plant Physiol. 112(3):1331-1341; Van Camp et al. (1996) Plant Physiol. 112(2):525-535; Canevascini et al. (1996) Plant Physiol. 112(2):513-524; Yamamoto et al. (1994) Plant Cell Physiol. 35(5):773-778; Lam (1994) Results Probl. Cell Differ. 20:181-196; Orozco et al. (1993) Plant Mol Biol. 23(6): 1129-1138; Matsuoka et al. (1993) Proc Natl. Acad. Sci. USA 90(20):9586-9590; and Guevara-Garcia et al. (1993) Plant J. 4(3):495-505. Leaf-preferred promoters are also known in the art. See, for example, Yamamoto et al. (1997) Plant J. 12(2):255-265; Kwon et al. (1994) Plant Physiol. 105:357-67; Yamamoto et al. (1994) Plant Cell Physiol. 35(5):773-778; Gotor et al. (1993) Plant J. 3:509-18; Orozco et al. (1993) Plant Mol. Biol. 23(6): 1129-1138; and Matsuoka et al. (1993) Proc. Natl. Acad. Sci. USA 90(20):9586-9590.

Alternatively, promoters for use in the methods of the present disclosure can be developmentally-regulated promoters. Such promoters may show a peak in expression at a particular developmental stage. Such promoters have been described in the art, e.g., US Patent No. 10,407,670; Gan and Amasino (1995) Science 270: 1986-1988; Rinehart et al. (1996) Plant Physiol 112: 1331-1341; Gray-Mitsumune et al. (1999) Plant Mol Biol 39: 657-669; Beaudoin and Rothstein (1997) Plant Mol Biol 33: 835-846; Genschik et al. (1994) Gene 148: 195-202, and the like.

Alternatively, promoters for use in the methods of the present disclosure can be promoters that are induced following the application of a particular biotic and/or abiotic stress. Such promoters have been described in the art, e.g., Yi et al. (2010) Planta 232: 743-754; Yamaguchi-Shinozaki and Shinozaki (1993) Mol Gen Genet 236: 331-340; U.S. Patent No. 7,674,952; Rerksiri et al. (2013) Sci World J2013: Article ID 397401; Khurana et al. (2013) PLoS One 8: e54418; Tao et al. (2015) Plant Mol Biol Rep 33: 200-208, and the like.

Alternatively, promoters for use in the methods of the present disclosure can be cell-preferred promoters. Such promoters may preferentially drive the expression of a downstream gene in a particular cell type such as a mesophyll or a bundle sheath cell. Such cell-preferred promoters have been described in the art, e.g., Viret et al. (1994) Proc Natl Acad USA 91: 8577-8581; U.S. Patent No. 8,455,718; U.S. Patent No. 7,642,347; Sattarzadeh et al. (2010) Plant Biotechnol J 8: 112-125; Engelmann et al. (2008) Plant Physiol 146: 1773-1785; Matsuoka et al. (1994) Plant J 6: 311-319, and the like.

It is recognized that a specific, non-constitutive expression profile may provide an improved plant phenotype relative to constitutive expression of a gene or genes of interest. For instance, many plant genes are regulated by light conditions, the application of particular stresses, the circadian cycle, or the stage of a plant's development. These expression profiles may be important for the function of the gene or gene product *in planta.* One strategy that may be used to provide a desired expression profile is the use of synthetic promoters containing cis-regulatory elements that drive the desired expression levels at the desired time and place in the plant. Cis-regulatory elements that can be used to alter gene expression *in planta* have been described in the scientific literature (Vandepoele et al. (2009) Plant Physiol 150: 535-546; Rushton et al. (2002) Plant Cell 14: 749-762). *Cis*-regulatory elements may also be used to alter promoter expression profiles, as described in Venter (2007) Trends Plant Sci 12: 118-124.

### 3. Transfer DNA

Nucleic acid molecules comprising transfer DNA (T-DNA) sequences can be used in the practice of the disclosure, e.g., to express editing reagents in plants, plant parts, or plant cells. For example, a construct of the present disclosure may contain T-DNA of tumor-inducing (Ti) plasmid of *Agrobacterium tumefaciens.* Alternatively, a recombinant DNA construct of the present disclosure may contain T-DNA of tumor-inducing (Ti) plasmid of *Agrobacterium rhizogenes.* The *vir* genes of the Ti plasmid may help in transfer of T-DNA of a recombinant DNA construct into nuclear DNA genome of a host plant. For example, Ti plasmid of *Agrobacterium tumefaciens* may help in transfer of T-DNA of a recombinant DNA construct of the present disclosure into nuclear DNA genome of a host plant, thus enabling the transfer of a gRNA of the present disclosure into nuclear DNA genome of a host plant (e.g., a pea plant).

### 4. Regulatory signal

Construct described herein may contain regulatory signals, including, but not limited to, transcriptional initiation sites, operators, activators, enhancers, other regulatory elements, ribosomal binding sites, an initiation codon, termination signals, and the like. See, for example, U.S. Pat. Nos. 5,039,523 and 4,853,331; EPO 0480762A2; Sambrook et al. (1992) Molecular Cloning: A Laboratory Manual, ed. Maniatis et al. (Cold Spring Harbor Laboratory Press, Cold Spring Harbor, N.Y.), hereinafter "Sambrook 11"; Davis et al., eds. (1980) Advanced Bacterial Genetics (Cold Spring Harbor Laboratory Press), Cold Spring Harbor, N.Y., and the references cited therein.

### 5. Reporter genes / selectable marker genes

Reporter genes or selectable marker genes may be included in the expression cassettes of the present invention. Examples of suitable reporter genes known in the art can be found in, for example, Jefferson, et al., (1991) in Plant Molecular Biology Manual, ed. Gelvin, et al., (Kluwer Academic Publishers), pp. 1-33; DeWet, et al., (1987) Mol. Cell. Biol. 7:725-737; Goff, et al., (1990) EMBO J. 9:2517-2522; Kain, et al., (1995) Bio Techniques 19:650-655 and Chiu, et al., (1996) Current Biology 6:325-330, herein incorporated by reference in their entirety.

Selectable marker genes for selection of transformed cells or tissues can include genes that confer antibiotic resistance or resistance to herbicides. Examples of suitable selectable marker genes include, but are not limited to, genes encoding resistance to chloramphenicol (Herrera Estrella, et al., (1983) EMBO J. 2:987-992); methotrexate (Herrera Estrella, et al., (1983) Nature 303:209-213; Meijer, et al., (1991) Plant Mol. Biol. 16:807-820); hygromycin (Waldron, et al., (1985) Plant Mol. Biol. 5:103-108 and Zhijian, et al., (1995) Plant Science 108:219-227); streptomycin (Jones, et al., (1987) Mol. Gen. Genet. 210:86-91); spectinomycin (Bretagne-Sagnard, et al., (1996) Transgenic Res. 5:131-137); bleomycin (Hille, et al., (1990) Plant Mol. Biol. 7:171-176); sulfonamide (Guerineau, et al., (1990) Plant Mol. Biol. 15:127-36); bromoxynil (Stalker, et al., (1988) Science 242:419-423); glyphosate (Shaw, et al., (1986) Science 233:478-481 and US Patent Application Serial Numbers 10/004,357 and 10/427,692); phosphinothricin (DeBlock, et al., (1987) EMBO J. 6:2513-2518), herein incorporated by reference in their entirety.

Selectable marker genes include genes encoding antibiotic resistance, such as those encoding neomycin phosphotransferase II (NEO), spectinomycin/streptinomycin resistance (SpcR, AAD), and hygromycin phosphotransferase (HPT or HGR) as well as genes conferring resistance to herbicidal compounds. Herbicide resistance genes generally code for a modified target protein insensitive to the herbicide or for an enzyme that degrades or detoxifies the herbicide in the plant before it can act. For example, resistance to glyphosate has been obtained by using genes coding for mutant target enzymes, 5-enolpyruvylshikimate-3-phosphate synthase (EPSPS). Genes and mutants for EPSPS are well known, and further described below. Resistance to glufosinate ammonium, bromoxynil, and 2,4-dichlorophenoxyacetate (2,4-D) have been obtained by using bacterial genes encoding PAT or DSM-2, a nitrilase, an AAD-1, or an AAD-12, each of which are examples of proteins that detoxify their respective herbicides.

Herbicides can inhibit the growing point or meristem, including imidazolinone or sulfonylurea, and genes for resistance/tolerance of acetohydroxyacid synthase (AHAS) and acetolactate synthase (ALS) for these herbicides are well known. Glyphosate resistance genes include mutant 5-enolpyruvylshikimate-3-phosphate synthase (EPSPs) and dgt-28 genes (via the introduction of recombinant nucleic acids and/or various forms of in vivo mutagenesis of native EPSPs genes), aroA genes and glyphosate acetyl transferase (GAT) genes, respectively). Resistance genes for other phosphono compounds include bar and pat genes from *Streptomyces* species, including *Streptomyces hygroscopicus* and *Streptomyces viridichromogenes*, and pyridinoxy or phenoxy proprionic acids and cyclohexones (ACCase inhibitor-encoding genes). Exemplary genes conferring resistance to cyclohexanediones and/or aryloxyphenoxypropanoic acid (including haloxyfop, diclofop, fenoxyprop, fluazifop, quizalofop) include genes of acetyl coenzyme A carboxylase (ACCase); Accl-S1, Accl-S2 and Accl-S3. Herbicides can also inhibit photosynthesis, including triazine (psbA and 1s+ genes) or benzonitrile (nitrilase gene). Further, such selectable markers can include positive selection markers such as phosphomannose isomerase (PMI) enzyme.

Selectable marker genes can further include, but are not limited to genes encoding: 2,4-D; SpcR; neomycin phosphotransferase II; cyanamide hydratase; aspartate kinase; dihydrodipicolinate synthase; tryptophan decarboxylase; dihydrodipicolinate synthase and desensitized aspartate kinase; bar gene; tryptophan decarboxylase; neomycin phosphotransferase (NEO); hygromycin phosphotransferase (HPT or HYG); dihydrofolate reductase (DHFR); phosphinothricin acetyltransferase; 2,2-dichloropropionic acid dehalogenase; acetohydroxyacid synthase; 5-enolpyruvyl-shikimate-phosphate synthase (aroA); haloarylnitrilase; acetyl-coenzyme A carboxylase; dihydropteroate synthase (sul I); and 32 kD photosystem II polypeptide (psbA). Selectable marker genes can further include genes encoding resistance to: chloramphenicol; methotrexate; hygromycin; spectinomycin; bromoxynil; glyphosate; and phosphinothricin.

Other selectable marker genes that could be employed on the expression constructs disclosed herein include, but are not limited to, GUS (beta-glucuronidase; Jefferson, (1987) Plant Mol. Biol. Rep. 5:387), GFP (green fluorescence protein; Chalfie, et al., (1994) Science 263:802), luciferase (Riggs, et al., (1987) Nucleic Acids Res. 15(19):8115 and Luehrsen, et al., (1992) Methods Enzymol. 216:397-414), red fluorescent protein (DsRFP, RFP, etc), beta-galactosidase, and the maize genes encoding for anthocyanin production (Ludwig, et al., (1990) Science 247:449), and the like (See Sambrook, et al., Molecular Cloning: A Laboratory Manual, Third Edition, Cold Spring Harbor Press, N.Y., 2001), herein incorporated by reference in their entirety. The above list of selectable marker genes is not meant to be limiting. Any reporter or selectable marker gene are encompassed by the present disclosure.

### 6. Terminator

A transcription terminator may also be included in the expression cassettes of the present invention. Plant terminators are known in the art and include those available from the Ti-plasmid of *A. tumefaciens*, such as the octopine synthase and nopaline synthase termination regions. See also Guerineau et al. (1991) Mol. Gen. Genet. 262:141-144; Proudfoot (1991) Cell 64:671-674; Sanfacon et al. (1991) Genes Dev. 5:141-149; Mogen et al. (1990) Plant Cell 2:1261-1272; Munroe et al. (1990) Gene 91:151-158; Ballas et al. (1989) Nucleic Acids Res. 17:7891-7903; and Joshi et al. (1987) Nucleic Acids Res. 15:9627-9639.

### 7. Vector

Disclosed herein are vectors containing constructs (e.g., recombinant DNA constructs encoding editing reagents) of the present disclosure. As used herein, "vector" refers to a nucleotide molecule (e.g., a plasmid, cosmid), bacterial phage, or virus for introducing a nucleotide construct, for example, a recombinant DNA construct, into a host cell. Cloning vectors typically contain one or a small number of restriction endonuclease recognition sites at which foreign DNA sequences can be inserted in a determinable fashion without loss of essential biological function of the vector, as well as a marker gene that is suitable for use in the identification and selection of cells transformed with the cloning vector. Marker genes typically include genes that provide tetracycline resistance, hygromycin resistance or ampicillin resistance. In some embodiments, provided herein are expression cassettes located on a vector comprising gRNA sequence specific for at least one *GAS* and/or *RS* gene or a regulatory region of the *GAS* and/or *RS* gene.

In some embodiments, a vector is a plasmid containing a recombinant DNA construct of the present disclosure. For example, the present disclosure may provide a plasmid containing a recombinant DNA construct that comprises a gRNA to drive mutations at the locus of at least one *GAS* and/or *RS* gene or the regulatory region of the *GAS* and/or *RS* gene.

In some embodiments, a vector is a recombinant virus containing a recombinant DNA construct of the present disclosure. For example, the present disclosure may provide a recombinant virus containing a recombinant DNA construct that comprises a gRNA, wherein the gRNA can drive mutations at the locus of at least one *GAS* and/or *RS* gene or the regulatory region of the *GAS* and/or *RS* gene. A recombinant virus described herein can be a recombinant lentivirus, a recombinant retrovirus, a recombinant cucumber mosaic virus (CMV), a recombinant tobacco mosaic virus (TMV), a recombinant cauliflower mosaic virus (CaMV), a recombinant odontoglossum ringspot virus (ORSV), a recombinant tomato mosaic virus (ToMV), a recombinant bamboo mosaic virus (BaMV), a recombinant cowpea mosaic virus (CPMV), a recombinant potato virus X (PVX), a recombinant Bean yellow dwarf virus (BeYDV), or a recombinant turnip vein-clearing virus (TVCV).

### 8. Cells

Also provided herein are cells comprising the reagent (e.g., editing reagent, e.g., nuclease, gRNA), the system (e.g., gene editing system), the construct (e.g., expression cassette), and/or the vector of the present disclosure for introducing mutations into at least one *GAS* and/or *RS* gene and/or a regulatory region of the *GAS* and/or *RS* gene. The cell can be a plant cell, a bacterial cell, and a fungal cell. The cell can be a bacterium, e.g., an *Agrobacterium tumefaciens*, containing the gRNA targeting at least one *GAS* and/or *RS* gene and/or a regulatory region of the *GAS* and/or *RS* gene and driving mutations at the target site of interest. The cells of the present disclosure may be grown, or have been grown, in a cell culture.

### C. Decreasing RFO content in plants

The methods of the present disclosure, by introducing a mutation that decreases GAS and/or RS activity, e.g., comprising one or more insertions, substitutions, or deletions in at least one native *GAS* and/or *RS* gene or homolog or in a regulatory region of such *GAS* and/or *RS* gene or homolog in plants, plant parts, or plant cells and/or regenerating plants from transformed cells, can decrease RFO (e.g., raffinose, stachyose) content and/or increase sucrose content in the plants, plant parts (e.g., seeds, leaves), a population of plants or plant parts, or plant products (e.g., seed composition, plant protein composition) as compared to a control plant, plant part, population of plants or plant parts, or plant product, e.g., without such mutation.

A control plant or plant part can be a plant or plant part to which a mutation provided herein has not been introduced, e.g., by methods of the present disclosure. Thus, a control plant, plant part, a population of plants or plant parts, or plant product may express a native (e.g., wild-type) *GAS* and/or *RS* gene endogenously or transgenically, and/or may have a wild-type GAS and/or RS activity. The methods provided herein can decrease RFO (e.g., raffinose, stachyose) content and/or increase sucrose content in plant, plant part, a population of plants or plant parts, or plant product as compared to a control plant, plant part, a population of plants or plant parts, or plant product, when the plant or plant part of the present disclosure is grown under the same environmental conditions (e.g., same or similar temperature, humidity, air quality, soil quality, water quality, and/or pH conditions) as the control plant or plant part.

In some embodiments, the methods can decrease raffinose and/or stachyose content by about 10-100%, 20-100%, 30-100%, 40-100%, 50-100%, 60-100%, 70-100%, 80-100%, 20-90%, 30-90%, 40-90%, 50-90%, 60-90%, or 70-90% (e.g., by about 10-20%, 20-30%, 30-40%, 40-50%, 50-60%, 60-70%, 70-80%, 80-90%, or 90-100%), by about 10%, 15%, 20%, 25%, 30%, 35%, 40%, 45%, 50%, 55%, 60%, 65%, 70%, 75%, 80%, 85%, 90%, 95%, or 100%, or at least 10%, 15%, 20%, 25%, 30%, 35%, 40%, 45%, 50%, 55%, 60%, 65%, 70%, 75%, 80%, 85%, 90%, 95%, or 100% as compared to a control plant, plant part, population, or plant product. In specific embodiments, the methods can reduce raffinose content in the plant, plant part, population of plants or plant parts, and/or plant product by about 15%, at least about 15%, about 85%, at least about 85%, or at least about 15-85%, such that the plant, plant part, population, or plant product comprises raffinose content that is about 85%, 85% or less, about 15%, 15% or less, or about 15%-85% or less as compared to a control plant, plant part, population, or plant product. In specific embodiments, the methods can decrease stachyose content in the plant, plant part, population of plants or plant parts, and/or plant product provided herein is reduced by about 50%, at last about 50%, about 70%, at least about 70%, about 80%, at least about 80%, at least about 50-80%, or at least about 70-80%, such that the plant, plant part, population, or plant product comprises stachyose content that is about 50%, 50% or less, 30%, 30% or less, about 20%, 20% or less, about 20-50% or less, or about 20-30% or less as compared to a control plant, plant part, population, or plant product. Without wishing to be bound by theory, seeds of a reference variety of pea cultivar may contain about 0.8-0.9% dry weight of raffinose and about 4.5-5.0% dry weight of stachyose. Seeds of a reference variety of soybean cultivar may contain about 0.7-0.9% dry weight of raffinose and about 4.0-4.5% dry weight of stachyose. The methods provided herein can reduce the seed raffinose content to about 0.7% or less, 0.6% or less, 0.5% or less, 0.4% or less, 0.3% or less, 0.2% or less, 0.1% or less; about 0.1-0.7%, 0.1-0.6%, 0.1-0.5%, 0.1-0.4%, 0.1-0.3%, 0.1-0.2%, 0.2-0.3%, 0.3-0.4%, 0.4-0.5%, 0.5-0.6%, 0.6-0.7%, 0.5-0.7%, 0.4-0.7%, 0.3-0.7%, or 0.2-0.7%; or about 0.7%, 0.6%, 0.5%, 0.4%, 0.3%, 0.2%, or 0.1% dry weight in the plant, plant part, population of plants or plant parts (e.g., pea), or a population of seeds (e.g., pea). The methods can reduce the seed stachyose content to about 1.5% or less, 1.4% or less, 1.3% or less, 1.2% or less, 1.1% or less, 1.0% or less, 0.9% or less, 0.8% or less; about 0.6-1.5%, 0.6-1.4%, 0.6-1.3%, 0.6-1.2%, 0.6-1.1%, 0.6-1.0%, 0.6-0.9%, 0.9-1.0%, 1.0-1.1%, 1.1-1.2%, 1.2-1.3%, 1.3-1.4%, 1.2-1.4%, 1.1-1.4%, 1.0-1.4%, 0.9-1.4%, 0.7%-1.5%, 0.8%-1.5%, 0.9%-1.5%, 1.0%-1.5%; or about 1.5%, 1.4%, 1.3%, 1.2%, 1.1%, 1.0%, 0.9%, or 0.8% dry weight in the plant, plant part, population of plants or plant parts (e.g., pea), or a population of seeds (e.g., pea).

In some embodiments, the methods provided herein can increase the sucrose content in the plant, plant part, population of plants or plant parts, and/or plant product by about 5-100%, 10-100%, 20-100%, 30-100%, 40-100%, 50-100%, 60-100%, 70-100%, 80-100%, 20-90%, 30-90%, 40-90%, 50-90%, 60-90%, 70-90%, 100-150%, 150-200%, or more than 200%, e.g., by about 10%, 15%, 20%, 25%, 30%, 35%, 40%, 45%, 50%, 55%, 60%, 65%, 70%, 75%, 80%, 85%, 90%, 95%, 100%, 150%, 200%, or more, or at least 10%, 15%, 20%, 25%, 30%, 35%, 40%, 45%, 50%, 55%, 60%, 65%, 70%, 75%, 80%, 85%, 90%, 95%, 100%, 150%, 200%, or more as compared to a control plant or plant part. In specific embodiments, the methods can increase sucrose content in the plant, plant part, population of plants or plant parts, and/or plant product by about 10%, at least about 10%, about 80%, at least about 80%, or at least about 10-80%, as compared to a control plant, plant part, population, or plant product. Without wishing to be bound by theory, seeds of a reference variety of pea cultivar may contain about 2.4-3.4% dry weight of sucrose. In contrast, the methods can increase the seed sucrose content to about 3.0% or more, 3.5% or more, 3.7% or more, 4.0% or more, 4.5% or more, 5.0% or more, 5.5% or more, 6.0% or more, 6.2% or more; about 3.0-6.2%, 3.7-6.2%, 4.0-6.2%, 4.5-6.2%, 5.0-6.2%, 5.5-6.2%, 6.0-6.2%, 3.7-4.0%, 4.0-4.5%, 4.5-5.0%, 5.0-5.5%, 5.5-6.2%, 6.0-6.2%, 5.0-6.2%, 4.5-6.2%, 4.0-6.2%, 3.5-6.2%, 3.0-6.2%, 3.5-6.2%; or about 3.0%, 3.7%, 4.0%, 4.5%, 5.0%, 5.5%, 6.0%, or 6.2% dry weight in the plant, plant part, population of plants or plant parts (e.g., pea), or a population of seeds (e.g., pea).

Content of raffinose, stachyose, and sucrose in a plant, plant part, population of plants or plant parts, or plant product can be measured by any standard methods, including spectroscopy (near infrared spectroscopy), refractometry, high performance liquid chromatography with refractive index detection (HPLC-RI), ion chromatography, size exclusion chromatography with refractive index detection (HPSEC-RI), liquid chromatography with mass spectrometry (LC-MS), derivatization and gas chromatography (GC), and ion chromatography with pulsed amperometric detection (HPAEC-PAD).

In specific embodiments, the methods provided herein decrease RFO (e.g., raffinose, stachyose) content and/or increased sucrose content in a plant, plant part, population of plants or plant parts, or plant product, without a significant decrease in yield or germination rate. In some embodiments, a reduction in yield or germination rate in the plant, plant part, or population of plants or plant parts of the present disclosure, having decreased RFO (e.g., raffinose, stachyose) content and/or increased sucrose content, is no more than about 0.5%, 1.0%, 1.5%, 2.0%, 2.5%, 3.0%, 3.5%, 4.0%, 4.5%, or about 5%, 6%, 7%, 8%, 9%, or 10%, e.g., no more than about 0-5%, 0.5-4.5%, 0.5-4%, 1-5%, 1-4%, 2-5%, 2-4%, 0.5-10%, 0.5-8%, 1-10%, 2-10%, 3-10%, 4-10%, 5-10%, 6-10%, 7-10%, or 8-10% as compared to a control plant, plant part, or population of plants or plant parts. Yield and germination rate can be measured and expressed by any means known in the art. In specific embodiments, yield is measured by seed weight or volume of seeds, fruits, leaves, or whole plants harvested from a given harvest area. Germination rate can be measured as percent of germinated plants over total seeds that were seeded.

In specific embodiments, the methods provided herein can decrease GAS and/or RS activity in a population of seeds, decrease seed RFO (e.g., raffinose, stachyose) content, and/or increase seed sucrose content as compared to control population. For example, the methods provided herein can decrease GAS and/or RS activity, decrease RFO content, and/or increased sucrose content in pea seeds or a population of pea seeds, such that the pea seeds have raffinose content of about 0.7% or less, stachyose content of about 1.4% or less, and/or sucrose content of about 3.7% or more.

### D. Increasing protein content in plants

The methods of the present disclosure, by introducing a mutation that decreases GAS and/or RS activity, e.g., comprising one or more insertions, substitutions, or deletions in at least one native *GAS* and/or *RS* gene or homolog or in a regulatory region of such *GAS* and/or *RS* gene or homolog in plants, plant parts, or plant cells and/or regenerating plants from transformed cells, can increase protein content in the plants, plant parts (e.g., seeds, leaves), a population of plants or plant parts, or plant products (e.g., seed composition, plant protein composition) as compared to a control plant, plant part, population of plants or plant parts, or plant product, e.g., without such mutation.

A control plant, plant part, a population of plants or plant parts, or plant product can comprise a plant or plant part to which a mutation provided herein has not been introduced, e.g., by methods of the present disclosure. Thus, a control plant, plant part, a population of plants or plant parts, or plant product may express a native (e.g., wild-type) *GAS* and/or *RS* gene endogenously or transgenically, and/or may have a wild-type GAS or RS polypeptide activity. A plant, plant part, a population of plants or plant parts, or plant product of the present disclosure may have increased protein content as compared to a control plant, plant part, a population of plants or plant parts, or plant product, when the plant or plant part of the present disclosure is grown under the same environmental conditions (e.g., same or similar temperature, humidity, air quality, soil quality, water quality, and/or pH conditions) as the control plant or plant part.

In some embodiments, the methods can increase total protein content in a plant, plant part, population of plants or plant parts, or plant product by about 0.1-0.5%, 0.5-1.0%, 1-10%, 10-100%, 20-100%, 30-100%, 40-100%, 50-100%, 60-100%, 70-100%, 80-100%, 20-90%, 30-90%, 40-90%, 50-90%, 60-90%, 70-90%, 100-1000%, 200-1000%, 300-1000%, 400-1000%, 500-1000%, 600-1000%, 700-1000%, 800-1000%, 200-900%, 300-900%, 400-900%, 500-900%, 600-900%, 700-900%, or more than 1000% (e.g., by about 10-20%, 20-30%, 30-40%, 40-50%, 50-60%, 60-70%, 70-80%, 80-90%, 90-100%, 100-200%, 200-300%, 300-400%, 400-500%, 500-600%, 600-700%, 700-800%, 800-900%, 900-1000%, or more than 1000%), e.g., by about 0.1%, 0.5%, 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 100%, 200%, 300%, 400%, 500%, 600%, 700%, 800%, 900%, 1000%, or more, or at least 10%, 15%, 20%, 25%, 30%, 35%, 40%, 45%, 50%, 55%, 60%, 65%, 70%, 75%, 80%, 85%, 90%, 95%, 100%, 200%, 300%, 400%, 500%, 600%, 700%, 800%, 900%, 1000%, or more as compared to a control plant, plant part, population of plants or plant parts, or plant product. In specific embodiments, the methods can increase protein content in a plant, plant part, population of plants or plant parts, and/or plant product by about 5%, at least about 5%, about 10%, at least about 10%, about 20%, at least about 20%, or at least about 5-20%, as compared to a control plant, plant part, population, or plant product. In some embodiments, the methods can increase total protein content, as expressed by % dry weight, in a plant, plant part, population of plant or plant parts, or plant product by about 0.25-10%, 0.5-10%, 0.75-10%, 1.0-10%, 1.5-10%, 2-10%, 2.5-10%, 3-10%, 3.5-10%, 4-10%, 4.5-10%, 5-10%, 6-10%, 7-10%, 8-10%, 9-10%, or more than 10% dry weight (e.g., by about 0.25-0.5%, 0.5-0.75%, 0.75-1.0%, 1.0-1.5%, 1.5-2.0%, 2.0-2.5%, 2.5-3.0%, 3.0-3.5%, 3.5-4.0%, 4.0-4.5%, 4.5-5.0%, 5-6%, 6-7%, 7-8%, or 8-9%, 9-10%, or more than 10% dry weight), by about 0.25%, 0.5%, 0.75%, 1.0%, 1.5%, 2.0%, 2.5%, 3.0%, 3.5%, 4.0%, 4.5%, 5%, 6%, 7%, 8%, 9%, or 10% dry weight, or more, or at least 0.25%, 0.5%, 0.75%, 1.0%, 1.5%, 2.0%, 2.5%, 3.0%, 3.5%, 4.0%, 4.5%, 5%, 6%, 7%, 8%, 9%, or 10% dry weight, or more (by subtraction) as compared to a control plant, plant part, population, or plant product. In specific embodiments, the methods can increase protein content in a plant, plant part, population of plants or plant parts, and/or plant product by about 2%, at least about 2%, about 5%, at least about 5%, about 10%, at least about 10%, or at least about 5-20% dry weight (by subtraction) as compared to a control plant, plant part, population, or plant product.

In some embodiments, the methods can increase protein content in seeds or a population of seeds as compared to control seeds or a control population of seeds (e.g., control seeds or population having a native GAS and/or RS protein, reference seeds or population, commodity seeds or population). The seeds can be legume seeds, e.g., pea seeds. Where typical pea cultivars average approximately 20-30% protein in the seed in dry weight (Meng & Cloutier, 2014 Microencapsulation in the Food Industry: A Practical Implementation Guide § 20.5), the methods can increase protein content in pea seeds or a population of pea seeds to at least 35%, 36%, 37%, 38%, 39%, 40%, 41%, 42%, 43%, 44%, 45%, 46%, 47%, 48%, 49%, 50%, or more by dry weight.

Protein content in a plant, plant part, plant product, or a population of plants or plant parts can be measured by standard methods for measuring total and specific amino acids in a plant sample, for example by high performance liquid chromatography (HPLC), spectrophotometer, mass spectrometry (MS), and combination thereof. Protein content in a plant sample can be measured by standard methods, for example by protein extraction and quantitation (e.g., BCA protein assay, Lowry protein assay, Bradford protein assay), spectroscopy, near-infrared reflectance (NIR) (e.g., analyzing 700 - 2500 nm), and nuclear magnetic resonance spectrometry (NMR). In specific embodiments, protein content is measured by the Dumas method, by combusting samples at a high temperature in the presence of high-purity oxygen, analyzing the gas from combustion for nitrogen content using a thermal conductivity detector, and calculating the amount of protein present in the sample using a conversion factor. The industry standard conversion factor for pea is 6.25.

In specific embodiments, the methods provided herein can produce seeds and a population of seeds with decreased GAS and/or RS activity, having increased protein content as compared to control seeds or a population of seeds. For example, the methods can produce pea seeds or a population of pea seeds with decreased GAS and/or RS activity and increased protein content, having seed protein content of about 40% or more.

The methods can decrease RFO content as well as increase protein content in a plant, plant part, a population of plants or plant parts, or plant product as compared to a control plant, plant part, population of plants or plant parts, or plant product. For example, the methods can reduce raffinose content by about 15%, at least about 15%, about 85%, at least about 85%, or at least about 15-85% in a plant, plant part, population of plants or plant parts, and/or plant product, such that the plant, plant part, population, or plant product comprises raffinose content that is about 85%, 85% or less, about 15%, 15% or less, or about 15%-85% or less as compared to a control plant, plant part, population, or plant product. Additionally or alternatively, the methods can reduce stachyose content in the plant, plant part, population of plants or plant parts, and/or plant product by about 50%, at last about 50%, about 70%, at least about 70%, about 80%, at least about 80%, at least about 50-80%, or at least about 70-80%, such that the plant, plant part, population, or plant product comprises stachyose content that is about 50%, 50% or less, 30%, 30% or less, about 20%, 20% or less, about 20-50% or less, or about 20-30% or less as compared to a control plant, plant part, population, or plant product. Additionally or alternatively, the methods can increase sucrose content in the plant, plant part, population of plants or plant parts, and/or plant product by about 10%, at least about 10%, about 80%, at least about 80%, or at least about 10-80%, as compared to a control plant, plant part, population, or plant product. At the same time, the methods can increase protein content in the plant, plant part, population of plants or plant parts, and/or plant product by about 5%, at least about 5%, about 10%, at least about 10%, about 20%, at least about 20%, or at least about 5-20% as compared to a control plant, plant part, population, or plant product.

In specific embodiments, the methods can produce seeds and a population of seeds with decreased GAS and/or RS activity, having decreased RFO (e.g., raffinose, stachyose) content (and/or increased sucrose content), as well as increased protein content, as compared to control seeds or a population of seeds. For example, the methods can produce pea seeds or a population of pea seeds with decreased GAS and/or RS activity, decreased RFO content (and/or increased sucrose content), as well as increased protein content, having seed raffinose content of about 0.7% or less, seed stachyose content of about 1.4% or less, and/or seed sucrose content of about 3.7%; as well as seed protein content of 40% or more.

### E. Plants, plant parts, population, and plant products produced by present methods

The present disclosure provides plants, plant parts, a population of plants or plant parts, and plant products produced according to the methods provided herein. Such plants, plant parts, population of plants or plant parts, and plant products can have reduced GAS and/or RS activity compared to a control plant, plant part, population, or plant product. In the population of plants or plant parts provided herein, having reduced GAS and/or RS level or activity relative to a control population, not all individual plants or plant parts need to have altered (e.g., decreased) GAS and/or RS level or activity, genetic mutation that cause altered (e.g., decreased) GAS and/or RS level or activity, or phenotypes caused by the altered (e.g., decreased) GAS and/or RS activity (e.g., decreased RFO (e.g., raffinose, stachyose) content, increased sucrose content). In specific embodiments at least 80%, 81%, 82%, 83%, 84%, 85%, 86%, 87%, 88%, 89%, 90%, 91%, 92%, 93%, 94%, 95%, 96%, 97%, 98%, 99%, or more plants within a given plant population have a mutation that alters the GAS and/or RS level or activity. A "plant part" produced according to the methods described herein can include any part of a plant, including seeds (e.g., a representative sample of seeds), plant cells, embryos, pollen, ovules, leaves, flowers, branches, fruit, kernels, ears, cobs, husks, stalks, roots, root tips, anthers, plant protoplasts, plant cell tissue cultures from which plants can be regenerated, plant calli, plant clumps, juice, pulp, nectar, stems, branches, and bark. A "plant product," as used herein, refers to any composition derived from the plant or plant part, including any composition derived from the plant or plant part, including any oil products, sugar products, fiber products, protein products (such as protein concentrate, protein isolate, flake, or other protein product), seed hulls, meal, or flour, for a food, feed, aqua, or industrial product, plant extract (e.g., sweetener, antioxidants, alkaloids, etc.), plant concentrate (e.g., whole plant concentrate or plant part concentrate), plant powder (e.g., formulated powder, such as formulated plant part powder (e.g., seed flour)), plant biomass (e.g., dried biomass, such as crushed and/or powdered biomass), grains, plant protein composition, plant oil composition, and food and beverage products containing plant compositions (e.g., plant parts, plant extract, plant concentrate, plant powder, plant protein, plant oil, and plant biomass) described herein. Plant parts and plant products provided herein can be intended for human or animal consumption.

A "protein product" or "protein composition" obtained from the plants or plant parts produced according to the methods provided herein can include any protein composition or product isolated, extracted, and/or produced from plants or plant parts (e.g., seed) and includes isolates, concentrates, and flours, e.g., plant protein composition, pea/soy protein concentrate (PPC/SPC), pea/soy protein isolate (PPI/SPI), plant flour, flake, white flake, texturized vegetable protein (TVP), or textured pea/soy protein (TPP/TSP)). Plant protein compositions obtained from the plants or plant parts produced according to the methods provided herein can be a concentrated protein solution in which the protein is in a higher concentration than the protein in the plant from which the protein composition is derived. The protein composition can comprise multiple proteins as a result of the extraction or isolation process. The plant protein composition can further comprise stabilizers, excipients, drying agents, desiccating agents, anti-caking agents, or any other ingredient to make the protein fit for the intended purpose. The protein composition can be a solid, liquid, gel, or aerosol and can be formulated as a powder. The protein composition can be extracted in a powder form from a plant and can be processed and produced in different ways, such as: *(i) as an isolate -* through the process of wet fractionation, which has the highest protein concentration; *(ii) as a concentrate* - through the process of dry fractionation, which are lower in protein concentration; and/or *(iii) in textured form* - when it is used in food products as a substitute for other products, such as meat substitution (e.g. a "meat" patty). "White flake" or "white flake protein" refers to a protein composition obtained by de-hulling, flaking, and defattening plants or plant parts (e.g., legume plants or plant parts) by solvent (e.g., hexane) extraction, with limited use of heat to run off the solvent (Lusas and Riaz, 1995). White flake protein is an intermediate product in the production of plant protein concentrates and isolates. In contrast to conventional toasted plant meal, white flakes contains undenaturated proteins due to the very mild heat treatment. Thus, little or no reduction of protease inhibitors would be expected. The undenaturated proteins in white flakes may be advantageous in supporting binding properties during production of the extruded compound feed. White flakes can be used for human and animal consumption, including as a source of protein in aquaculture feeds for any type of fish or aquatic animal in a farmed or wild environment.

In specific embodiments, the plant protein compositions provided herein are obtained from a yellow pea *(Pisum sativum)* plant or plant part produced according to the methods of the present disclosure, e.g., a pea plant or plant part to which a mutation that decreases GAS and/or RS activity, e.g., one or more insertions, substitutions, or deletions is introduced into at least one native *GAS* and/or *RS* gene or homolog or into a regulatory region of such *GAS* and/or *RS* gene or homolog.

Also provided herein are food and/or beverage products obtained from the plants, plant parts, or plant compositions (e.g., seed composition, plant protein compositions) produced according to the methods of the present disclosure. Such food and/or beverage products can be meant for human or animal consumption, and can include animal feed, shakes (e.g., protein shakes), health drinks, alternative meat products (e.g., meatless burger patties, meatless sausages), alternative egg products (e.g., eggless mayo), non-dairy products (e.g., non-dairy whipped toppings, non-dairy milk, non-dairy creamer, non-dairy milk shakes, non-diary ice cream), energy bars (e.g., protein energy bars), infant formula, baby foods, cereals, baked goods, edamame, tofu, and tempeh.

Plant parts (e.g., seeds) and plant products (e.g., plant biomass, seed compositions, protein compositions, food and/or beverage products) produced by the methods provided herein can be meant for consumption by agricultural animals or for use as feed in an agriculture or aquaculture system. In specific embodiments, plant parts and plant products produced according to the methods provided herein include animal feed (e.g., roughages - forage, hay, silage; concentrates - cereal grains, pea cake, soybean cake) intended for consumption by bovine, porcine, poultry, lambs, goats, or any other agricultural animal. In some embodiments, plant parts and plant products produced according to the methods include aquaculture feed for any type of fish or aquatic animal in a farmed or wild environment including, without limitation, trout, carp, catfish, salmon, tilapia, crab, lobster, shrimp, oysters, clams, mussels, and scallops.

The plants, plant parts, and plant products, including plant protein compositions and plant-based food/beverage products produced according to the methods of the present disclosure can contain a mutation that decreases GAS and/or RS activity, e.g., one or more insertions, substitutions, or deletions in at least one native *GAS* and/or *RS* gene or homolog or in a regulatory region of such *GAS* and/or *RS* gene or homolog. The plants, plant parts, and plant products produced according to the methods of the present disclosure can have reduced GAS and/or RS activity, reduced expression level of the *GAS* and/or *RS* gene or homolog, reduced expression level of the GAS and/or RS (e.g., the full-length GAS and/or RS) encoded by the *GAS* and/or *RS* gene, loss of function or reduced function or GAS and/or RS activity encoded by the *GAS* and/or *RS* gene, decreased RFO (e.g., raffinose, stachyose) content, increased sucrose content, and/or increased protein content compared to a control plant part or plant product, e.g., without the mutation, comprising a native (e.g., wild-type) *GAS* and/or *RS* gene or GAS and/or RS, or comprising wild-type GAS and/or RS activity. For example, pea seeds, a population of pea seeds, or pea composition (e.g., flour, PPC) produced according to the methods disclosed herein can have a mutation that decreases GAS and/or RS activity, decreased GAS and/or RS activity, decreased RFO content, increased sucrose content, and/or increased protein content, such as raffinose content of about 0.7% or less or 0.3% or less, stachyose content of about 1.5% or less or 1.4% or less, and/or sucrose content of about 3.0% or more or 3.7% or more. In specific embodiments, the pea seeds or pea compositions (e.g., flour, PPC) provided herein have raffinose content of about 0.3% or less, stachyose content of about 1.5% or less, and sucrose content of about 3.0% or more. The pea seeds or pea compositions can have protein content of 40% or more. Further, pea seeds or a population of pea seeds produced according to the methods disclosed herein can have a mutation that decreases GAS and/or RS activity, decreased GAS and/or RS activity, decreased RFO content, increased sucrose content, as well as increased protein content, such as (i) seed raffinose content of about 0.7% or less or 0.3% or less, seed stachyose content of about 1..5% or less or 1.4% or less, and/or seed sucrose content of about 3.0% or more or 3.7% or more, and (ii) seed protein content of 40% or more.

### F. Transformation of plants

Provided herein are methods for transforming plants or plant parts by introducing into the plants or plant parts one or more mutations (e.g., insertions, substitutions, and/or deletions) to at least one *GAS* and/or *RS* gene and/or a regulatory region of the *GAS* and/or *RS* gene. The methods can comprise introducing a system (e.g., a gene editing system), reagents (e.g., editing reagents), or a construct for introducing mutations at the target site of interest.

The term "transform" or "transformation" as used herein refers to any method used to introduce genetic mutations (e.g., insertions substitutions, or deletions in the genome), polypeptides, or polynucleotides into plant cells. For purpose of the present disclosure, the transformation can be "stable transformation," wherein the one or more mutations (e.g., in at least one *GAS* and/or *RS* gene and/or a regulatory region of the *GAS* and/or *RS* gene) or the transformation constructs (e.g., a construct comprising a nucleic acid molecule encoding a gRNA and/or a nuclease for use in the methods of the present invention) are introduced into a host (e.g., a host plant, plant part, plant cell, etc.), integrate into the genome of the host, and are capable of being inherited by the progeny thereof; or "transient transformation," wherein the one or more mutations (e.g., in at least one *GAS* and/or *RS* gene and/or a regulatory region of the *GAS* and/or *RS* gene) or the transformation constructs (e.g., a construct comprising a gRNA and/or a gene encoding a nuclease for use in the methods of the present invention) are introduced into a host (e.g., a host plant, plant part, plant cell, etc.) and expressed temporarily. The methods disclosed herein can also be used for insertion of heterologous genes and/or modification of native plant gene expression to achieve desirable plant traits, e.g., decreased RFO (e.g., raffinose, stachyose) content and/or increased sucrose content.

Any mutation or any polynucleotide of interest (e.g., editing reagents, e.g., a nuclease and a guide RNA) can be introduced into a plant cell, organelle, or plant embryo by a variety of means of transformation, including mutagenesis by contacting the plant, plant part, plant cell, organelle, or plant embryo with a mutagen (e.g., EMS, ENU), microinjection (Crossway et al. (1986) Biotechniques 4:320-334), electroporation (Riggs et al. (1986) Proc. Natl. Acad. Sci. USA 83:5602-5606, *Agrobacterium*-mediated transformation (U.S. Patent No. 5,563,055 and U.S. Patent No. 5,981,840), direct gene transfer (Paszkowski et al. (1984) EMBO J. 3:2717-2722), and ballistic particle acceleration [see, for example, U.S. Patent Nos. 4,945,050; U.S. Patent No. 5,879,918; U.S. Patent No. 5,886,244; and, 5,932,782; Tomes et al. (1995) in Plant Cell, Tissue, and Organ Culture: Fundamental Methods, ed. Gamborg and Phillips (Springer-Verlag, Berlin); McCabe et al. (1988) Biotechnology 6:923-926); and Lec1 transformation (WO 00/28058). Also see Weissinger et al. (1988) Ann. Rev. Genet. 22:421-477; Sanford et al. (1987) Particulate Science and Technology 5:27-37 (onion); Christou et al. (1988) Plant Physiol. 87:671-674 (soybean); McCabe et al. (1988) Bio/Technology 6:923-926 (soybean); Finer and McMullen (1991) In Vitro Cell Dev. Biol. 27P: 175-182 (soybean); Singh et al. (1998) Theor. Appl. Genet. 96:319-324 (soybean); Datta et al. (1990) Biotechnology 8:736-740 (rice); Klein et al. (1988) Proc. Natl. Acad. Sci. USA 85:4305-4309 (maize); Klein et al. (1988) Biotechnology 6:559-563 (maize); U.S. Patent Nos. 5,240,855; 5,322,783; and, 5,324,646; Klein et al. (1988) Plant Physiol. 91:440-444 (maize); Fromm et al. (1990) Biotechnology 8:833-839 (maize); Hooykaas-Van Slogteren et al. (1984) Nature (London) 311:763-764; U.S. Patent No. 5,736,369 (cereals); Bytebier et al. (1987) Proc. Natl. Acad. Sci. USA 84:5345-5349 (*Liliaceae*); De Wet et al. (1985) in The Experimental Manipulation of Ovule Tissues, ed. Chapman et al. (Longman, New York), pp. 197-209 (pollen); Kaeppler et al. (1990) Plant Cell Reports 9:415-418 and Kaeppler et al. (1992) Theor. Appl. Genet. 84:560-566 (whisker-mediated transformation); D'Halluin et al. (1992) Plant Cell 4:1495-1505 (electroporation); Li et al. (1993) Plant Cell Reports 12:250-255 and Christou and Ford (1995) Annals of Botany 75:407-413 (rice); Osjoda et al. (1996) Nature Biotechnology 14:745-750 (maize via *Agrobacterium tumefaciens*)]: all of which are herein incorporated by reference.

The embodiments disclosed herein are not limited to certain methods of introducing nucleic acids into a plant, and are not limited to certain forms or structures that the introduced nucleic acids take. Any method of transforming a cell of a plant described herein with nucleic acids are incorporated into the teachings of this innovation. *Agrobacterium-and* biolistic-mediated transformation remain the two predominantly employed approaches. However, transformation may be performed by infection, transfection, microinjection, electroporation, microprojection, biolistics or particle bombardment, electroporation, silica/carbon fibers, ultrasound mediated, PEG mediated, calcium phosphate co-precipitation, polycation DMSO technique, DEAE dextran procedure, viral infection, *Agrobacterium* and viral mediated (Caulimoriviruses, Geminiviruses, RNA plant viruses), liposome mediated and the like. Methods disclosed herein are not limited to any size of nucleic acid sequences that are introduced, and thus one could introduce a nucleic acid comprising a single nucleotide (e.g. an insertion) into a nucleic acid of the plant and still be within the teachings described herein. Nucleic acids introduced in substantially any useful form, for example, on supernumerary chromosomes (e.g. B chromosomes), plasmids, vector constructs, additional genomic chromosomes (e.g. substitution lines), and other forms is also anticipated. It is envisioned that new methods of introducing nucleic acids into plants and new forms or structures of nucleic acids will be discovered and yet fall within the scope of the claimed invention when used with the teachings described herein.

More than one polynucleotides of interest can be introduced into the plant, plant cell, plant organelle, or plant embryo simultaneously or sequentially. For example, different editing reagents, e.g., nuclease polypeptides (or encoding nucleic acid), guide RNAs (or DNA molecules encoding the guide RNAs), donor polynucleotide(s), and/or repair templates can be introduced into the plant cell, organelle, or plant embryo simultaneously or sequentially. The amount or ratio of more than one polynucleotides of interest, or molecules encoded therein, can be adjusted by adjusting the amount or concentration of the polynucleotides and/or timing and dosage of introducing the polynucleotides into the plant or plant part. For example, the ratio of the nuclease (or encoding nucleic acid) to the guide RNA(s) (or encoding DNA) to be introduced into plants or plant parts generally will be about stoichiometric such that the two components can form an RNA-protein complex with the target DNA. In one embodiment, DNA encoding a nuclease and DNA encoding a guide RNA are delivered together within a plasmid vector.

Alteration of the GAS and/or RS level or activity in plants, plant parts, or plant cells may also be achieved through the use of transposable element technologies to alter gene expression. It is well understood that transposable elements can alter the expression of nearby DNA (McGinnis et al. (1983) Cell 34:75-84).

Alteration of the GAS and/or RS level or activity may be achieved by inserting a transposable element into at least one *GAS* and/or *RS* gene and/or a regulatory region of the *GAS* and/or *RS* gene.

The cells that have been transformed may be grown into plants (i.e., cultured) in accordance with conventional ways. See, for example, McCormick et al. (1986) Plant Cell Reports 5:81-84. In this manner, the present invention provides transformed plants or plant parts, transformed seed (also referred to as "transgenic seed") or transformed plant progenies having a nucleic acid modification stably incorporated into their genome.

The present invention may be used for transformation of any plant species, e.g., both monocots and dicots (including legumes). Plants or plant parts to be transformed according to the methods disclosed herein can be a legume, i.e., a plant belonging to the family Fabaceae (or Leguminosae), or a part (e.g., fruit or seed) of such a plant. When used as a dry grain, the seed of a legume is also called a pulse. Examples of legume include, without limitation, pea *(Pisum sativum),* soybean (*Glycine max*), beans (*Phaseolus* spp., *Vigna* spp.), common bean (*Phaseolus vulgaris*), mung bean (*Vigna radiata*), cowpea *(Vigna unguiculata*), adzuki bean (*Vigna angularis*), fava bean (*Vicia faba*)*,* chickpea (*Cicer arietinum*), peanut *(Arachis hypogaea*)*,* lentils (*Lens culinaris, Lens esculenta*), lupins (*Lupinus* spp.), white lupin (*Lupinus albus*), mesquite (*Prosopis* spp.), carob *(Ceratonia siliqua*), tamarind (*Tamarindus indica*), alfalfa (*Medicago sativa*), barrel medic (*Medicago truncatula*), birdsfood trefoil (*Lotus japonicus*), licorice (*Glycyrrhiza glabra*), and clover (*Trifolium* spp.). In specific embodiments, a plant or plant part to be transformed according to the methods of the present disclosure is *Glycine max* or a part of *Glycine max.* Additionally, a plant or plant part to be transformed according to the methods present disclosure can be a crop plant or part of a crop plant, including legumes. Examples of crop plants include, but are not limited to, corn (*Zea mays*), *Brassica* sp. (e.g., *B. napus, B. rapa, B. juncea),* particularly those *Brassica* species useful as sources of seed oil, yellow pea *(Pisum sativum),* alfalfa (*Medicago sativa*), rice (*Oryza sativa*), rye (*Secale cereale*), sorghum (*Sorghum bicolor, Sorghum vulgare*), camelina (*Camelina sativa*), millet (e.g., pearl millet (*Pennisetum glaucum*)*,* proso millet (*Panicum miliaceum*), foxtail millet (*Setaria italica*), finger millet (*Eleusine coracana*))*,* sunflower (*Helianthus annuus*), quinoa (*Chenopodium quinoa*), chicory (*Cichorium intybus*), lettuce (*Lactuca sativa*), safflower (*Carthamus tinctorius*), wheat (*Triticum aestivum*), soybean (*Glycine max*), tobacco (*Nicotiana* spp., e.g., *Nicotiana tabacum, Nicotiana sylvestris*), potato (*Solanum tuberosum*), tomato (*Solanum lycopersicum*), peanuts (*Arachis hypogaea*)*,* cotton (*Gossypium barbadense, Gossypium hirsutum*), sweet potato (*Ipomoea batatus*), cassava (*Manihot esculenta*), coffee (*Coffea* spp.), coconut (*Cocos nucifera*)*,* pineapple (*Ananas comosus*), citrus trees (*Citrus* spp.), cocoa (*Theobroma cacao*), tea (*Camellia sinensis*), banana (*Musa* spp.), avocado (*Persea americana*), fig (*Ficus casica*)*,* guava (*Psidium guajava*), mango (*Mangifera indica*), grapes (*Vitis vinifera, Vitis riparia*)*,* olive (*Olea europaea*), papaya (*Carica papaya*), cashew (*Anacardium occidentale*), macadamia (*Macadamia integrifolia*), almond (*Prunus amygdalus*), sugar beets (*Beta vulgaris*), sugarcane (*Saccharum* spp.), oil palm (*Elaeis guineensis*), poplar (*Populus* spp.), eucalyptus (*Eucalyptus* spp.), oats (*Avena sativa*), barley (*Hordeum vulgare*), vegetables, ornamentals, and conifers. Additionally, a plant or plant part of the present disclosure can be an oilseed plant (e.g., canola (*Brassica napus*), cotton (*Gossypium* sp.), camelina (*Camelina sativa*) and sunflower (*Helianthus* sp.)), or other species including wheat (*Triticum* sp., such as *Triticum aestivum* L. ssp. *aestivum* (common or bread wheat), other subspecies of *Triticum aestivum, Triticum turgidum* L. ssp. durum (durum wheat, also known as macaroni or hard wheat), *Triticum monococcum* L. ssp. *monococcum* (cultivated einkorn or small spelt), *Triticum timopheevi* ssp. *timopheevi, Triticum turgigum* L. ssp. dicoccon (cultivated emmer), and other subspecies of *Triticum turgidum* (Feldman)), barley (*Hordeum vulgare*), maize (*Zea mays*), oats (*Avena sativa*), or hemp (*Cannabis sativa*). Additionally, a plant or plant part of the present disclosure can be a forage plant or part of a forage plant. Examples of forage plants include legumes and crop plants described herein as well as grass forages including *Agrostis* spp., *Lolium* spp., *Festuca* spp., *Poa* spp., and *Bromus* spp.

The embodiments disclosed herein are not limited to certain methods of introducing nucleic acids into a plant and are not limited to certain forms or structures that the introduced nucleic acids take. Any method of transforming a cell of a plant described herein with mutations, polynucleotides, or polypeptides are also incorporated into the teachings of this innovation. For example, one of ordinary skill in the art will realize that the use of particle bombardment (e.g. using a gene-gun), *Agrobacterium* infection and/or infection by other bacterial species capable of transferring DNA into plants (e.g., *Ochrobactrum* sp., *Ensifer* sp., *Rhizobium* sp.), viral infection, and other techniques can be used to deliver mutations, polynucleotides, or polypeptides into a plant, plant part, or plant cell described herein.

The present disclosure provides plants and plant parts transformed according to the methods of the present disclosure. Transformed plant parts of the invention include plant cells, plant protoplasts, plant cell tissue cultures from which plants can be regenerated, plant calli, plant clumps, and plant cells that are intact in plants or parts of plants such as embryos, pollen, ovules, seeds, grains, leaves, flowers, branches, fruit, kernels, ears, cobs, husks, stalks, roots, root tips, anthers, and the like. Progeny, variants, and mutants of the regenerated plants are also included within the scope of the disclosure, provided that these parts comprise the introduced mutations, polynucleotides, or polypeptides.

### G. Breeding of Plants

Also disclosed herein are methods for breeding a plant, such as a plant which contains (i) a mutation that decreases the GAS and/or RS activity, e.g., one or more insertions, substitutions, or deletions in at least one native *GAS* and/or *RS* gene or homolog or in a regulatory region of such *GAS* and/or *RS* gene or homolog, (ii) editing reagents, e.g., a polynucleotide encoding a guide RNA specific to at least one *GAS* and/or *RS* gene or homolog or in a regulatory region of such *GAS* and/or *RS* gene or homolog, and/or (iii) a polynucleotide comprising a mutated *GAS* and/or *RS* gene or a *GAS* and/or *RS* gene with a mutated regulatory region. A plant containing the one or more mutations or the polynucleotide of the present disclosure may be regenerated from a plant cell or plant part, wherein the genome of the plant cell or plant part is genetically-modified to contain the one or more mutations or the polynucleotide of the present disclosure. Using conventional breeding techniques or self-pollination, one or more seeds may be produced from the plant that contains the one or more mutations or the polynucleotide of the present disclosure. Such a seed, and the resulting progeny plant grown from such a seed, may contain the one or more mutations or the polynucleotide of the present disclosure, and therefore may be transgenic. Progeny plants are plants having a genetic modification to contain the one or more mutations or the polynucleotide of the present disclosure, which descended from the original plant having modification to contain the one or more mutations or the polynucleotide of the present disclosure. Seeds produced using such a plant of the invention can be harvested and used to grow generations of plants having genetic modification to contain the one or more mutations or the polynucleotide of the present disclosure, e.g., progeny plants, of the invention, comprising the polynucleotide and optionally expressing a gene of agronomic interest (e.g., herbicide resistance gene).

Descriptions of breeding methods that are commonly used for different crops can be found in one of several reference books, see, e.g., Allard, Principles of Plant Breeding, John Wiley & Sons, NY, U. of CA, Davis, Calif., 50-98 (1960); Simmonds, Principles of Crop Improvement, Longman, Inc., NY, 369-399 (1979); Sneep and Hendriksen, Plant breeding Perspectives, Wageningen (ed), Center for Agricultural Publishing and Documentation (1979); Fehr, Soybeans: Improvement, Production and Uses, 2nd Edition, Monograph, 16:249 (1987); Fehr, Principles of Variety Development, Theory and Technique, (Vol. 1) and Crop Species Soybean (Vol. 2), Iowa State Univ., Macmillan Pub. Co., NY, 360-376 (1987).

Methods disclosed herein include conferring desired traits (e.g., increased sucrose content) to plants, for example, by mutating sequences of a plant, introducing nucleic acids into plants, using plant breeding techniques and various crossing schemes, etc. These methods are not limited as to certain mechanisms of how the plant exhibits and/or expresses the desired trait. In certain nonlimiting embodiments, the trait is conferred to the plant by introducing a nucleic acid sequence (e.g. using plant transformation methods) that encodes production of a certain protein by the plant. In certain embodiments, the desired trait is conferred to a plant by causing a null mutation in the plant's genome (e.g. when the desired trait is reduced expression or no expression of a certain trait). In certain embodiments, the desired trait is conferred to a plant by causing a null mutation into at least one but not all alleles of the *GAS* and/or *RS* gene(s) or its regulatory region, e.g., by introducing heterozygous mutation into a *GAS* and/or *RS* gene or its regulatory region. In certain embodiments, the desired trait is conferred to a plant by crossing two plants to create offspring that express the desired trait. It is expected that users of these teachings will employ a broad range of techniques and mechanisms known to bring about the expression of a desired trait in a plant. Thus, as used herein, conferring a desired trait to a plant is meant to include any process that causes a plant to exhibit a desired trait, regardless of the specific techniques employed.

In certain embodiments, a user can combine the teachings herein with high-density molecular marker profiles spanning substantially the entire genome of a plant to estimate the value of selecting certain candidates in a breeding program in a process commonly known as genome selection.

### V. Nucleic Acid Molecules, Constructs, and Cells Comprising Mutated GAS and/or RS gene or Mutated Regulatory Region of GAS and/or RS gene

### A. Nucleic acid molecules

Nucleic acid molecules are provided herein comprising a mutated genomic sequence that alters (e.g., decreases) GAS and/or RS activity in a plant or plant part. The nucleic acid molecule can comprise any nucleic acid sequence that alters (e.g., decreases) GAS and/or RS activity in a plant or plant part including those described herein, e.g., an altered (e.g., mutated, alternatively spliced) nucleic acid sequence of a *GAS* and/or *RS* gene, a regulatory region of the *GAS* and/or *RS* gene, or a *GAS* and/or *RS* gene transcript, encoding an altered (e.g., mutated, alternatively spliced, truncated) GAS and/or RS relative to a corresponding native *GAS* and/or *RS* gene or GAS and/or RS. Such nucleic acid molecules may be present in, or obtained from, a plant cell, plant part, or plant of the present disclosure, or may be obtained by the methods described herein, e.g., by introducing one or more mutations into at least one *GAS* and/or *RS* gene or a regulatory region of the *GAS* and/or *RS* gene and/or by introducing editing reagents targeting a site of interest in at least one *GAS* and/or *RS* gene or a regulatory region of the *GAS* and/or *RS* gene in a plant or plant part. The nucleic acid molecule described herein can encode an altered (e.g., mutated, truncated, alternatively spliced) GAS and/or RS that can comprise a different amino acid sequence from a native GAS and/or RS (e.g., without mutations). The nucleic acid molecule described herein can encode a GAS and/or RS with reduced function or loss-of-function, as compared to a native GAS and/or RS (e.g., without mutations). The mutated sequence, e.g., altered nucleic acid sequence of the *GAS* and/or *RS* gene and/or the regulatory region of the *GAS* and/or *RS* gene can result in reduced expression levels of the *GAS* and/or *RS* gene or GAS and/or RS (e.g., full-length GAS and/or RS, functional GAS and/or RS), as compared to a native *GAS* and/or *RS* gene and/or a regulatory region of a native *GAS* and/or *RS* gene, e.g., without mutations.

The nucleic acid molecule provided herein can comprise one or more (e.g., about 1, 2, 3, 4, 5, 6, 7, 8, 9, 10, 11, 12, 13, 14, 15, 16, 17, 18, 19, 20, or more) insertions, substitutions, and/or deletions in a *GAS* and/or *RS* gene or homolog and/or a regulatory region (e.g., promoter, 5'UTR) of the *GAS* and/or *RS* gene or homolog compared to a corresponding native a *GAS* and/or *RS* gene or homolog and/or a regulatory region of the native *GAS* and/or *RS* gene or homolog. The nucleic acid molecule may comprise an in-frame mutation (e.g., a missense mutation), a frameshift (out-of-frame) mutation, or a nonsense mutation of the *GAS* and/or *RS* gene or homolog.

The mutation in the nucleic acid molecule provided herein can be located in *Pisum sativum GAS* and/or *RS* genes (e.g., *Pisat.03G015560, Pisat.06G066360, Pisat.05G054130,* and *Pisat.01G021000*), and/or a regulatory region of such one or more *Pisum sativum GAS* and/or *RS* genes. In some embodiments, mutation in the nucleic acid molecule provided herein is located in a *GAS* and/or *RS* gene or its regulatory region, and (i) the *GAS* and/or *RS* gene (before the mutation is located) comprises a nucleic acid sequence having at least 80% (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) sequence identity to a nucleic acid sequence of any one of SEQ ID NOs: 1-8, 25, 31, 37-40, 42, and 50, wherein the nucleic acid sequence encodes a polypeptide that retains GAS and/or RS activity; (ii) the *GAS* and/or *RS* gene (before the mutation is located) comprises the nucleic acid sequence of any one of SEQ ID NOs: 1-8, 25, 31, 37-40, 42, and 50; (iii) the *GAS* and/or *RS* gene (before the mutation is located) encodes a polypeptide comprising an amino acid sequence having at least 80% (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) sequence identity to an amino acid sequence of any one of any one of SEQ ID NOs: 9-12, 41, 43, and 51, wherein the polypeptide retains GAS and/or RS activity; and/or (iv) the *GAS* and/or *RS* gene (before the mutation is located) encodes a polypeptide comprising an amino acid sequence of any one of any one of SEQ ID NOs: 9-12, 41, 43, and 51. In specific embodiments, the mutation that decreases the GAS and/or RS activity is located in one or two alleles of one or more (e.g., one, more than one but not all, or all) copies of *Pisum sativum GAS* or *RS* gene (e.g., *Pisat.03G015560, Pisat.06G066360, Pisat.05G054130,* and *Pisat.01G021000*), and/or a regulatory region thereof.

In some specific embodiments, the nucleic acid molecule provided herein comprises a nucleic acid sequence of a mutated *GAS* and/or *RS* gene or coding sequence thereof, wherein said nucleic acid sequence comprises any one of SEQ ID NOs: 1-8, 25, 31, 37-40, 42, and 50, comprising one or more insertions, substitutions, or deletions therein. The mutated *GAS* and/or *RS* gene or coding sequence thereof can encode a GAS and/or RS with reduced function or loss of function, or can produce reduced expression of GAS and/or RS as compared to a control (e.g., wild-type) *GAS* and/or *RS* gene or coding sequence thereof. The nucleic acid molecule provided herein can comprise a mutation that is at least partially located in a nucleic acid region of a *Pisum sativum GAS* gene comprising the nucleic acid sequence of SEQ ID NO: 1, 2, 5, 6, 25, 37, or 38, and/or in a nucleic acid region of a *Pisum sativum RS* gene comprising the nucleic acid sequence of SEQ ID NO: 3, 4, 7, 8, 31, 39, or 40, and/or in a nucleic acid region of a *Glycine max GAS* gene comprising the nucleic acid sequence of SEQ ID NO: 42 or 50. For example, the nucleic acid molecule provided herein can comprise a mutated *Pisum sativum GAS* gene sequence (i) comprising a G to A substitution of nucleotide 128 of SEQ ID NO: 5 or 25, or comprising a nucleic acid sequence of SEQ ID NO: 15 or 26; (ii) comprising a deletion of nucleotides 115-119 of SEQ ID NO: 25, or comprising a nucleic acid sequence of SEQ ID NO: 27; (iii) comprising a deletion of nucleotides 111-117 of SEQ ID NO: 25, or comprising a nucleic acid sequence of SEQ ID NO: 28; (iv) encoding a mutated *Ps*GAS protein comprising a G to D substitution of amino acid 43 of SEQ ID NO: 9, or comprising an amino acid sequence of SEQ ID NO: 17; (v) encoding a mutated *Ps*GAS protein comprising an amino acid sequence of SEQ ID NO: 29; and/or (vi) encoding a mutated *Pisum sativum* GAS protein comprising an amino acid sequence of SEQ ID NO: 30. The nucleic acid molecule provided herein can comprise a mutated *Glycine max GAS* gene sequence (i) comprising a deletion of nucleotides 197-209 of SEQ ID NO: 42, or comprising a nucleic acid sequence of SEQ ID NO: 44; (ii) a mutated *Glycine max GAS* gene comprising a deletion of nucleotides 199-209 of SEQ ID NO: 42, or comprising a nucleic acid sequence of SEQ ID NO: 45; (iii) a mutated *Glycine max GAS* gene comprising a deletion of nucleotide 204 of SEQ ID NO: 42, or comprising a nucleic acid sequence of SEQ ID NO: 46; and/or (iv) encoding a mutated *Gm*GAS protein comprising an amino acid sequence of any one of SEQ ID NOs: 47-49. The nucleic acid molecule provided herein can comprise a mutated *Pisum sativum RS* gene sequence (i) comprising a G to A substitution of nucleotide 1044 of SEQ ID NO: 7 or 31, or comprising a nucleic acid sequence of SEQ ID NO: 16 or 32; (ii) comprising a deletion of nucleotides 1246-1253 of SEQ ID NO: 31, or comprising a nucleic acid sequence of SEQ ID NO: 33; (iii) comprising a deletion of nucleotides 1248-1254 of SEQ ID NO: 31, or comprising a nucleic acid sequence of SEQ ID NO: 34; (iv) encoding a truncated *Ps*RS protein comprising a deletion of amino acids 348-798 of SEQ ID NO: 11, or comprising an amino acid sequence of SEQ ID NO: 18; (v) encoding a mutated *Ps*RS protein comprising an amino acid sequence of SEQ ID NO: 35; and/or (vi) encoding a mutated *Ps*RS protein comprising an amino acid sequence of SEQ ID NO: 36. The nucleic acid molecule provided herein can also comprise a mutated *PsGAS* gene sequence comprising the nucleic acid sequence of SEQ ID NO: 1, 2, 5, 6, 25, 37, or 38 with a mutation at or near the binding site of a *PsGAS* guide RNA (e.g., SEQ ID NO: 13), and/or a mutated *PsRS* gene sequence comprising the nucleic acid sequence of SEQ ID NO: 3, 4, 7, 8, 31, 39, or 40 with a mutation at or near the binding site of a *PsRS* guide RNA (SEQ ID NO: 14); and/or a mutation in the *GmGAS* gene comprising the nucleic acid sequence of SEQ ID NO: 42 or 50 at or near the binding site of a *GmGAS* guide RNA (SEQ ID NO: 52).

In some embodiments, the nucleic acid molecules described herein do not comprise a regulatory region (e.g., a promoter region) of a *GAS* and/or *RS* gene or homolog. Alternatively, the nucleic acid molecules can comprise the regulatory region (e.g., promoter region) of the *GAS* and/or *RS* gene or homolog. The regulatory region (e.g., promoter regions) in the nucleic acid molecule can comprise one or more (e.g., about 1, 2, 3, 4, 5, 6, 7, 8, 9, 10, 11, 12, 13, 14, 15, 16, 17, 18, 19, 20, or more) insertions, substitutions, and/or deletions. The one or more insertions, substitutions, and/or deletions in the regulatory region of the *GAS* and/or *RS* gene or homolog can alter expression level or manner of the *GAS* and/or *RS* gene or homolog. For example, the one or more insertions, substitutions, and/or deletions in the promoter region of the *GAS* and/or *RS* gene or homolog can alter the transcription initiation activity of the promoter. The modified promoter can alter (e.g., reduce) transcription of the operably linked nucleic acid molecule, initiate transcription in a developmentally-regulated manner, initiate transcription in a cell-specific, cell-preferred, tissue-specific, or tissue-preferred manner, or initiate transcription in an inducible manner. The modified promoter can comprise a deletion, a substitution, or an insertion, e.g., introduction of a heterologous promoter sequence, a cis-acting factor, a motif or a partial sequence from any promoter, including those described elsewhere in the present disclosure, to confer an altered (e.g., reduced) transcription initiation function to the promoter region of the *GAS* and/or *RS* gene according to the present disclosure.

In some specific embodiments, the nucleic acid molecule comprises a nucleic acid sequence of a mutated promoter of a *GAS* and/or *RS* gene comprising a nucleic acid sequence of any one of SEQ ID NOs: 1-8, 25, 31, 37-40, 42, and 50, wherein the mutated promoter comprises one or more insertions, substitutions, or deletions in the nucleic acid sequence of a native promoter of the *GAS* and/or *RS* gene. The mutated promoter can produce reduced level or activity of a GAS and/or RS protein gene or polypeptide.

The nucleic acid molecule described herein can comprise one or more insertions, substitutions, and/or deletions in the regulatory region (e.g., promoter region) of the *GAS* and/or *RS* gene as well as in the exon/intron region of the *GAS* and/or *RS* gene.

### B. DNA constructs, vectors, and cells

The nucleic acid molecules encoding molecules of interest (e.g., comprising a mutated *GAS* or *RS* gene) of the present invention can be assembled within a DNA construct with an operably-linked promoter. When transiently or stably transformed with such DNA construct, a plant, plant part, or plant cell can express or accumulate polynucleotides comprising an altered (e.g., mutated, alternatively spliced) sequence of a *GAS* and/or *RS* gene or a *GAS* and/or *RS* gene transcript, or a GAS and/or RS encoded by the polynucleotides. For example, the nucleic acid molecules described herein can be provided in expression cassettes or expression constructs along with a promoter sequence of interest, typically a heterologous promoter sequence, for expression in the plant of interest. By "heterologous promoter sequence" is intended a sequence that is not naturally operably linked with the nucleic acid molecule of interest. For instance, a 2x35s promoter, a native promoter, or a promoter (native or heterologous) comprising an exogenous or synthetic motif sequence may be operably linked to the nucleic acid sequences comprising an altered (e.g., mutated, alternatively spliced) sequence of a *GAS* and/or *RS* gene or a *GAS* and/or *RS* gene transcript. The GAS and/or RS-encoding nucleic acid sequences or the promoter sequence may each be homologous, native, heterologous, or foreign to the plant host. It is recognized that the heterologous promoter may also drive expression of its homologous or native nucleic acid sequence. In this case, the transformed plant will have a change in phenotype.

Accordingly, the present disclosure provides DNA constructs comprising, in operable linkage, a promoter that is functional in a plant cell, and a nucleic acid molecule of the present disclosure, e.g., comprising an altered nucleic acid sequence of a *GAS* and/or *RS* gene or coding sequence thereof relative to a corresponding native nucleic acid sequence, e.g., comprising one or more insertions, substitutions, or deletions in a nucleic acid sequence of any one of SEQ ID NOs: 1-8, 25, 31, 37-40, 42, and 50. Also provided herein are DNA constructs comprising, in operable linkage, a regulatory region of a *GAS* and/or *RS* gene that can be native (without mutation) or mutated (e.g., comprising one or more insertions, substitutions, or deletions in a promoter sequence of a *GAS* and/or *RS* gene comprising a nucleic acid sequence of any one of SEQ ID NOs: 1-8, 25, 31, 37-40, 42, and 50), and a polynucleotide of interest [e.g., a *GAS* and/or *RS* gene or a reporter gene, e.g., GFP, luciferase, HA tag).

When the DNA construct or nucleic acid molecule provided herein is introduced in a plant, plant part, or plant cell, GAS and/or RS activity can be reduced, expression levels of the *GAS* and/or *RS* gene or homolog can be decreased, GAS and/or RS level or activity can be decreased, and/or protein content is increased in the plant, plant part, or plant cell as compared to a control plant, plant part, or plant cell, e.g., a plant, plant part, or plant cell to which the construct or the nucleic acid molecule comprising the corresponding wild-type *GAS* and/or *RS* gene or its regulatory region is introduced. The DNA construct can further comprise, in operable linkage, a reporter / selectable marker construct (e.g., GFP, a HA tag). Any reporter or selectable marker can be used, including the reporters and selectable markers described elsewhere in the present disclosure.

Provided herein are vectors comprising the nucleic acid molecule and/or the DNA construct of the present disclosure comprising an altered nucleic acid sequence of the *GAS* and/or *RS* gene, the regulatory region of the *GAS* and/or *RS* gene and/or the *GAS* and/or *RS* gene transcript. Any vectors can be used, including the vectors described elsewhere in the present disclosure.

Also provided herein are cells comprising the nucleic acid molecule, the DNA construct, and/or the vector of the present disclosure comprising an altered nucleic acid sequence of the *GAS* and/or *RS* gene, the regulatory region of the *GAS* and/or *RS* gene, and/or the *GAS* and/or *RS* gene transcript. The cell can be a plant cell, a bacterial cell, and a fungal cell. The cell can be a bacterium, e.g., an *Agrobacterium tumefaciens,* containing the nucleic acid molecule, the DNA construct, or the vector of the present disclosure. The cell can be a plant cell. The cells of the present disclosure may be grown, or have been grown, in a cell culture.

Also provided herein are methods for generating a plant, plant part (e.g., seed), plant cell, or a population of plants or plant parts (e.g., seeds) comprising decreased GAS and/or RS activity and/or increased amino acid content, by introducing into the plant, plant part, or plant cell the nucleic acid molecule, the DNA construct, the vector, or the cell of the present disclosure. In some embodiments, the nucleic acid molecule, DNA construct, vector, or cell is introduced into the plant by stable transformation. In other embodiments, the nucleic acid molecule, DNA construct, vector, or cell is introduced into the plant by transient transformation. The present disclosure further provides plants, plant parts (seed, juice, pulp, fruit, flowers, nectar, embryos, pollen, ovules, leaves, stems, branches, bark, kernels, ears, cobs, husks, stalks, roots, root tips, anthers, etc.), or plant products (e.g., seed compositions, plant protein, plant protein compositions, plant extract, plant concentrate, plant powder, plant biomass, and food and beverage products) generated by the methods described herein.

It will be readily apparent to those skilled in the art that other suitable modifications and adaptations of the methods of the invention described herein are obvious and may be made using suitable equivalents without departing from the scope of the invention or the embodiments disclosed herein. Having now described the invention in detail, the same will be more clearly understood by reference to the following examples, which are included for purposes of illustration only and are not intended to be limiting. Unless otherwise noted, all parts and percentages are by dry weight.

### EXAMPLES

### EXAMPLE 1: Expression of GAS and RS genes in wild-type pea tissues

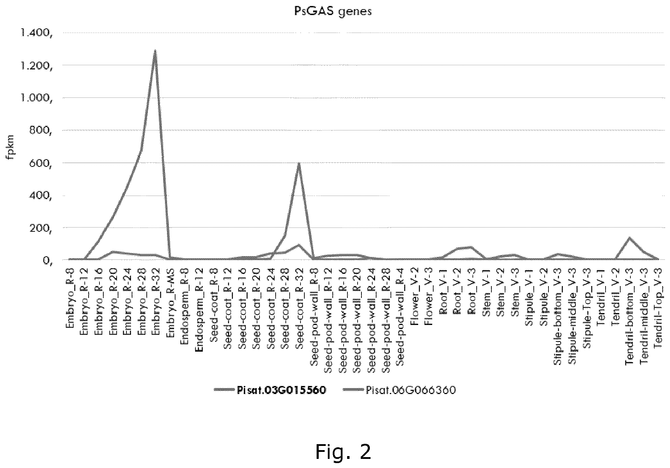

Transcript expression levels of pea *GAS gene* copies (*Pisat. 03G015560* and *Pisat. 06G066360)* in a pea expression database were studied. As shown in FIG. 2, *GAS* gene transcripts were expressed across various tissues of pea, including embryo, seed coat, and tendril.

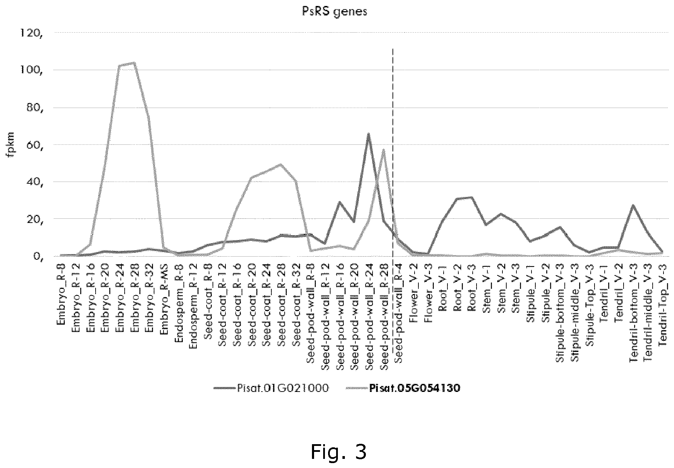

Transcript expression levels of pea *RS* gene copies (*Pisat.05G054130* and *Pisat.01G021000*) in the pea expression database were studied. As shown in FIG. 3, *RS* gene transcripts were expressed across various tissues of pea, including embryo, seed coat, seed pod, root, stem, stipule, and tendril.

### EXAMPLE 2: Chemical mutagenesis in the GAS and/or RS gene

Pea seeds were treated with 0.3% ethyl methanesulfonate (EMS) and 0.1 mM N-ethyl-N-nitrosourea (ENU) for 16 hours, followed by five washes in pure water. The resulting M1 seeds were planted and grown to maturity. DNA samples from six pooled M2 plants were prepared and sequenced. M2 plants having a mutation in the *GAS* and/or *RS* gene were selected based on the sequencing results.

M3 seeds were harvested from M2 plants identified as having a mutation in the *GAS* and/or RS gene. Progeny plants and seeds were grown from M3 seeds. Expression levels of GAS and/or RS, as well as content of protein, RFOs (e.g., raffinose, stachyose), and/or sucrose in seeds of mutant plants are analyzed, as described in Example 4.

### EXAMPLE 3: Gene editing in the GAS and/or RS gene

Guide RNAs targeting a *PsGAS* and/or *PsRS* gene were designed according to standard methods of the art (Zetsche et al., Cell, Volume 163, Issue 3, Pages 759-771, 2015; Cui et al., Interdisciplinary Sciences: Computational Life Sciences, volume 10, pages 455-465, 2018). Optimized gRNA design to maximize activity and minimize off-target effects of CRISPR-Cas9 and CRISPR-Cas12a have been extensively characterized (Nat Biotechnol 2016;34:184-191, doi: 10.1038/nbt.3437). The CRISPR-Cas12a system described herein can be employed for targeting PAM sites such as TTN, TTV, TTTV, NTTV, TATV, TATG, TATA, YTTN, GTTA, and GTTC, utilizing corresponding gRNAs. Example sequences encoding a targeting sequence of a *PsGAS* guide RNA and a *PsRS* guide RNA are set forth as SEQ ID NOs: 13 and 14, respectively.

Pea protoplasts are transformed with constructs comprising guide RNAs targeting a genomic site in the *GAS* or *RS* gene and a nuclease using *Agrobacterium* transformation. Amplicons are produced near the target sites, and are sequenced to detect mutations. A mutated read is recorded for any sequence with more than two reads containing a deletion at the predicted cleavage site. Editing efficiency is calculated based on the percentage of mutated reads to total aligned reads using next generation sequencing (NGS).

A number of mutants having mutations in the *GAS* or *RS* gene or its regulatory region (e.g., promoter, 5'UTR) are generated by introducing into protoplasts the gene editing system provided herein, including one or more guide RNAs (e.g., having a target sequence set forth in SEQ ID NO: 13, 14, or 52). In specific experiments, two or more guide RNAs are used.

The mutants having mutation in the *GAS* and/or *RS* gene (e.g., in the coding region) are screened for editing efficiency and expression levels. Expression cassettes comprising the (mutated or wild-type) *GAS* or *RS* gene, operably linked to a functional promoter, are generated. The cassettes with mutations, as well as no mutations (wild-type) are transiently expressed in tobacco leaves. Levels of the *GAS* and/or *RS* gene are measured by standard methods for measuring mRNA levels of a gene, including quantitative RT-PCR, northern blot, and serial analysis of gene expression (SAGE). Levels of the GAS and/or RS encoded by the *GAS* and/or *RS* gene are also measured by standard methods for measuring protein levels, including western blot analysis, ELISA, or dot blot analysis of a plant sample using an antibody directed to the GAS and/or RS.

The mutants having mutation in the regulatory region of the *GAS* and/or *RS* gene are screened for editing efficiency and effects on expression levels of a downstream gene. Expression cassettes comprising the (mutated or wild-type) *GAS* or *RS* gene, operably linked to a polynucleotide encoding GFP, are generated. Expression cassettes comprising the (mutated or wild-type) promoter of the *GAS* or *RS* gene, operably linked to a polynucleotide encoding a reporter (e.g., GFP, luciferase), are generated. The cassettes with mutations, as well as no mutations (wild-type) are transiently expressed in tobacco leaves, and GFP protein levels in infiltrated leaves are quantified as a readout for expression levels of genes operably linked to the mutated or wild-type) promoter or 5'UTR of the *GAS* or *RS* gene.

Embryonic axes of mature seeds of yellow pea varieties are stably transformed with constructs comprising one, two, or multiple guide RNAs targeting *GAS* or *RS* gene and a nuclease using *Agrobacterium* transformation. Embryonic axes are infected using an *Agrobacterium tumefaciens* strain carrying a construct encoding CP4 selectable marker cassette (e.g., conferring resistance to glyphosate), nuclease (e.g., a RNA guided nuclease), and gRNA cassettes to drive mutations at the *GAS* or *RS* gene locus. Transformed plants are identified by selective marker (e.g., resistance to glyphosate). Amplicons are produced of the genomic regions near the targeted sites and sequenced to evaluate the presence of the mutation using a pair of primers to detect mutations introduced. Transgenic events are recorded, and the T0 plants were assigned unique plant names and are subjected to molecular characterization and propagation. T0 plants are self-pollinated and T1 plants are generated. Crosses are made to generate lines that are homozygous or heterozygous for the target mutation and lack the editing reagents. Expression levels of GAS and/or RS, as well as content of protein, RFOs (e.g., raffinose, stachyose), and/or sucrose in seeds of transformed plants are analyzed, as described in Example 4.

### EXAMPLE 4: Screening of plants with mutations

Transformed plants (e.g., with gene editing or chemical mutagenesis) are screened using a variety of molecular tools to identify plants and genotypes that will result in the expected phenotype. For example, expression levels of *GAS* and/or *RS* genes and levels and activities of GAS and/or RS are measured in mutant plants (e.g., having a homozygous or heterozygous mutation in the *GAS* and/or *RS* gene promoter). Expression levels of the *GAS* and/or *RS* genes are measured by any standard methods for measuring mRNA levels of a gene, including quantitative RT-PCR, northern blot, and serial analysis of gene expression (SAGE). Expression levels of GAS and/or RSs (e.g., full-length GAS and/or RS) are measured by any standard methods for measuring protein levels, including western blot analysis, ELISA, or dot blot analysis of a protein sample obtained from the plant using an antibody directed to the GAS and/or RS.

GAS and/or RS activity in the plant sample is assessed by one or more standard methods for measuring enzymatic activity in a plant sample. For example, GAS activity can be determined by contacting UDP-galactose and myo-inositol with a sample obtained from a plant, plant part, or plant product and measuring the level of galactinol, e.g., by HPLC with refractive index detection or any other standard methods. RS activity can be determined by contacting galactinol with a sample obtained from a plant, plant part, or plant product and measuring the level of raffinose, e.g., by HPLC with refractive index detection or any other standard methods.

Amount of raffinose, stachyose, and sucrose in a sample obtained from a plant, plant part, or plant product is measured by any standard method, such as by spectroscopy (near infrared spectroscopy), refractometry, high performance liquid chromatography (HPLC), high performance liquid chromatography with refractive index detection (HPLC-RI), ion chromatography, size exclusion chromatography with refractive index detection (HPSEC-RI), liquid chromatography with mass spectrometry (LC-MS), derivatization and gas chromatography (GC), and ion chromatography with pulsed amperometric detection (HPAEC-PAD).

Amount of protein in a sample obtained from a plant, plant part, or plant product (e.g., seed protein content) is measured by any standard method, for example by protein extraction and quantitation (e.g., BCA protein assay, Lowry protein assay, Bradford protein assay), spectroscopy, near-infrared (NIR) spectroscopy (e.g., analyzing 700 - 2500 nm), nuclear magnetic resonance spectrometry (NMR), and the Dumas method. The Dumas method measures protein content by combusting samples at a high temperature in the presence of high-purity oxygen, analyzing the gas from combustion for nitrogen content using a thermal conductivity detector, and calculating the amount of protein present in the sample using a conversion factor. The industry standard conversion factor for pea is 6.25.

The plant with mutation and desirable phenotype is selected, e.g., having reduced activity or level of GAS and/or RS, reduced expression levels of the *GAS* and/or *RS* genes, decreased RFO (e.g., raffinose, stachyose) content, increased sucrose content, and/or increased protein content as compared to a control plant (e.g., without the mutation), when grown under the same environmental conditions.

### Example 5: Decreased RFO and Increased Sucrose Content in Plants with GAS and/or RS Mutations

Seed raffinose, stachyose, and sucrose content was measured by HPLC.

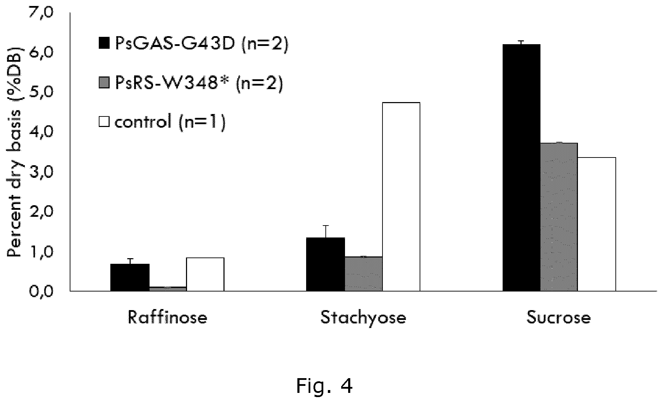

As shown in FIG. 4 and Table 1, seeds of M3 pea plants having a *GAS* or *RS* mutation had decreased raffinose content, decreased stachyose content, and increased sucrose content relative to a control. Specifically, a G128A substitution (by EMS mutagenesis) in the *PsGAS* gene, resulting in a G43D missense mutation in the GAS protein, decreased raffinose content from about 0.8% (control) to about 0.7%, decreased stachyose content from about 4.7% (control) to about 1.3%, and increased sucrose content from about 3.3% (control) to about 6.1% in seeds. Further, seeds of plants having a G1044A substitution (by EMS mutagenesis) in the *PsRS* gene, resulting in a W348* mutation in the RS protein, decreased raffinose content from about 0.8% (control) to about 0.1%, decreased stachyose content from about 4.7% (control) to about 0.8%, and increased sucrose content from about 3.3% (control) to about 3.7% in seeds.

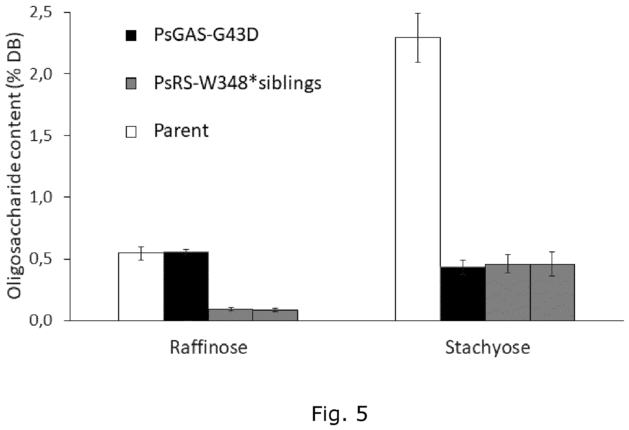

As shown in FIG. 5 and Table 2, decrease in raffinose and stachyose content was consistent through generations and increased sample size. M5 seeds of plants having a G43D mutation in the GAS protein showed raffinose and stachyose content of about 0.55% and 0.43%, respectively, and M5 seeds of plants having a W348* mutation in the RS protein showed raffinose and stachyose content of about 0.09% and about 0.46%, respectively, showing a decrease relative to a parent without the mutation having raffinose and stachyose content of about 0.54% and about 2.30%, respectively.

As shown in FIGs. 6A-6D, T2 seeds of pea plants having a *GAS* or *RS* mutation had decreased raffinose content, decreased stachyose content, and/or increased sucrose content relative to a control. Specifically, the deletion in the *PsRS* gene (resulting in SEQ ID NO: 33 or 34) decreased raffinose content from about 0.5% (control) to about 0.15% in seeds (FIG. 6A). The deletion in the *PsRS* gene (resulting in SEQ ID NO: 33 or 34) or *PsGAS* gene (resulting in SEQ ID NO: 27 or 28) decreased stachyose content from about 1.8% (control) to about 0.7-0.9% in seeds (FIG. 6B). The deletion in the *PsRS* gene (resulting in SEQ ID NO: 33) and the deletion in the *PsGAS* gene (resulting in SEQ ID NO: 27) decreased content of raffinose plus stachyose from about 2.25% (control) to about 0.9% and about 1.4%, respectively, in seeds (FIG. 6C). The deletion in the *PsRS* gene (resulting in SEQ ID NO: 33 or 34) and the deletion in the *PsGAS* gene (resulting in SEQ ID NO: 27 or 28) increased sucrose content from about 2.1% (control) to about 3.0% (*PsRS* mutants) and about 4.7% (*PsGAS* mutants), respectively, in seeds (FIG. 6D). In FIGs. 6A-6D, each bar represents average ± SD of measurement of seeds from 12 plants.

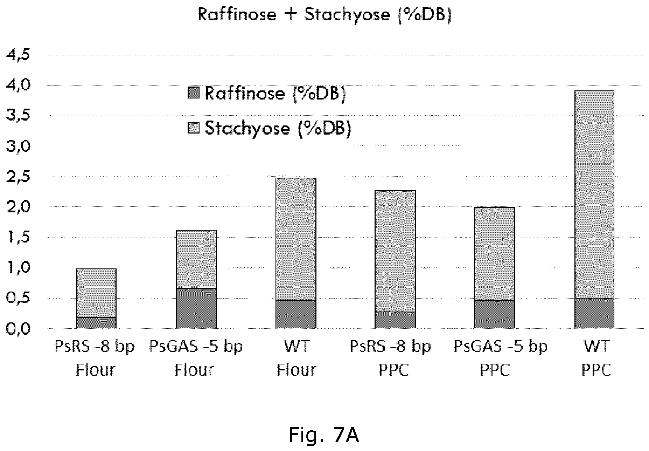

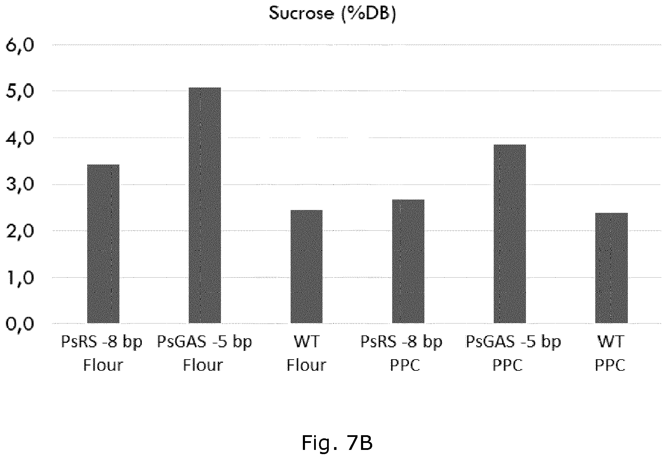

As shown in FIGs. 7A and 7B, flour and pea protein concentrate (PPC) produced from pea T2 seeds having a *GAS* or *RS* mutation had decreased raffinose content, decreased stachyose content, and/or increased sucrose content relative to a control. Specifically, the deletion in the *PsRS* gene (resulting in SEQ ID NO: 33) and the deletion in the *PsGAS* gene (resulting in SEQ ID NO: 27) decreased content of raffinose plus stachyose from about 2.5% (control) to about 1.0% and about 1.6%, respectively, in flour, and from about 3.9% (control) to about 2.25% and about 2.0%, respectively, in PPC (FIG. 7A). The deletion in the *PsRS* gene (resulting in SEQ ID NO: 33) and the deletion in the *PsGAS* gene (resulting in SEQ ID NO: 27) increased sucrose content from about 2.4% (control) to about 3.4% and about 5.1%, respectively, in flour, and from about 2.3% (control) to about 2.7% and about 3.8%, respectively, in PPC (FIG. 7B).

**Table 1. Raffinose, Stachyose, and Sucrose Content in GAS and RS Mutant Pea Seeds (M4)**

| | Raffinose | Stachyose | Sucrose |
|---|---|---|---|
| Control | 0.84% | 4.74% | 3.36% |
| *Ps*GAS-G43D | 0.69% | 1.36% | 6.18% |
| *Ps*RS-W348* | 0.10% | 0.87% | 3.73% |

**Table 2. Raffinose and Stachyose Content in GAS and RS Mutant Pea Seeds (M5**

| Averages oligosaccharide values | Raffinose (% dry basis) | Stachyose (% dry basis) |
|---|---|---|
| Parent | 0.54 ± 0.05 | 2.30 ± 0.20 |
| *Ps*GAS-G43D | 0.55 ± 0.02 | 0.43 ± 0.06 |
| *Ps*RS-W348* | 0.09 ± 0.02 | 0.46 ± 0.09 |

### Example 6: Increased Protein Content in Plants with GAS and/or RS Mutations

Seed protein content was measured by the Dumas method, by combusting samples at a high temperature in the presence of high-purity oxygen, analyzing the gas from combustion for nitrogen content using a thermal conductivity detector, and calculating the amount of protein present in the sample using a conversion factor. The industry standard conversion factor for pea is 6.25.

As shown in Table 3, plants with a *GAS* or *RS* mutation had increased protein content relative to a control. Mutagen (EMS/ENU) treatment increased the seed protein content by about 6-7% as compared to a mutagen-untreated control. Of note, plants having a G43D mutation in the GAS protein and plants having a W348* mutation in the RS protein had increased seed protein content by about 4-5% relative to the mutagen-treated control.

**Table 3. Protein Content in GAS and RS Mutant Pea Seeds (M5)**

| Average protein values | Protein (% dry basis) | Relative to parent (% change / difference % dry basis) | Relative to EMS-treated control average (% change / difference % dry basis) |
|---|---|---|---|
| Parent | 29.5 ± 2.02 | | |
| EMS-treated control 1 | 36.5 ± 1.80 | 23.7% / 7.0% | 1.0% / 0.3% |
| EMS-treated control 2 | 35.8 ± 2.47 | 21.4% / 6.3% | -1.0% / -0.3% |
| *Ps*GAS-G43D | 40.8 ± 2.43 | 38.3% / 11.3% | 12.9% / 4.9% |
| *Ps*RS-W348* | 41.0 ± 1.83 | 39.0% / 11.6% | 13.4% / 4.7% |

The section headings used herein are for organizational purposes only and are not to be construed as limiting the subject matter described in any way.

It is appreciated that certain features of the disclosure, which are, for clarity, described in the context of separate embodiments, may also be provided in combination in a single embodiment. Conversely, various features of the disclosure, which are, for brevity, described in the context of a single embodiment, may also be provided separately or in any suitable sub-combination or as suitable in any other described embodiment of the disclosure. Certain features described in the context of various embodiments are not to be considered essential features of those embodiments, unless the embodiment is inoperative without those elements.

While various aspects of the invention are described herein, it is not intended that the invention be limited by any particular aspect. On the contrary, the invention encompasses various alternatives, modifications, and equivalents, as will be appreciated by those of skill in the art. Furthermore, where feasible, any of the aspects disclosed herein may be combined with each other (e.g., the feature according to one aspect may be added to the features of another aspect or replace an equivalent feature of another aspect) or with features that are well known in the art, unless indicated otherwise by context.

**TABLE 4. Sequence Descriptions**

| SEQ ID NO: | DESCRIPTION |
|---|---|
| 1 | Nucleic acid sequence of full-length *Pisum sativum* galactinol synthase (GAS) gene (*Pisat.03G015560*)*,* including regulatory regions |
| 2 | Nucleic acid sequence of full-length *Pisum sativum* galactinol synthase (GAS) gene *(Pisat.06G066360* or Cameor *Psat6g153600*) |
| 3 | Nucleic acid sequence of full-length *Pisum sativum* raffinose synthase (RS) gene *(Pisat.05G054130* or Cameor *Psat0s2597g0160*), including regulatory regions |
| 4 | Nucleic acid sequence of full-length *Pisum sativum* raffinose synthase (RS) gene (*Pisat.01G021000*) |
| 5 | Full-length coding sequence of *Pisum sativum* galactinol synthase (GAS) (*Pisat.03G015560*) |
| 6 | Full-length coding sequence of *Pisum sativum* galactinol synthase (GAS) (*Pisat.06G066360* or Cameor *Psat6g153600*) |
| 7 | Full-length coding sequence of *Pisum sativum* raffinose synthase (RS) (*Pisat.05G054130* or Cameor *Psat0s2597g0160*) |
| 8 | Full-length coding sequence of *Pisum sativum* raffinose synthase (RS) (*Pisat.01G021000*) |
| 9 | Amino acid sequence of full-length *Pisum sativum* galactinol synthase (GAS) encoded by *Pisat.03G015560* or Cameor *Psat3G018520* |
| 10 | Amino acid sequence of full-length *Pisum sativum* galactinol synthase (GAS) encoded by *Pisat.06G066360* or Cameor *Psat6g153600* |
| 11 | Amino acid sequence of full-length *Pisum sativum* raffinose synthase (RS) encoded by *Pisat.05G054130* or Cameor *Psat0s2597g0160* |
| 12 | Amino acid sequence of full-length *Pisum sativum* raffinose synthase (RS) encoded by *Pisat.01G021000* |
| 13 | Nucleic acid sequence encoding targeting sequence of *Pisum sativum* galactinol synthase (GAS) guide RNA1 |
| 14 | Nucleic acid sequence encoding targeting sequence of *Pisum sativum* raffinose synthase (RS) guide RNA1 |
| 15 | Nucleic acid sequence of mutated *PsGAS* gene (*Pisat.03G015560*) with G128A mutation in SEQ ID NO: 5 |
| 16 | Nucleic acid sequence of mutated *PsRS* gene (*Pisat.05G054130*) with G1044A mutation in SEQ ID NO: 7 |
| 17 | Amino acid sequence of mutated *Pisum sativum* galactinol synthase (GAS) with G43D mutation encoded by *Pisat.03G015560* with G128A mutation |
| 18 | Amino acid sequence of mutated *Pisum sativum* raffinose synthase (RS) with W348* mutation encoded by *Pisat.05G054130* with G1044A mutation |
| 19 | Forward primer for detecting G128A mutation in *PsGAS* gene *Pisat.03G015560* |
| 20 | Reverse primer for detecting G128A mutation in *PsGAS* gene *Pisat.03G015560* |
| 21 | Forward primer for detecting G1044A mutation in *PsRS* gene *Pisat.05G054130* |
| 22 | Reverse primer for detecting G1044A mutation in *PsRS* gene *Pisat.05G054130* |
| 23 | Forward primer for detecting mutation in *PsRS* near binding site of *PsRS* guide RNA |
| 24 | Reverse primer for detecting mutation in *PsRS* near binding site of *PsRS* guide RNA |
| 25 | Nucleic acid sequence of full-length *Pisum sativum* galactinol synthase (GAS) gene (*Pisat.03G015560*) |
| 26 | Nucleic acid sequence of mutated *PsGAS* gene (*Pisat.03G015560*) with G128A mutation in SEQ ID NO: 25 |
| 27 | Nucleic acid sequence of mutated *PsGAS* gene (*Pisat.03G015560*) with 5 bp deletion in SEQ ID NO:25 |
| 28 | Nucleic acid sequence of mutated *PsGAS* gene (*Pisat.03G015560*) with 7 bp deletion in SEQ ID NO:25 |
| 29 | Amino acid sequence of mutated *Pisum sativum* galactinol synthase (GAS) encoded by *Pisat.03G015560* with 5 bp deletion |
| 30 | Amino acid sequence of mutated *Pisum sativum* galactinol synthase (GAS) encoded by *Pisat.03G015560* with 7 bp deletion |
| 31 | Nucleic acid sequence of full-length *Pisum sativum* raffinose synthase (RS) gene (*Pisat.05G054130*) |
| 32 | Nucleic acid sequence of mutated *PsRS* gene (*Pisat.05G054130*) with G1044A mutation in SEQ ID NO: 31 |
| 33 | Nucleic acid sequence of mutated *PsRS* gene (*Pisat.05G054130*) with 8 bp deletion in SEQ ID NO: 31 |
| 34 | Nucleic acid sequence of mutated *PsRS* gene (*Pisat.05G054130*) with 7 bp deletion in SEQ ID NO: 31 |
| 35 | Amino acid sequence of mutated *Pisum sativum* raffinose synthase (RS) encoded by *Pisat.05G054130* with 8 bp deletion |
| 36 | Amino acid sequence of mutated *Pisum sativum* raffinose synthase (RS) encoded by *Pisat.05G054130* with 7 bp deletion |
| 37 | Nucleic acid sequence of full-length *Pisum sativum* galactinol synthase (GAS) gene (Cameor *Psat3G018520*) |
| 38 | Full-length coding sequence of *Pisum sativum* galactinol synthase (GAS) (Cameor *Psat3G018520*) |
| 39 | Nucleic acid sequence of full-length *Pisum sativum* raffinose synthase (RS) gene (Cameor *Psat1g055560*) |
| 40 | Full-length coding sequence of *Pisum sativum* raffinose synthase (RS) (Cameor *Psat1g055560*) |
| 41 | Amino acid sequence of full-length *Pisum sativum* raffinose synthase (RS) encoded by Cameor *Psat1g055560* |
| 42 | Nucleic acid sequence of full-length *Glycine max* galactinol synthase (GAS) gene *(Glyma.19G227800)* |
| 43 | Amino acid sequence of full-length *Glycine max* galactinol synthase (GAS) encoded by *Glyma.19G227800* |
| 44 | Nucleic acid sequence of mutated *GmGAS* gene (*Glyma.19G227800*) with 13 bp deletion |
| 45 | Nucleic acid sequence of mutated *GmGAS* gene (*Glyma.19G227800*) with 11 bp deletion |
| 46 | Nucleic acid sequence of mutated *GmGAS* gene (*Glyma.19G227800*) with 1 bp deletion |
| 47 | Amino acid sequence of mutated *Glycine max* galactinol synthase (GAS) encoded by *Glyma.19G227800* with 13 bp deletion |
| 48 | Amino acid sequence of mutated *Glycine max* galactinol synthase (GAS) encoded by *Glyma.19G227800* with 11 bp deletion |
| 49 | Amino acid sequence of mutated *Glycine max* galactinol synthase (GAS) encoded by *Glyma.19G227800* with 1 bp deletion |
| 50 | Nucleic acid sequence of full-length *Glycine max* galactinol synthase (GAS) gene *(Glyma.03G229800)* |
| 51 | Amino acid sequence of full-length *Glycine max* galactinol synthase (GAS) encoded by *Glyma.03G229800* |
| 52 | Nucleic acid sequence encoding targeting sequence of *Glycine max* galactinol synthase (GAS) guide RNA1 |

### Embodiments:

1. A plant or plant part comprising decreased galactinol synthase (GAS) activity and/or raffinose synthase (RS) activity compared to a control plant or plant part, wherein said plant or plant part comprises a genetic mutation that decreases the GAS activity and/or the RS activity.
2. The plant or plant part of embodiment 1, comprising decreased raffinose family oligosaccharide (RFO) content and/or increased sucrose content as compared to a control plant or plant part.
3. The plant or plant part of embodiment 2, wherein the RFO comprises raffinose and/or stachyose.
4. The plant or plant part of any one of embodiments 1-3, wherein the mutation comprises one or more insertions, substitutions, or deletions in at least one native *GAS* and/or *RS* gene or homolog thereof or regulatory region thereof in said plant or plant part, wherein:
   an expression level of said at least one mutated *GAS* and/or *RS* gene or homolog thereof is reduced compared to corresponding at least one native *GAS* and/or *RS* gene or homolog thereof without said mutation; and/or
   level or activity of a GAS and/or RS protein encoded by said at least one mutated *GAS* and/or *RS* gene or homolog thereof is reduced compared to a GAS and/or RS protein encoded by corresponding at least one native *GAS* and/or *RS* gene or homolog thereof without said mutation.
5. The plant or plant part of embodiment 3, wherein the mutation is located at least partially in a *GAS* or *RS* gene or regulatory region thereof, wherein:
   (i) said *GAS* or *RS* gene comprises a nucleic acid sequence having at least 80% sequence identity to a nucleic acid sequence of any one of SEQ ID NO: 1-8, 25, 31, 37-40, 42, and 50, wherein said nucleic acid sequence encodes a polypeptide that retains GAS or RS activity;
   (ii) said *GAS* or *RS* gene comprises the nucleic acid sequence of any one of SEQ ID NO: 1-8, 25, 31, 37-40, 42, and 50;
   (iii) said *GAS* or *RS* gene encodes a polypeptide comprising an amino acid sequence having at least 80% sequence identity to an amino acid sequence of any one of SEQ ID NO: 9-12, 41, 43, and 51, wherein said polypeptide retains GAS or RS activity; and/or
   (iv) said *GAS* or *RS* gene encodes a polypeptide comprising an amino acid sequence of any one of SEQ ID NO: 9-12, 41, 43, and 51.
6. The plant or plant part of embodiment 4, comprising:
   (i) a mutated *Pisum sativum GAS* gene comprising an insertion, a substitution, or a deletion of one or more nucleotides of SEQ ID NO: 1, 2, 5, 6, 25, 37, or 38;
   (ii) a mutated *Pisum sativum RS* gene comprising an insertion, a substitution, or a deletion of one or more nucleotides of SEQ ID NO: 3, 4, 7, 8, 31, 39, or 40; and/or
   (iii) a mutated *Glycine max GAS* gene comprising an insertion, a substitution, or a deletion of one or more nucleotides of SEQ ID NO: 42 or 50.
7. The plant or plant part of embodiment 4 or 5, comprising:
   (i) a mutated *Pisum sativum GAS* gene comprising a G to A substitution of nucleotide 128 of SEQ ID NO: 5 or 25, or comprising a nucleic acid sequence of SEQ ID NO: 15 or 26;
   (ii) a mutated *Pisum sativum GAS* gene comprising a deletion of nucleotides 115-119 of SEQ ID NO: 25, or comprising a nucleic acid sequence of SEQ ID NO: 27;
   (iii) a mutated *Pisum sativum GAS* gene comprising a deletion of nucleotides 111-117 of SEQ ID NO: 25, or comprising a nucleic acid sequence of SEQ ID NO: 28;
   (iv) a mutated *Pisum sativum RS* gene comprising a G to A substitution of nucleotide 1044 of SEQ ID NO: 7 or 31, or comprising a nucleic acid sequence of SEQ ID NO: 16 or 32;
   (v) a mutated *Pisum sativum RS* gene comprising a deletion of nucleotides 1246-1253 of SEQ ID NO: 31, or comprising a nucleic acid sequence of SEQ ID NO: 33;
   (vi) a mutated *Pisum sativum RS* gene comprising a deletion of nucleotides 1248-1254 of SEQ ID NO: 31, or comprising a nucleic acid sequence of SEQ ID NO: 34;
   (vii) a mutated *Pisum sativum* GAS protein comprising a G to D substitution of amino acid 43 of SEQ ID NO: 9, or comprising an amino acid sequence of SEQ ID NO: 17;
   (viii) a mutated *Pisum sativum* GAS protein encoded by the nucleic acid sequence of SEQ ID NO: 27, or comprising an amino acid sequence of SEQ ID NO: 29;
   (ix) a mutated *Pisum sativum* GAS protein encoded by the nucleic acid sequence of SEQ ID NO: 28, or comprising an amino acid sequence of SEQ ID NO: 30;
   (x) a truncated *Pisum sativum* RS protein comprising a deletion of amino acids 348-798 of SEQ ID NO: 11, or comprising an amino acid sequence of SEQ ID NO: 18;
   (xi) a mutated *Pisum sativum* RS protein encoded by the nucleic acid sequence of SEQ ID NO: 33, or comprising an amino acid sequence of SEQ ID NO: 35;
   (xii) a mutated *Pisum sativum* RS protein encoded by the nucleic acid sequence of SEQ ID NO: 34, or comprising an amino acid sequence of SEQ ID NO: 36;
   (xiii) a mutated *Glycine max GAS* gene comprising a deletion of nucleotides 197-209 of SEQ ID NO: 42, or comprising a nucleic acid sequence of SEQ ID NO: 44;
   (xiv) a mutated *Glycine max GAS* gene comprising a deletion of nucleotides 199-209 of SEQ ID NO: 42, or comprising a nucleic acid sequence of SEQ ID NO: 45;
   (xv) a mutated *Glycine max GAS* gene comprising a deletion of nucleotide 204 of SEQ ID NO: 42, or comprising a nucleic acid sequence of SEQ ID NO: 46; and/or
   (xvi) a mutated *Glycine max* GAS protein encoded by the nucleic acid sequence of any one of SEQ ID NOs: 44-46, or comprising an amino acid sequence of any one of SEQ ID NOs: 47-49.
8. The plant or plant part of any one of embodiments 4-6, wherein the mutation comprises a missense mutation, a nonsense mutation, or an out-of-frame mutation of the at least one *GAS* and/or *RS* gene or homolog thereof.
9. The plant or plant part of any one of embodiments 1-8, wherein said plant or plant part comprises 2-5 genes encoding a GAS and/or RS protein.
10. The plant or plant part according to embodiment 9, wherein said 2-5 genes have less than 100% sequence identity to one another.
11. The plant or plant part of any one of embodiments 1-10, wherein said plant or plant part is a legume.
12. The plant or plant part of embodiment 11, wherein said plant or plant part is selected from the group consisting of pea (*Pisum sativum*), soybean (*Glycine max*), beans (*Phaseolus* spp., *Vigna* spp.), common bean (*Phaseolus vulgaris*), mung bean (*Vigna radiata*), cowpea (*Vigna unguiculata*)*,* adzuki bean (*Vigna angularis*)*,* fava bean (*Vicia faba*)*,* chickpea (*Cicer arietinum*)*,* peanut *(Arachis hypogaea*)*,* lentils (*Lens culinaris*, *Lens esculenta*)*,* lupins (*Lupinus* spp.), white lupin (*Lupinus albus*), mesquite (*Prosopis* spp.), carob (*Ceratonia siliqua*)*,* tamarind (*Tamarindus indica*), alfalfa (*Medicago sativa*), barrel medic (*Medicago truncatula*), birdsfood trefoil (*Lotus japonicus*), licorice (*Glycyrrhiza glabra*), and clover (*Trifolium* spp.).
13. The plant or plant part of any one of embodiments 1-10, wherein said plant or plant part is selected from the group consisting of corn (*Zea mays*), Brassica species, *Brassica napus*, *Brassica rapa*, *Brassica juncea*, rice (*Oryza sativa*), rye (*Secale cereale*), sorghum (*Sorghum bicolor*, *Sorghum vulgare*), millet, pearl millet (*Pennisetum glaucum*)*,* proso millet (*Panicum miliaceum*), foxtail millet (*Setaria italica*)*,* finger millet (*Eleusine coracana*)*,* sunflower (*Helianthus annuus*), safflower (*Carthamus tinctorius*), wheat (*Triticum aestivum*), tobacco (*Nicotiana tabacum*), potato (*Solanum tuberosum*), peanuts *(Arachis hypogaea*)*,* cotton (*Gossypium barbadense*, *Gossypium hirsutum*), sweet potato (*Ipomoea batatus*), cassava (*Manihot esculenta*)*,* coffee (*Coffea spp.*), coconut (*Cocos nucifera*)*,* pineapple (*Ananas comosus*), citrus trees (*Citrus spp.*), cocoa (*Theobroma cacao*), tea (*Camellia sinensis*), banana (*Musa spp.*), avocado (*Persea americana*), fig (*Ficus casica*)*,* guava (*Psidium guajava*)*,* mango (*Mangifera indica*), olive (*Olea europaea*), papaya (*Carica papaya*), cashew (*Anacardium occidentale*), macadamia (*Macadamia integrifolia*)*,* almond (*Prunus amygdalus*)*,* sugar beets (*Beta vulgaris*), sugarcane (*Saccharum spp.*), oats, barley, vegetables, ornamentals, and conifers.
14. The plant or plant part of any one of embodiments 1-13, wherein said plant or plant part is a seed.
15. A population of plants or plant parts comprising the plant or plant part of any one of embodiments 1-14, wherein the population comprises decreased GAS and/or RS activity, decreased raffinose family oligonucleotide (RFO) content and/or increased sucrose content compared to a control population.
16. The population of plants or plant parts of embodiment 15, wherein said plant or plant part is a seed, and said population is a population of seeds.
17. The plant, plant part, or population of plants or plant parts of any one of embodiments 1-16, wherein the plant, plant part, or population of plants or plant parts comprises seed raffinose content that is 85% or less relative to a control, seed stachyose content that is 50% or less relative to a control, and/or seed sucrose content that is increased by about 10% or more relative to a control.
18. The plant, plant part, or population of plants or plant parts of any one of embodiments 1-17, wherein the plant, plant part, or population of plants or plant parts comprises seed raffinose content of about 0.7% or less, seed stachyose content of about 1.5% or less, and/or seed sucrose content of about 3.0% or more dry weight of total seed content.
19. The plant, plant part, or population of plants or plant parts of embodiment 18, wherein the plant, plant part, or population of plants or plant parts comprises seed raffinose content of about 0.3% or less dry weight of total seed content.
20. The plant, plant part, or population of plants or plant parts of embodiment 18 or 19, wherein the plant, plant part, or population of plants or plant parts comprises seed stachyose content of about 1.4% or less, and/or seed sucrose content of about 3.7% or more dry weight of total seed content.
21. A method for decreasing raffinose family oligosaccharide (RFO) content and/or increasing protein content in a plant or plant part, said method comprising decreasing galactinol synthase (GAS) activity and/or raffinose synthase (RS) activity in the plant or plant part.
22. The method of embodiment 21, comprising introducing a genetic mutation that decreases the GAS and/or RS activity into said plant or plant part, wherein:
   the GAS and/or RS activity is decreased; and
   RFO content is decreased and/or sucrose content is increased in the plant or plant part relative to a control plant or plant part.
23. The method of embodiment 22, further comprising introducing the mutation into a plant cell, and regenerating said plant or plant part from said plant cell.
24. The method of embodiment 22 or 23, wherein the mutation comprises one or more insertions, substitutions, or deletions in at least one *GAS* and/or *RS* gene or homolog thereof or regulatory region thereof in said plant or plant part, wherein:
   an expression level of said at least one *GAS* and/or *RS* gene or homolog thereof is reduced by said mutation; and/or
   level or activity of a GAS and/or RS protein encoded by said at least one *GAS* and/or *RS* gene or homolog thereof is reduced by said mutation.
25. The method of embodiment 24, wherein the mutation is introduced at least partially into a *GAS* or *RS* gene or regulatory region thereof in said plant or plant part, wherein:
   (i) said *GAS* or *RS* gene comprises a nucleic acid sequence having at least 80% sequence identity to a nucleic acid sequence of any one of SEQ ID NO: 1-8, 25, 31, 37-40, 42, and 50, wherein said nucleic acid sequence encodes a polypeptide that retains GAS or RS activity;
   (ii) said *GAS* or *RS* gene comprises the nucleic acid sequence of any one of SEQ ID NO: 1-8, 25, 31, 37-40, 42, and 50;
   (iii) said *GAS* or *RS* gene encodes a polypeptide comprising an amino acid sequence having at least 80% sequence identity to an amino acid sequence of any one of SEQ ID NO: 9-12, 41, 43, and 51, wherein said polypeptide retains GAS or RS activity; and/or
   (iv) said *GAS* or *RS* gene encodes a polypeptide comprising an amino acid sequence of any one of SEQ ID NO: 9-12, 41, 43, and 51.
26. The method of embodiment 25, wherein the mutation comprises an insertion, a substitution, or a deletion of one or more nucleotides of (i) SEQ ID NO: 1, 2, 5, 6, 25, 37, or 38 in a *Pisum sativum GAS* gene, (ii) SEQ ID NO: 3, 4, 7, 8, 31, 39, or 40 in a *Pisum sativum RS* gene, and/or (iii) SEQ ID NO: 42 or 50 in a *Glycine max* GAS gene.
27. The method of embodiment 25 or 26, wherein:
   (i) the mutation comprises a G to A substitution of nucleotide 128 of SEQ ID NO: 5 or 25, or said plant or plant part comprises a polynucleotide comprising a nucleic acid sequence of SEQ ID NO: 15 or 26 when said mutation is introduced;
   (ii) the mutation comprises a deletion of nucleotides 115-119 of SEQ ID NO: 25, or said plant or plant part comprises a polynucleotide comprising a nucleic acid sequence of SEQ ID NO: 27 when said mutation is introduced;
   (iii) the mutation comprises a deletion of nucleotides 111-117 of SEQ ID NO: 25, or said plant or plant part comprises a polynucleotide comprising a nucleic acid sequence of SEQ ID NO: 28 when said mutation is introduced;
   (iv) the mutation comprises a G to A substitution of nucleotide 1044 of SEQ ID NO: 7 or 31, or said plant or plant part comprises a polynucleotide comprising a nucleic acid sequence of SEQ ID NO: 16 or 32 when said mutation is introduced;
   (v) the mutation comprises a deletion of nucleotides 1246-1253 of SEQ ID NO: 31, or said plant or plant part comprises a polynucleotide comprising a nucleic acid sequence of SEQ ID NO: 33 when said mutation is introduced;
   (vi) the mutation comprises a deletion of nucleotides 1248-1254 of SEQ ID NO: 31, or said plant or plant part comprises a polynucleotide comprising a nucleic acid sequence of SEQ ID NO: 34 when said mutation is introduced;
   (vii) the mutation produces a G to D substitution of amino acid 43 of SEQ ID NO: 9, or said plant or plant part comprises a polypeptide comprising an amino acid sequence of SEQ ID NO: 17 when said mutation is introduced;
   (viii) the mutation produces a mutated *Pisum sativum* GAS protein comprising an amino acid sequence of SEQ ID NO: 29 or 30;
   (ix) the mutation produces a deletion of amino acids 348-798 of SEQ ID NO: 11, or said plant or plant part comprises a polypeptide comprising an amino acid sequence of SEQ ID NO: 18 when said mutation is introduced;
   (x) the mutation produces a mutated *Pisum sativum* RS protein comprising an amino acid sequence of SEQ ID NO: 35 or 36;
   (xi) the mutation comprises a deletion of nucleotides 197-209 of SEQ ID NO: 42, or said plant or plant part comprises a polynucleotide comprising a nucleic acid sequence of SEQ ID NO: 44 when said mutation is introduced;
   (xii) the mutation comprises a deletion of nucleotides 199-209 of SEQ ID NO: 42, or said plant or plant part comprises a polynucleotide comprising a nucleic acid sequence of SEQ ID NO: 45 when said mutation is introduced;
   (xiii) the mutation comprises a deletion of nucleotide 204 of SEQ ID NO: 42, or said plant or plant part comprises a polynucleotide comprising a nucleic acid sequence of SEQ ID NO: 46 when said mutation is introduced; and/or
   (xiv) the mutation produces a mutated Glycine max GAS protein comprising an amino acid sequence of any one of SEQ ID NO: 47-49.
28. The method of any one of embodiments 24-26, wherein introducing the mutation comprises introducing a missense mutation, a nonsense mutation, or an out-of-frame mutation into said at least one native *GAS* and/or *RS* gene or homolog thereof.
29. The method of any one of embodiments 22-28, further comprising contacting the plant or plant part with a mutagen, thereby introducing said mutation into said plant or plant part.
30. The method of embodiment 29, wherein the mutagen is ethyl methanesulfonate (EMS) and/or N-ethyl-N-nitrosourea (ENU).
31. The method of any one of embodiments 22-30, further comprising introducing editing reagents or a nucleic acid construct encoding said editing reagents into said plant, plant part, or plant cell, thereby introducing said mutation into said plant or plant part.
32. The method of embodiment 31, wherein said editing reagents comprise at least one nuclease, wherein the nuclease cleaves a target site in a genome of said plant, plant part, or plant cell, and wherein said mutation is introduced at said cleaved target site.
33. The method of embodiment 32, wherein the at least one nuclease comprises a CRISPR nuclease.
34. The method of embodiment 33, wherein the CRISPR nuclease is a Type II CRISPR system nuclease, a Type V CRISPR system nuclease, a Cas9 nuclease, a Cas12a (Cpf1) nuclease, or a Cms1 nuclease.
35. The method of embodiment 33, wherein the CRISPR nuclease is a Cas12a nuclease or an ortholog thereof.
36. The method of any one of embodiments 31-35, wherein the editing reagents comprise one or more guide RNAs (gRNAs).
37. The method of embodiment 36, wherein the one or more gRNAs comprise a nucleic acid sequence complementary to a region of a nucleic acid sequence comprising at least one native *GAS* or *RS* gene or homolog thereof or regulatory region thereof in said plant or plant part.
38. The method of embodiment 36 or 37, wherein at least one of the one or more gRNAs comprises a nucleic acid sequence encoded by:
   (i) a nucleic acid sequence that shares at least 80% sequence identity with a nucleic acid sequence of SEQ ID NO: 13, 14, or 52; or
   (ii) the nucleic acid sequence of SEQ ID NO: 13, 14, or 52.
39. The method of any one of embodiments 22-38, wherein the RFO comprises raffinose and/or stachyose.
40. The method of any one of embodiments 21-39, wherein said plant or plant part is a legume.
41. The method of embodiment 40, wherein said plant or plant part is selected from the group consisting of pea (*Pisum sativum*), soybean (*Glycine max*), beans (*Phaseolus* spp., *Vigna* spp.), common bean (*Phaseolus vulgaris*), mung bean (*Vigna radiata*), cowpea (*Vigna unguiculata*)*,* adzuki bean (*Vigna angularis*)*,* fava bean (*Viciafaba*)*,* chickpea (*Cicer arietinum*)*,* peanut *(Arachis hypogaea*)*,* lentils (*Lens culinaris*, *Lens esculenta*)*,* lupins (*Lupinus* spp.), white lupin (*Lupinus albus*), mesquite (*Prosopis* spp.), carob (*Ceratonia siliqua*)*,* tamarind (*Tamarindus indica*), alfalfa (*Medicago sativa*), barrel medic (*Medicago truncatula*), birdsfood trefoil (*Lotus japonicus*), licorice (*Glycyrrhiza glabra*), and clover (*Trifolium* spp.).
42. The method of any one of embodiments 21-39, wherein said plant or plant part is selected from the group consisting of corn (*Zea mays*), Brassica species, *Brassica napus, Brassica rapa, Brassica juncea,* rice (*Oryza sativa*)*,* rye (*Secale cereale*), sorghum (*Sorghum bicolor*, *Sorghum vulgare*), millet, pearl millet (*Pennisetum glaucum*), proso millet (*Panicum miliaceum*), foxtail millet (*Setaria italica*)*,* finger millet (*Eleusine coracana*)*,* sunflower (*Helianthus annuus*), safflower (*Carthamus tinctorius*), wheat (*Triticum aestivum*), tobacco (*Nicotiana tabacum*), potato (*Solanum tuberosum*), peanuts *(Arachis hypogaea*)*,* cotton (*Gossypium barbadense*, *Gossypium hirsutum*), sweet potato (*Ipomoea batatus*), cassava (*Manihot esculenta*)*,* coffee (*Coffea spp.*), coconut (*Cocos nucifera*)*,* pineapple (*Ananas comosus*), citrus trees (*Citrus spp.*), cocoa (*Theobroma cacao*), tea (*Camellia sinensis*), banana (*Musa spp.*), avocado (*Persea americana*), fig (*Ficus casica*)*,* guava (*Psidium guajava*)*,* mango (*Mangifera indica*), olive (*Olea europaea*), papaya (*Carica papaya*), cashew (*Anacardium occidentale*), macadamia (*Macadamia integrifolia*), almond (*Prunus amygdalus*), sugar beets (*Beta vulgaris*), sugarcane (*Saccharum spp.*), oats, barley, vegetables, ornamentals, and conifers.
43. A plant or plant part produced by the method of any one of embodiments 21-42, wherein said plant or plant part comprises reduced GAS and/or RS activity compared to a control plant or plant part.
44. The plant or plant part of embodiment 43, comprising decreased raffinose family oligosaccharide content and/or increased sucrose content compared to a control plant or plant part.
45. The plant or plant part of embodiment 43 or 44, wherein said plant or plant part is a seed.
46. A population of plants or plant parts produced by the method of any one of embodiments 21-42, wherein the population comprises decreased GAS and/or RS activity, decreased raffinose family oligosaccharide content and/or increased sucrose content compared to a control population.
47. The population of plants or plant parts of embodiment 46, wherein said population is a population of seeds.
48. The plant, plant part, or population of plants or plant parts of any one of embodiments 43-47, wherein the plant, plant part, or population of plants or plant parts comprises seed raffinose content that is 85% or less relative to a control, seed stachyose content that is 50% or less relative to a control, and/or seed sucrose content that is increased by about 10% or more relative to a control.
49. The plant, plant part, or population of plants or plant parts of any one of embodiments 43-48, wherein the plant, plant part, or population of plants or plant parts comprises seed raffinose content of about 0.7% or less, seed stachyose content of about 1.5% or less, and/or seed sucrose content of about 3.0% or more dry weight of total seed content.
50. The plant, plant part, or population of plants or plant parts of embodiment 49, wherein the plant, plant part, or population of plants or plant parts comprises seed raffinose content of about 0.3% or less dry weight of total seed content.
51. The plant, plant part, or population of plants or plant parts of embodiment 49 or 50, wherein the plant, plant part, or population of plants or plant parts comprises seed stachyose content of about 1.4% or less, and/or seed sucrose content of about 3.7% or more dry weight of total seed content.
52. A seed composition produced from the plant, plant part, or population of plants or plant parts of any one of embodiments 1-20 and 43-51.
53. A protein and/or oil composition produced from the plant, plant part, population of plants or plant parts of any one of embodiments 1-20 and 43-51, or the seed composition of embodiment 52.
54. A food or beverage product comprising the plant, plant part, or plant population of any one of embodiments 1-20 and 43-51, the seed composition of embodiment 52, or the protein and/or oil composition of embodiment 53.
55. The composition of embodiment 53 or 54, or the product of embodiment 55, comprising decreased raffinose family oligosaccharide content and/or increased sucrose content compared to a control composition or product.
56. A pea composition comprising raffinose content of about 0.7% or less, and/or stachyose content of about 1.5% or less dry weight.
57. The pea composition of embodiment 56, comprising raffinose content of about 0.3% or less dry weight.
58. The pea composition of embodiment 56 or 57, further comprising sucrose content of about 3.0% or more dry weight.
59. The pea composition of any one of embodiments 56-58, comprising stachyose content of about 1.4% or less and/or sucrose content of about 3.7% or more dry weight.
60. The pea composition of any one of embodiments 56-59, wherein the pea composition comprises a pea seed composition, a pea protein composition, a pea protein concentrate, a pea protein isolate, pea flour, pea flake, pea white flake, textured pea protein, or a pea oil composition.
61. The pea composition of any one of embodiments 56-60, comprising a mutated *GAS* and/or RS gene or homolog thereof or regulatory region thereof.
62. The pea composition of embodiment 61, comprising:
   (i) a mutated *Pisum sativum GAS* gene comprising an insertion, a substitution, or a deletion of one or more nucleotides of SEQ ID NO: 1, 2, 5, 6, 25, 37, or 38; and/or
   (ii) a mutated *Pisum sativum RS* gene comprising an insertion, a substitution, or a deletion of one or more nucleotides of SEQ ID NO: 3, 4, 7, 8, 31, 39, or 40.
63. The pea composition of embodiment 62, comprising:
   (i) a mutated *Pisum sativum GAS* gene comprising a G to A substitution of nucleotide 128 of SEQ ID NO: 5 or 25, or comprising a nucleic acid sequence of SEQ ID NO: 15 or 26;
   (ii) a mutated *Pisum sativum GAS* gene comprising a deletion of nucleotides 115-119 of SEQ ID NO: 25, or comprising a nucleic acid sequence of SEQ ID NO: 27;
   (iii) a mutated *Pisum sativum GAS* gene comprising a deletion of nucleotides 111-117 of SEQ ID NO: 25, or comprising a nucleic acid sequence of SEQ ID NO: 28;
   (iv) a mutated *Pisum sativum RS* gene comprising a G to A substitution of nucleotide 1044 of SEQ ID NO: 7 or 31, or comprising a nucleic acid sequence of SEQ ID NO: 16 or 32;
   (v) a mutated *Pisum sativum RS* gene comprising a deletion of nucleotides 1246-1253 of SEQ ID NO: 31, or comprising a nucleic acid sequence of SEQ ID NO: 33;
   (vi) a mutated *Pisum sativum RS* gene comprising a deletion of nucleotides 1248-1254 of SEQ ID NO: 31, or comprising a nucleic acid sequence of SEQ ID NO: 34;
   (vii) a mutated *Pisum sativum* GAS protein comprising a G to D substitution of amino acid 43 of SEQ ID NO: 9, or comprising an amino acid sequence of SEQ ID NO: 17;
   (viii) a mutated *Pisum sativum* GAS protein encoded by the nucleic acid sequence of SEQ ID NO: 27, or comprising an amino acid sequence of SEQ ID NO: 29;
   (ix) a mutated *Pisum sativum* GAS protein encoded by the nucleic acid sequence of SEQ ID NO: 28, or comprising an amino acid sequence of SEQ ID NO: 30;
   (x) a truncated *Pisum sativum* RS protein comprising a deletion of amino acids 348-798 of SEQ ID NO: 11, or comprising an amino acid sequence of SEQ ID NO: 18;
   (xi) a mutated *Pisum sativum* RS protein encoded by the nucleic acid sequence of SEQ ID NO: 33, or comprising an amino acid sequence of SEQ ID NO: 35; and/or
   (xii) a mutated *Pisum sativum* RS protein encoded by the nucleic acid sequence of SEQ ID NO: 34, or comprising an amino acid sequence of SEQ ID NO: 36.
64. A nucleic acid molecule comprising a nucleic acid sequence of a mutated *GAS* or *RS* gene or coding sequence thereof, wherein said mutation is located in a *GAS* or *RS* gene:
   (i) comprising a nucleic acid sequence having at least 80% sequence identity to a nucleic acid sequence of any one of SEQ ID NO: 1-8, 25, 31, 37-40, 42, and 50, wherein said nucleic acid sequence encodes a polypeptide that retains GAS or RS activity;
   (ii) comprising the nucleic acid sequence of any one of SEQ ID NO: 1-8, 25, 31, 37-40, 42, and 50;
   (iii) encoding a polypeptide comprising an amino acid sequence having at least 80% sequence identity to an amino acid sequence of any one of SEQ ID NO: 9-12, 41, 43, and 51, wherein said polypeptide retains GAS or RS activity; and/or
   (iv) encoding a polypeptide comprising an amino acid sequence of any one of SEQ ID NO: 9-12, 41, 43, and 51,
   wherein said mutation decreases level or activity of a GAS and/or RS protein encoded by said *GAS* or *RS* gene.
65. The nucleic acid molecule of embodiment 64, wherein said nucleic acid sequence:
   (i) has at least 80% identity to a nucleic acid sequence of any one of SEQ ID NOs: 15, 16, 26-28, 32-34, and 44-46;
   (ii) comprises the nucleic acid sequence of any one of SEQ ID NOs: 15, 16, 26-28, 32-34, and 44-46;
   (iii) encodes a polypeptide comprising an amino acid sequence having at least 80% sequence identity to an amino acid sequence of any one of SEQ ID NOs: 17, 18, 29, 30, 35, 36, and 47-49; and/or
   (iv) encodes a polypeptide comprising an amino acid sequence of any one of SEQ ID NOs: 17, 18, 29, 30, 35, 36, and 47-49.
66. A DNA construct comprising, in operable linkage:
   (i) a promoter that is functional in a plant cell; and
   (ii) the nucleic acid molecule of embodiment 63 or 64.
67. A cell comprising the nucleic acid molecule of embodiment 64 or 65, or the DNA construct of embodiment 66.
68. The cell of embodiment 67, wherein the cell is a plant cell or a bacterial cell.

## Claims

1. A plant or plant part comprising decreased galactinol synthase (GAS) activity and/or raffinose synthase (RS) activity compared to a control plant or plant part, wherein said plant or plant part comprises a genetic mutation that decreases the GAS activity and/or the RS activity.

2. The plant or plant part of claim 1,
wherein the plant or plant part comprises decreased raffinose family oligosaccharide (RFO) content and/or increased sucrose content as compared to a control plant or plant part, optionally wherein the RFO comprises raffinose and/or stachyose;
wherein the mutation comprises one or more insertions, substitutions, or deletions in at least one native GAS and/or RS gene or homolog thereof or regulatory region thereof in said plant or plant part, wherein
- an expression level of said at least one mutated GAS and/or RS gene or homolog thereof is reduced compared to corresponding at least one native GAS and/or RS gene or homolog thereof without said mutation, and/or
- level or activity of a GAS and/or RS protein encoded by said at least one mutated GAS and/or RS gene or homolog thereof is reduced compared to a GAS and/or RS protein encoded by corresponding at least one native GAS and/or RS gene or homolog thereof without said mutation;
wherein the mutation is located at least partially in a *GAS* or *RS* gene or regulatory region thereof, wherein:
(i) said *GAS* or *RS* gene comprises a nucleic acid sequence having at least 80% sequence identity to a nucleic acid sequence of any one of SEQ ID NO: 1-8, 25, 31, 37-40, 42, and 50, wherein said nucleic acid sequence encodes a polypeptide that retains GAS or RS activity;
(ii) said *GAS* or *RS* gene comprises the nucleic acid sequence of any one of SEQ ID NO: 1-8, 25, 31, 37-40, 42, and 50;
(iii) said *GAS* or *RS* gene encodes a polypeptide comprising an amino acid sequence having at least 80% sequence identity to an amino acid sequence of any one of SEQ ID NO: 9-12, 41, 43, and 51, wherein said polypeptide retains GAS or RS activity; and/or
(iv) said GAS or RS gene encodes a polypeptide comprising an amino acid sequence of any one of SEQ ID NO: 9-12, 41, 43, and 51; and/or
wherein the plant or plant part comprises:
(v) a mutated *Pisum sativum* GAS gene comprising an insertion, a substitution, or a deletion of one or more nucleotides of SEQ ID NO: 1, 2, 5, 6, 25, 37, or 38;
(vi) a mutated *Pisum sativum* RS gene comprising an insertion, a substitution, or a deletion of one or more nucleotides of SEQ ID NO: 3, 4, 7, 8, 31, 39, or 40; and/or
(vii) a mutated Glycine max GAS gene comprising an insertion, a substitution, or a deletion of one or more nucleotides of SEQ ID NO: 42 or 50.

3. The plant or plant part of claim 1 or 2, comprising:
(i) a mutated *Pisum sativum* GAS gene comprising a G to A substitution of nucleotide 128 of SEQ ID NO: 5 or 25, or comprising a nucleic acid sequence of SEQ ID NO: 15 or 26;
(ii) a mutated *Pisum sativum* GAS gene comprising a deletion of nucleotides 115-119 of SEQ ID NO: 25, or comprising a nucleic acid sequence of SEQ ID NO: 27;
(iii) a mutated *Pisum sativum* GAS gene comprising a deletion of nucleotides 111-117 of SEQ ID NO: 25, or comprising a nucleic acid sequence of SEQ ID NO: 28;
(iv) a mutated *Pisum sativum* RS gene comprising a G to A substitution of nucleotide 1044 of SEQ ID NO: 7 or 31, or comprising a nucleic acid sequence of SEQ ID NO: 16 or 32;
(v) a mutated *Pisum sativum* RS gene comprising a deletion of nucleotides 1246-1253 of SEQ ID NO: 31, or comprising a nucleic acid sequence of SEQ ID NO: 33;
(vi) a mutated *Pisum sativum* RS gene comprising a deletion of nucleotides 1248-1254 of SEQ ID NO: 31, or comprising a nucleic acid sequence of SEQ ID NO: 34;
(vii) a mutated *Pisum sativum* GAS protein comprising a G to D substitution of amino acid 43 of SEQ ID NO: 9, or comprising an amino acid sequence of SEQ ID NO: 17;
(viii) a mutated *Pisum sativum* GAS protein encoded by the nucleic acid sequence of SEQ ID NO: 27, or comprising an amino acid sequence of SEQ ID NO: 29;
(ix) a mutated *Pisum sativum* GAS protein encoded by the nucleic acid sequence of SEQ ID NO: 28, or comprising an amino acid sequence of SEQ ID NO: 30;
(x) a truncated *Pisum sativum* RS protein comprising a deletion of amino acids 348-798 of SEQ ID NO: 11, or comprising an amino acid sequence of SEQ ID NO: 18;
(xi) a mutated *Pisum sativum* RS protein encoded by the nucleic acid sequence of SEQ ID NO: 33, or comprising an amino acid sequence of SEQ ID NO: 35;
(xii) a mutated *Pisum sativum* RS protein encoded by the nucleic acid sequence of SEQ ID NO: 34, or comprising an amino acid sequence of SEQ ID NO: 36;
(xiii) a mutated Glycine max GAS gene comprising a deletion of nucleotides 197-209 of SEQ ID NO: 42, or comprising a nucleic acid sequence of SEQ ID NO: 44;
(xiv) a mutated Glycine max GAS gene comprising a deletion of nucleotides 199-209 of SEQ ID NO: 42, or comprising a nucleic acid sequence of SEQ ID NO: 45;
(xv) a mutated Glycine max GAS gene comprising a deletion of nucleotide 204 of SEQ ID NO: 42, or comprising a nucleic acid sequence of SEQ ID NO: 46; and/or
(xvi) a mutated Glycine max GAS protein encoded by the nucleic acid sequence of any one of SEQ ID NOs: 44-46, or comprising an amino acid sequence of any one of SEQ ID NOs: 47-49,
optionally wherein said plant or plant part is a legume.

4. The plant or plant part of any one of the preceding claims, wherein the plant or plant part comprises:
seed raffinose content that is 85% or less relative to a control, seed stachyose content that is 50% or less relative to a control, and/or seed sucrose content that is increased by about 10% or more relative to a control; and/or
seed raffinose content of about 0.7% or less, such as about 0.3% or less, seed stachyose content of about 1.5% or less, such as about 1.4% or less, and/or seed sucrose content of about 3.0% or more, such as about 3.7% or more, dry weight of total seed content.

5. A method for decreasing raffinose family oligosaccharide (RFO) content and/or increasing protein content in a plant or plant part, said method comprising decreasing galactinol synthase (GAS) activity and/or raffinose synthase (RS) activity in the plant or plant part.

6. The method of claim 5, comprising introducing a genetic mutation that decreases the GAS and/or RS activity into said plant or plant part, wherein:
the GAS and/or RS activity is decreased; and
RFO content is decreased and/or sucrose content is increased in the plant or plant part relative to a control plant or plant part, optionally wherein the RFO content is raffinose and/or stachyose content,
optionally wherein the mutation comprises one or more insertions, substitutions, or deletions in at least one GAS and/or RS gene or homolog thereof or regulatory region thereof in said plant or plant part, wherein:
an expression level of said at least one GAS and/or RS gene or homolog thereof is reduced by said mutation; and/or
level or activity of a GAS and/or RS protein encoded by said at least one GAS and/or RS gene or homolog thereof is reduced by said mutation;
optionally wherein the mutation is introduced at least partially into a GAS or RS gene or regulatory region thereof in said plant or plant part, wherein:
(i) said GAS or RS gene comprises a nucleic acid sequence having at least 80% sequence identity to a nucleic acid sequence of any one of SEQ ID NO: 1-8, 25, 31, 37-40, 42, and 50, wherein said nucleic acid sequence encodes a polypeptide that retains GAS or RS activity;
(ii) said GAS or RS gene comprises the nucleic acid sequence of any one of SEQ ID NO: 1-8, 25, 31, 37-40, 42, and 50;
(iii) said GAS or RS gene encodes a polypeptide comprising an amino acid sequence having at least 80% sequence identity to an amino acid sequence of any one of SEQ ID NO: 9-12, 41, 43, and 51, wherein said polypeptide retains GAS or RS activity; and/or
(iv) said GAS or RS gene encodes a polypeptide comprising an amino acid sequence of any one of SEQ ID NO: 9-12, 41, 43, and 51; and wherein the mutation comprises an insertion, a substitution, or a deletion of one or more nucleotides of (v) SEQ ID NO: 1, 2, 5, 6, 25, 37, or 38 in a *Pisum sativum* GAS gene, (vi) SEQ ID NO: 3, 4, 7, 8, 31, 39, or 40 in a *Pisum sativum* RS gene, and/or (vii) SEQ ID NO: 42 or 50 in a Glycine max GAS gene;
optionally wherein:
(a) the mutation comprises a G to A substitution of nucleotide 128 of SEQ ID NO: 5 or 25, or said plant or plant part comprises a polynucleotide comprising a nucleic acid sequence of SEQ ID NO: 15 or 26 when said mutation is introduced;
(b) the mutation comprises a deletion of nucleotides 115-119 of SEQ ID NO: 25, or said plant or plant part comprises a polynucleotide comprising a nucleic acid sequence of SEQ ID NO: 27 when said mutation is introduced;
(c) the mutation comprises a deletion of nucleotides 111-117 of SEQ ID NO: 25, or said plant or plant part comprises a polynucleotide comprising a nucleic acid sequence of SEQ ID NO: 28 when said mutation is introduced;
(d) the mutation comprises a G to A substitution of nucleotide 1044 of SEQ ID NO: 7 or 31, or said plant or plant part comprises a polynucleotide comprising a nucleic acid sequence of SEQ ID NO: 16 or 32 when said mutation is introduced;
(e) the mutation comprises a deletion of nucleotides 1246-1253 of SEQ ID NO: 31, or said plant or plant part comprises a polynucleotide comprising a nucleic acid sequence of SEQ ID NO: 33 when said mutation is introduced;
(f) the mutation comprises a deletion of nucleotides 1248-1254 of SEQ ID NO: 31, or said plant or plant part comprises a polynucleotide comprising a nucleic acid sequence of SEQ ID NO: 34 when said mutation is introduced;
(g) the mutation produces a G to D substitution of amino acid 43 of SEQ ID NO: 9, or said plant or plant part comprises a polypeptide comprising an amino acid sequence of SEQ ID NO: 17 when said mutation is introduced;
(h) the mutation produces a mutated *Pisum sativum* GAS protein comprising an amino acid sequence of SEQ ID NO: 29 or 30;
(i) the mutation produces a deletion of amino acids 348-798 of SEQ ID NO: 11, or said plant or plant part comprises a polypeptide comprising an amino acid sequence of SEQ ID NO: 18 when said mutation is introduced;
(j) the mutation produces a mutated *Pisum sativum* RS protein comprising an amino acid sequence of SEQ ID NO: 35 or 36;
(k) the mutation comprises a deletion of nucleotides 197-209 of SEQ ID NO: 42, or said plant or plant part comprises a polynucleotide comprising a nucleic acid sequence of SEQ ID NO: 44 when said mutation is introduced;
(l) the mutation comprises a deletion of nucleotides 199-209 of SEQ ID NO: 42, or said plant or plant part comprises a polynucleotide comprising a nucleic acid sequence of SEQ ID NO: 45 when said mutation is introduced;
(m) the mutation comprises a deletion of nucleotide 204 of SEQ ID NO: 42, or said plant or plant part comprises a polynucleotide comprising a nucleic acid sequence of SEQ ID NO: 46 when said mutation is introduced; and/or
(n) the mutation produces a mutated Glycine max GAS protein comprising an amino acid sequence of any one of SEQ ID NO: 47-49;
optionally wherein said plant or plant part is a legume.

7. The method of claim 6, further comprising
contacting the plant or plant part with a mutagen, thereby introducing said mutation into said plant or plant part, and/or
introducing editing reagents or a nucleic acid construct encoding said editing reagents into said plant or plant part, thereby introducing said mutation into said plant or plant part,
wherein said editing reagents comprise at least one nuclease and/or one or more guide RNAs (gRNAs),
wherein the nuclease cleaves a target site in a genome of said plant, plant part, or plant cell, and wherein said mutation is introduced at said cleaved target site, and
optionally wherein at least one of the one or more gRNAs comprises a nucleic acid sequence encoded by:
(i) a nucleic acid sequence that shares at least 80% sequence identity with a nucleic acid sequence of SEQ ID NO: 13, 14, or 52; or
(ii) the nucleic acid sequence of SEQ ID NO: 13, 14, or 52.

8. A plant or plant part produced by the method of any one of claims 5-7,
wherein said plant or plant part comprises reduced GAS and/or RS activity compared to a control plant or plant part, optionally wherein said plant or plant part comprises decreased raffinose family oligosaccharide (RFO) content and/or increased sucrose content compared to a control plant or plant part;
wherein the plant or plant part comprises seed raffinose content that is 85% or less relative to a control, seed stachyose content that is 50% or less relative to a control, and/or seed sucrose content that is increased by about 10% or more relative to a control; and/or
wherein the plant or plant part comprises seed raffinose content of about 0.7% or less, seed stachyose content of about 1.5% or less, and/or seed sucrose content of about 3.0% or more dry weight of total seed content.

9. A population of plants or plant parts comprising the plant or plant part of any one of claims 1-4 and 8, wherein the population comprises decreased GAS and/or RS activity, decreased raffinose family oligosaccharide (RFO) content and/or increased sucrose content compared to a control population, optionally wherein the population of plants or plant parts comprises:
seed raffinose content that is 85% or less relative to a control, seed stachyose content that is 50% or less relative to a control, and/or seed sucrose content that is increased by about 10% or more relative to a control; and/or
seed raffinose content of about 0.7% or less, seed stachyose content of about 1.5% or less, and/or seed sucrose content of about 3.0% or more dry weight of total seed content.

10. A composition produced from the plant, plant part, or population of plants or plant parts of any one of claims 1-4 and 8-9, wherein the composition is a seed composition or a protein and/or oil composition, optionally wherein the composition is comprised within a food or beverage product.

11. A pea composition comprising raffinose content of about 0.7% or less, such as about 0.3% or less, stachyose content of about 1.5% or less, such as about 1.4% or less dry weight, and/or sucrose content of about 3.0% or more, such as about 3.7% or more, dry weight.

12. The pea composition of claim 11, comprising a mutated *GAS* and/or *RS* gene or homolog thereof or regulatory region thereof, wherein the pea composition comprises:
(a) a mutated *Pisum sativum GAS* gene comprising an insertion, a substitution, or a deletion of one or more nucleotides of SEQ ID NO: 1, 2, 5, 6, 25, 37, or 38; and/or
(b) a mutated *Pisum sativum RS* gene comprising an insertion, a substitution, or a deletion of one or more nucleotides of SEQ ID NO: 3, 4, 7, 8, 31, 39, or 40, and wherein the pea composition comprises:
(i) a mutated *Pisum sativum* GAS gene comprising a G to A substitution of nucleotide 128 of SEQ ID NO: 5 or 25, or comprising a nucleic acid sequence of SEQ ID NO: 15 or 26;
(ii) a mutated *Pisum sativum* GAS gene comprising a deletion of nucleotides 115-119 of SEQ ID NO: 25, or comprising a nucleic acid sequence of SEQ ID NO: 27;
(iii) a mutated *Pisum sativum* GAS gene comprising a deletion of nucleotides 111-117 of SEQ ID NO: 25, or comprising a nucleic acid sequence of SEQ ID NO: 28;
(iv) a mutated *Pisum sativum* RS gene comprising a G to A substitution of nucleotide 1044 of SEQ ID NO: 7 or 31, or comprising a nucleic acid sequence of SEQ ID NO: 16 or 32;
(v) a mutated *Pisum sativum* RS gene comprising a deletion of nucleotides 1246-1253 of SEQ ID NO: 31, or comprising a nucleic acid sequence of SEQ ID NO: 33;
(vi) a mutated *Pisum sativum* RS gene comprising a deletion of nucleotides 1248-1254 of SEQ ID NO: 31, or comprising a nucleic acid sequence of SEQ ID NO: 34;
(vii) a mutated *Pisum sativum* GAS protein comprising a G to D substitution of amino acid 43 of SEQ ID NO: 9, or comprising an amino acid sequence of SEQ ID NO: 17;
(viii) a mutated *Pisum sativum* GAS protein encoded by the nucleic acid sequence of SEQ ID NO: 27, or comprising an amino acid sequence of SEQ ID NO: 29;
(ix) a mutated *Pisum sativum* GAS protein encoded by the nucleic acid sequence of SEQ ID NO: 28, or comprising an amino acid sequence of SEQ ID NO: 30;
(x) a truncated *Pisum sativum* RS protein comprising a deletion of amino acids 348-798 of SEQ ID NO: 11, or comprising an amino acid sequence of SEQ ID NO: 18;
(xi) a mutated *Pisum sativum* RS protein encoded by the nucleic acid sequence of SEQ ID NO: 33, or comprising an amino acid sequence of SEQ ID NO: 35; and/or
(xii) a mutated *Pisum sativum* RS protein encoded by the nucleic acid sequence of SEQ ID NO: 34, or comprising an amino acid sequence of SEQ ID NO: 36.

13. A nucleic acid molecule comprising a nucleic acid sequence of a mutated GAS or RS gene or coding sequence thereof, wherein said mutation is located in a *GAS* or *RS* gene:
(i) comprising a nucleic acid sequence having at least 80% sequence identity to a nucleic acid sequence of any one of SEQ ID NO: 1-8, 25, 31, 37-40, 42, and 50, wherein said nucleic acid sequence encodes a polypeptide that retains GAS or RS activity;
(ii) comprising the nucleic acid sequence of any one of SEQ ID NO: 1-8, 25, 31, 37-40, 42, and 50;
(iii) encoding a polypeptide comprising an amino acid sequence having at least 80% sequence identity to an amino acid sequence of any one of SEQ ID NO: 9-12, 41, 43, and 51, wherein said polypeptide retains GAS or RS activity; and/or
(iv) encoding a polypeptide comprising an amino acid sequence of any one of SEQ ID NO: 9-12, 41, 43, and 51,
wherein said mutation decreases level or activity of a GAS and/or RS protein encoded by said *GAS* or *RS* gene;
wherein said nucleic acid sequence:
(i) has at least 80% identity to a nucleic acid sequence of any one of SEQ ID NOs: 15, 16, 26-28, 32-34, and 44-46;
(ii) comprises the nucleic acid sequence of any one of SEQ ID NOs: 15, 16, 26-28, 32-34, and 44-46;
(iii) encodes a polypeptide comprising an amino acid sequence having at least 80% sequence identity to an amino acid sequence of any one of SEQ ID NOs: 17, 18, 29, 30, 35, 36, and 47-49; and/or
(iv) encodes a polypeptide comprising an amino acid sequence of any one of SEQ ID NOs: 17, 18, 29, 30, 35, 36, and 47-49.

14. A DNA construct comprising, in operable linkage:
(i) a promoter that is functional in a plant cell; and
(ii) the nucleic acid molecule of claim 13.

15. A cell comprising the nucleic acid molecule of claim 13, or the DNA construct of claim 14.
